# EUROPEAN PATENT SPECIFICATION

(11) **EP 3 405 559 B1**
(45) Date of publication and mention of the grant of the patent: **17.11.2021**
(21) Application number: 17742044.5
(22) Date of filing: 20.01.2017
(51) Int. Cl.: C12M 1/00, C12M 1/26, C12M 1/28, C12M 1/34, C12Q 1/04, C12Q 1/68, C12Q 1/70

(54) **RAPID ANTIMICROBIAL SUSCEPTIBILITY TESTING USING HIGH-SENSITIVITY DIRECT DETECTION METHODS**
SCHNELLER TEST DER ANTIMIKROBIELLEN EMPFINDLICHKEIT MITHILFE VON VERFAHREN ZUR DIREKTEN HOCHEMPFINDLICHKEITSDETEKTION
ANTIBIOGRAMME RAPIDE EFFECTUÉ AU MOYEN DE PROCÉDÉS DE DÉTECTION DIRECTE À HAUTE SENSIBILITÉ

(30) Priority: 21.01.2016 US 201662281603 P
(43) Date of publication of application: 28.11.2018
(73) Proprietor: T2 Biosystems, Inc., Lexington, Massachusetts 02421 (US)
(72) Inventor: LOWERY, Thomas, Jay, Jr., Belmont MA 02478 (US); PFALLER, Michael, Andy, Cedar Key FL 32625 (US); DHANDA, Rahul, Krishan, Needham MA 02492 (US); MCDONOUGH, John, P., Sudbury MA 01776 (US); MAGNUSON, Glenn, Littleton MA 01460 (US); NEELY, Lori, Anne, Reading MA 01867 (US); THOMANN, Ulrich, Hans, Stow MA 01775 (US); HARRIS, William, Coulter, North Chelmsford MA 01863 (US); BLACK, Justin, Layne, Billerica MA 01862 (US)
(74) Representative: Bösl, Raphael Konrad
(86) International application number: PCT/US2017/014405
(87) International publication number: WO 2017/127727

(56) References cited:
- WO-A1-2014/145899
- CA-A1- 2 815 085
- US-A1- 2004 219 517
- US-A1- 2013 224 073
- US-A1- 2013 260 367
- US-A1- 2015 064 703
- US-A1- 2015 118 688
- US-B2- 8 546 082
- US-B2- 8 741 565
- IRENE SINN ET AL: "Asynchronous Magnetic Bead Rotation Microviscometer for Rapid, Sensitive, and Label-Free Studies of Bacterial Growth and Drug Sensitivity", ANALYTICAL CHEMISTRY, vol. 84, no. 12, 1 June 2012 (2012-06-01), pages 5250-5256, XP55611121, US ISSN: 0003-2700, DOI: 10.1021/ac300128p
- IRENE SINN ET AL: "Asynchronous magnetic bead rotation (AMBR) biosensor in microfluidic droplets for rapid bacterial growth and susceptibility measurements", LAB ON A CHIP, vol. 11, no. 15, 1 January 2011 (2011-01-01), page 2604, XP55051299, ISSN: 1473-0197, DOI: 10.1039/c0lc00734j
- ANJA MEZGER ET AL: "A General Method for Rapid Determination of Antibiotic Susceptibility and Species in Bacterial Infections", JOURNAL OF CLINICAL MICROBIOLOGY, vol. 53, no. 2, 19 November 2014 (2014-11-19), pages 425-432, XP55201455, ISSN: 0095-1137, DOI: 10.1128/JCM.02434-14

## Description

### FIELD OF THE INVENTION

The invention features methods for rapid and sensitive detection and identification of pathogens and determination of the susceptibility of pathogens to antimicrobial agents useful in the diagnosis and treatment of disease, including bloodstream infections (e.g., bacteremia and fungemia) and sepsis.

### BACKGROUND OF THE INVENTION

The current paradigm of *in vitro* diagnostic testing for patients suspected of bloodstream infections (e.g., bacteremia and fungemia), sepsis, and related conditions is laborious, insensitive, and requires multiple days. These bloodstream and tissue infections can be challenging to detect with existing methods due to the low titer level of the infectious pathogen in the sampled biofluid. Titer levels of microbial pathogens are typically less than 1 colony-forming unit (CFU)/mL to as high as 100 CFU/mL in these diseases.

For example, blood culture is currently the reference standard for diagnosis of bloodstream infections. Typically, multiple blood draws are taken for a blood culture. These blood samples are drawn into blood culture vials (also known as blood culture bottles) that contain media suitable for enhanced growth of aerobic, anaerobic, or fungal organisms. One downside to blood culture is that it may take from 1 to 5 days for sufficient growth to occur in the blood culture vial for the blood culture instrument to flag the culture as positive. Growth curves for organisms are typically logarithmic with a lag phase and shape that depends on initial titer level, volume of blood collected, timing and number of blood cultures obtained, duration of blood culture incubation, antibiotics that may be present, and the type of pathogen (e.g., rapidly growing, fastidious, or uncultivatable). Determination of blood culture positivity typically relies on a solid state sensor in the blood culture vial that changes its optical properties upon adequate production of carbon dioxide, although electrochemical, PCR, and immunological approaches are being developed to more rapidly detect blood culture positivity. The titer level necessary to produce enough carbon dioxide is typically about 1×10⁶ to 1×10⁸ CFU/ml. Another significant weakness of blood culture is its low overall sensitivity. At present, between 30% and 50% of patients have false negative results from blood culture and therefore do not receive adequate therapy. Unfortunately, inappropriate or delayed antimicrobial therapy in patients with sepsis is associated with a five-fold reduction in survival. It has been documented that only about 50% of septic shock patients receive effective antimicrobial therapy within 6 h of documented hypotension and that mortality increased by 7.6% each hour of delay after onset of hypotension.

After blood culture positivity, an aliquot is typically removed from the culture tube and characterized by microscopy. This analysis typically identifies the category of microorganism that has cultured positive, for example, as gram positive, gram negative, or yeast. After gram staining, an aliquot of the blood culture is subcultured to further isolate and grow the organism. Finally, the subcultured organism is subjected to species identification and antimicrobial susceptibility testing (AST). During AST, the level and type of antimicrobial agent that adequately arrests growth is identified. In total, current methods can take as long as about 3 to 8 days from start of blood culture until AST results are available.

Sinn et al., ANALYTICAL CHEMISTRY 84: 5250 - 5256, and Sinn et al., 2011, LAB ON A CHIP, 11: 2604 describe a magnetic bead-based method of isolating and determining the identity of a microorganism in a sample and determining susceptibility. Mezger et al., 2014, JOURNAL OF CLINICAL MICROBIOLOGY 53: 425 - 432 discloses a method in which microorganisms are isolated and grown in the presence and absence of a antimicrobial for determining susceptibility. US 2013/260367 relates to methods for detecting the presence of an analyte in a liquid sample, including methods comprising contacting a sample with magnetic particles and detecting the presence or concentration of an analyte. US 2015/064703 provides a device that uses radiofrequency detection of the aggregation or disaggregation of magnetic nanoparticles without using nuclear magnetic resonance methods. Depending on the specific reagents used, the detected aggregation or disaggregation can be interpreted as an indicator of the presence of a molecule or microorganism of interest in a liquid sample.

Thus, there remains a need in the art for methods and compositions that allow for rapid determination of both the presence and identity of pathogens associated with infection as well as antimicrobial susceptibility for the causative pathogen.

### SUMMARY OF THE INVENTION

The description also provides a method of performing rapid antimicrobial susceptibility testing for a pathogen present in a biological sample obtained from a subject, the method including the following steps: (a) providing a biological sample obtained from a subject infected by a pathogen, wherein the genus of the pathogen has been determined by a detection method characterized by one or more of the following: (i) the presence and genus of the pathogen in the biological sample is determined within about 5 hours from obtaining the sample from the patient; (ii) the presence and genus of the pathogen is determined directly from the biological sample without a prior culturing step; and/or (iii) the pathogen is present in the biological sample at a concentration of about 10 colony-forming units (CFU)/mL of biological sample or less; and (b) testing the susceptibility of the pathogen in the biological sample or a subculture thereof to one or more antimicrobial agents selected based on the genus of the pathogen, thereby determining whether the pathogen is susceptible to the one or more antimicrobial agents. The method may further include a step of incubating a portion of the biological sample or a subculture thereof under conditions suitable for enhanced growth of the pathogen to form a pathogen culture, and wherein step (b) includes testing the susceptibility of the pathogen culture to the one or more antimicrobial agents. The method may further include obtaining an additional biological sample from the subject, and wherein step (b) includes testing the susceptibility of the pathogen in the additional biological sample or a subculture thereof. The detecting method of step (a) may include amplifying a nucleic acid in the biological sample characteristic of the pathogen and detecting the amplified nucleic acid, thereby determining the presence and genus of the pathogen.

The presence and genus of the pathogen may be determined by the detection method of step (a) within about 3 hours from obtaining the sample from the patient. The pathogen may be present in the biological sample at a concentration of about 5 CFU/mL of biological sample or less in the detection method of step (a). The pathogen may be present in the biological sample at a concentration of about 1 CFU/mL of biological sample or less in the detection method of step (a). The method may further include a step of performing centrifugation, filtration, or lysis centrifugation of a portion of the biological sample or a subculture thereof prior to step (b), thereby producing a pellet comprising the pathogen. The method may further include a step of performing centrifugation, filtration, or lysis centrifugation of the additional biological sample prior to step (b), thereby producing a pellet comprising the pathogen. The method may further include plating the pellet or a portion thereof on one or more media plates selected based on the genus of the pathogen, and incubating the one or more media plates under conditions suitable for growth of the pathogen. The one or more media plates may be used in step (b) to test the susceptibility of the pathogen to the one or more antimicrobial agents. Testing the susceptibility of the pathogen in step (b) may include a broth dilution test, a disk diffusion test, an antimicrobial gradient test, growth on chromogenic media, an enzyme activity assay, and/or an automated instrument. Step (b) may include testing the susceptibility of the pathogen in the biological sample or a subculture thereof to four or more antimicrobial agents. Step (b) may include testing the susceptibility of the pathogen in the biological sample or a subculture thereof to 10 or more antimicrobial agents. The method may further include selecting an antimicrobial therapy for the subject based on the results of step (b). The method may further include administering to the subject an antimicrobial agent to which the pathogen has been determined to be susceptible in step (b). The genus may be a taxonomic family or a taxonomic genus. The species of the pathogen may not have been determined. The species of the pathogen may have been determined in step (a). Step (b) may further include testing the susceptibility of the pathogen in the biological sample or a subculture thereof to one or more antimicrobial agents selected based on the species of the pathogen. The species may be a taxonomic species. The method may further include obtaining a subsequent biological sample from the subject following administration of the antimicrobial agent. The method may further include determining the presence of the pathogen in the subsequent biological sample. The method may further include quantifying the expression level of a target nucleic acid characteristic of the pathogen in the subsequent biological sample.

The description also features a method of performing rapid antimicrobial susceptibility testing for a pathogen present in a biological sample obtained from a subject, the method including the following steps: (a) determining the presence and genus of a pathogen in the biological sample by amplifying a nucleic acid in the biological sample characteristic of the pathogen, and detecting the amplified nucleic acid, thereby determining the presence and genus of the pathogen, wherein: (i) the presence and genus of the pathogen is determined within 5 hours from the onset of step (a); (ii) the biological sample is obtained directly from the subject without a culturing step prior to step (a); and/or (iii) the pathogen is present in the biological sample at a concentration of 10 CFU/mL of biological sample or less; (b) in parallel to step (a), incubating a second portion of the biological sample under conditions suitable for growth of the pathogen to form a pathogen culture; and (c) comparing the growth rate of a first aliquot of the pathogen culture in the presence of the antimicrobial agent to the growth rate of a second aliquot of the pathogen culture in the absence of the antimicrobial agent, thereby determining whether the pathogen is susceptible to the antimicrobial agent.

In a first aspect, the invention features a method of performing rapid antimicrobial susceptibility testing for a pathogen in a biological sample obtained from a subject, the method including: (a) determining the presence and genus of a pathogen in a biological sample by (i) preparing an assay sample by contacting a first portion of the biological sample with magnetic particles, wherein the magnetic particles have binding moieties on their surfaces, the binding moieties operative to alter the specific aggregation of the magnetic particles in the presence of an analyte associated with the pathogen; (ii) placing the assay sample in a device, the device including a support defining a well for holding the assay sample, and having an RF coil configured to detect a signal produced by exposing the assay sample to a bias magnetic field created using one or more magnets and an RF pulse sequence; (iii) exposing the assay sample to the bias magnetic field and the RF pulse sequence; (iv) following step (iii), measuring the signal produced by the assay sample; and (v) based on the results of step (iv), determining the presence and genus of the pathogen in the biological sample; (b) in parallel to step (a), incubating a second portion of the biological sample under conditions suitable for growth of the pathogen to form a pathogen culture; and (c) comparing the growth rate of a first aliquot of the pathogen culture in the presence of the antimicrobial agent to the growth rate of a second aliquot of the pathogen culture in the absence of the antimicrobial agent, thereby determining whether the pathogen is susceptible to the antimicrobial agent.

In some embodiments of the first aspect, step (b) further includes inoculating a growth medium with an aliquot of the biological sample to form a subculture and incubating the subculture under conditions suitable for enhanced growth of the pathogen, wherein the growth medium is selected based on the results of step (a). In some embodiments, step (c) includes comparing the growth rate of a first aliquot of the subculture in the presence of the antimicrobial agent to the growth rate of a second aliquot of the subculture in the absence of the antimicrobial agent. In some embodiments, the method further includes contacting the pathogen culture with an additive prior to step (c), wherein the additive is selected based on the results of step (a), thereby enhancing growth of the culture. In some embodiments, the method further includes a step of performing centrifugation, filtration, or lysis centrifugation of the pathogen culture or an additional portion of the biological sample prior to step (c), thereby producing a pellet comprising the pathogen. In some embodiments, the method further includes plating the pellet or a portion thereof on one or more media plates selected based on the genus of the pathogen, and incubating the one or more media plates under conditions suitable for growth of the pathogen. In some embodiments, the one or more media plates are used in step (c) to compare the growth rates of the pathogen in the first and second aliquots of the pathogen culture.

The description also provides a method of performing rapid antimicrobial susceptibility testing for a pathogen present in a biological sample obtained from a subject, the method including the following steps: (a) determining the presence and genus of a pathogen in the biological sample by amplifying a nucleic acid in the biological sample characteristic of the pathogen, and detecting the amplified nucleic acid, thereby determining the presence and genus of the pathogen, wherein: (i) the presence and genus of the pathogen is determined within 5 hours from the onset of step (a); (ii) the biological sample is obtained directly from the subject without an intervening culturing step prior to step (a); and/or (iii) the pathogen is present in the biological sample at a concentration of 10 CFU/mL of biological sample or less; (b) obtaining an additional biological sample directly from the patient following step (a); and (c) comparing the growth rate of the pathogen in a first aliquot of the additional biological sample in the presence of the antimicrobial agent to the growth rate of the pathogen in a second aliquot of the additional biological sample in the absence of the antimicrobial agent, thereby determining whether the pathogen is susceptible to the antimicrobial agent.

In a second aspect, the invention features a method of performing rapid antimicrobial susceptibility testing for a pathogen in a biological sample obtained from a subject, the method including: (a) determining the presence and genus of a pathogen in a biological sample by (i) preparing an assay sample by contacting a first portion of the biological sample with magnetic particles, wherein the magnetic particles have binding moieties on their surfaces, the binding moieties operative to alter the specific aggregation of the magnetic particles in the present of an analyte associated with the pathogen; (ii) placing the assay sample in a device, the device including a support defining a well for holding the assay sample, and having an RF coil configured to detect a signal produced by exposing the assay sample to a bias magnetic field created using one or more magnets and an RF pulse sequence; (iii) exposing the assay sample to the bias magnetic field and the RF pulse sequence; (iv) following step (iii), measuring the signal produced by the assay sample; and (v) based on the results of step (iv), determining the presence and genus of the pathogen in the biological sample; (b) obtaining an additional biological sample from the patient following step (a); and (c) comparing the growth rate of the pathogen in a first aliquot of the additional biological sample in the presence of the antimicrobial agent to the growth rate of the pathogen in a second aliquot of the additional biological sample in the absence of the antimicrobial agent, thereby determining whether the pathogen is susceptible to the antimicrobial agent wherein the additional biological has been obtained sample from the patient following step (a).

In some embodiments of the the second aspect, step (b) further includes inoculating a growth medium with an aliquot of the additional biological sample to form a subculture and incubating the subculture under conditions suitable for enhanced growth of the pathogen, wherein the growth medium is selected based on the results of step (a). In some embodiments, step (c) includes comparing the growth rate of a first aliquot of the subculture in the presence of the antimicrobial agent to the growth rate of a second aliquot of the subculture in the absence of the antimicrobial agent. In some embodiments, the method further includes a step of performing centrifugation, filtration, or lysis centrifugation of the additional biological sample prior to step (c), thereby producing a pellet comprising the pathogen. In some embodiments, the method further includes plating the pellet or a portion thereof on one or more media plates selected based on the genus of the pathogen, and incubating the one or more media plates under conditions suitable for growth of the pathogen. In some embodiments, the one or more media plates are used in step (c) to compare the growth rates of the pathogen in the first and second aliquots of the additional biological sample. In some embodiments, growth rates are determined using a broth dilution test, a disk diffusion test, an antimicrobial gradient test, chromogenic media, an enzyme activity assay, and/or an automated instrument. In some embodiments, the antimicrobial gradient test is an epsilometertest (ETEST®). In some embodiments, the antimicrobial agent of step (c) is selected based on the results of step (a). In some embodiments, a plurality of antimicrobial agents are tested in step (c) to determine an antimicrobial susceptibility profile of the pathogen. In some embodiments, at least 4 antimicrobial agents are tested in step (c). In some embodiments, at least 10 antimicrobial agents are tested in step (c). In some embodiments, the analyte is an amplicon characteristic of the pathogen generated by amplifying a corresponding target nucleic acid in the presence of a forward and a reverse primer.

In some embodiments of any of the preceding aspects, amplifying is performed by asymmetric polymerase chain reaction (PCR).

In some embodiments of the first aspect or the second aspect, the magnetic particles include a first population of magnetic particles conjugated to a first probe, and a second population of magnetic particles conjugated to a second probe, wherein the magnetic particles form aggregates in the presence of the amplicon characteristic of the pathogen. In some embodiments, substep (i) includes adding to the liquid sample from 1×10⁶ to 1×10¹³ magnetic particles per milliliter of the liquid sample. In some embodiments, the magnetic particles have a mean diameter of from 700 nm to 950 nm. In some embodiments, the magnetic particles have a T₂ relaxivity per particle of from 1×10⁹ to 1×10¹² mM⁻¹s⁻¹. In some embodiments, a plurality of assay samples are prepared in substep (i) by independently contacting each member of the plurality of assay samples with a population of magnetic particles configured to form aggregates in the presence of an analyte associated with a member of the panel of pathogens, and wherein each member of the plurality of assay samples is subjected to substeps (ii) through (iv) of the method.

In some embodiments of any of the preceding aspects, step (a) includes assaying for the presence of a panel of pathogens.

In some embodiments of the first aspect or the second aspect, step (a) or (c) further includes quantifying the expression level of a target nucleic acid characteristic of the pathogen. In some embodiments, step (a) or (c) includes amplifying the target nucleic acid characteristic of the pathogen in an reaction mixture in a detection tube resulting in the production of an amplicon corresponding to the target nucleic acid characteristic of the pathogen, wherein the method is performed in the device recited in step (a), the reaction mixture including a portion of the biological sample including the target nucleic acid characteristic of the pathogen, primers specific for the target nucleic acid, and superparamagnetic particles, the superparagmagnetic particles operable to aggregate or disaggregate in the presence of the amplicon; and the amplification including the following steps: (i) performing one or more cycles of amplification; (ii) exposing the reaction mixture to conditions permitting the aggregation or disaggregation of the superparamagnetic particles; (iii) exposing the sample to a bias magnetic field and an RF pulse sequence; (iv) following step (iii), measuring the signal from the detection tube; (v) repeating steps (i)-(iv) until a desired amount of amplification is obtained; and (vi) on the basis of the result of step (iv), quantifying the amplicons present at the corresponding cycle of amplification; wherein the initial quantity of target nucleic acid characteristic of the antimicrobial resistance gene in the sample is determined based on the quantity of amplicons determined at each cycle of the amplification. In some embodiments, the method further includes applying a magnetic field to the detection tube following the measuring the signal from the detection tube, resulting in the sequestration of the superparamagnetic particles to the side of the detection tube, and releasing the magnetic field subsequent to the completion of one or more additional cycles of amplification. In some embodiments, the superparamagnetic particles are greater than 100 nm in diameter. In some embodiments, the superparamagnetic particles are less than 100 nm in diameter. In some embodiments, the superparamagnetic particles have a diameter of 30 nm. In some embodiments, the target nucleic acid characteristic of the pathogen is an antimicrobial resistance gene or a housekeeping gene.

In some embodiments of any of the preceding aspects, step (a) further includes determining the titer of the pathogen.

In some embodiments of any of the preceding aspects, the steps of the method are completed within 2 days. In some embodiments, the steps of the method are completed within 12 hours. In some embodiments, the steps of the method are completed within 7 hours.

In some embodiments of the first aspect or the second aspect, the method is capable of detecting 10 CFU of the pathogen per milliliter of the biological sample. In some embodiments, the method is capable of detecting 3 CFU of the pathogen per milliliter of the biological sample. In some embodiments, the method is capable of detecting 1 CFU of the pathogen per milliliter of the biological sample.

In some embodiments of the first aspect or the second aspect, the method further includes selecting a therapy including an antimicrobial agent for the subject based on the results of step (c). In some embodiments, the method further includes administering to the subject an effective amount of the antimicrobial agent based on the results of step (c). In some embodiments, the method further includes obtaining a subsequent biological sample from the subject following administration of the antimicrobial agent. In some embodiments, the method further includes determining the presence of the pathogen in the subsequent biological sample. In some embodiments, the method further includes quantifying the expression level of a target nucleic acid characteristic of the pathogen in the subsequent biological sample.

In some embodiments of the first aspect or the second aspect, the genus is a taxonomic family or a taxonomic genus. In some embodiments, the species of the pathogen is not determined by step (a). In some embodiments, step (a) further includes determining the species of the pathogen. In some embodiments, the antimicrobial agent of step (c) is selected based on the species of the pathogen. In some embodiments, the species is a taxonomic species.

In some embodiments of any of the preceding aspects, the biological sample is selected from the group consisting of whole blood, cerebrospinal fluid (CSF), pleural fluid, urine, or synovial fluid. In some embodiments, the biological sample is whole blood.

In some embodiments of any of the preceding aspects, the pathogen is a fungal pathogen, a bacterial pathogen, a protozoan pathogen, or a viral pathogen. In some embodiments, the fungal pathogen is a *Candida* spp. In some embodiments, the *Candida* spp. is selected from the group consisting of *Candida albicans, Candida guilliermondii, Candida glabrata, Candida krusei, Candida lusitaniae, Candida parapsilosis,* and *Candida tropicalis.* In some embodiments, the bacterial pathogen is selected from the group consisting of *Escherichia coli, Acinetobacter baumannii, Enterococcus faecalis, Enterococcus faecium, Klebsiella pneumoniae, Pseudomonas aeruginosa, Staphylococcus aureus, Borrelia burgdorferi, Borrelia afzelii, Borrelia garinii, Rickettsia rickettsii, Anaplasma phagocytophilum, Coxiella burnetii, Ehrlichia chaffeensis, Ehrlichia ewingii, Francisella tularensis, Streptococcus pneumoniae,* and *Neisseria meningitides.* In some embodiments, the protozoan pathogen is *Babesia microti* or *Babesia divergens.*

In some embodiments of any of the preceding embodiments, the pathogen is associated with bloodstream infection, sepsis, septic arthritis, pneumonia, peritonitis, osteomyelitis, meningitis, urinary tract infection or Lyme disease. In some embodiments, the bloodstream infection is fungemia, bacteremia, or viremia. In some embodiments, the fungemia is Candidemia.

Other features and advantages of the invention will be apparent from the following detailed description, drawings, and the claims.

### BRIEF DESCRIPTION OF THE DRAWINGS

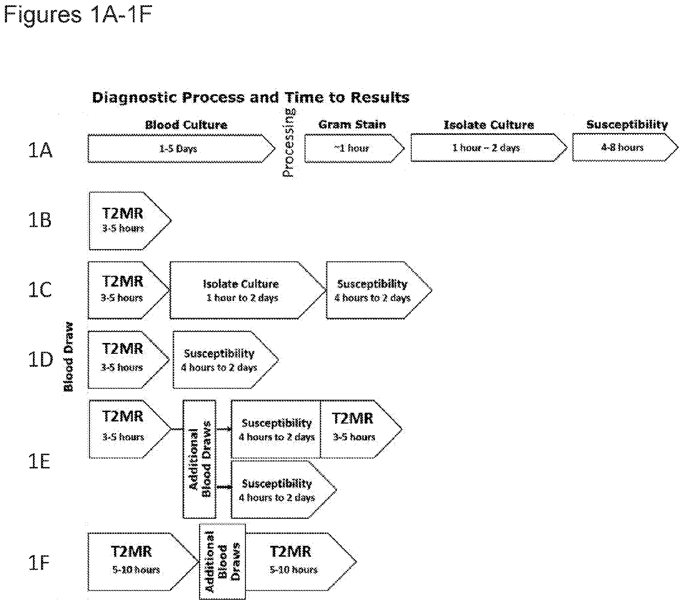

FIGURE 1A is a schematic of a current diagnostic microbiology flow for isolation and identification of a pathogen from blood followed by AST. Following blood draw, blood cultures are grown for 1 to 5 days, followed by a gram stain, culture isolation, and susceptibility testing. The approximate time to results for each step of the diagnostic flow is indicated.
FIGURE 1B is a schematic of a diagnostic and therapeutic method in which pathogen identification by T₂ magnetic resonance (T2MR) in a blood sample obtained from a subject is followed immediately by an appropriate targeted therapy against the identified pathogen.
FIGURE 1C is a schematic of a diagnostic method that includes pathogen identification by T2MR in a blood sample obtained from a subject followed by subculture of the pathogen under optimal conditions for the identified pathogen. AST is performed on an portion of the subculture. The approximate time to results for each step of the method is indicated. A skilled artisan appreciates that the length of time required to isolate the culture and test susceptibility may vary from hours to days based on organism, titer, and growth conditions.
FIGURE 1D is a schematic of a diagnostic method that includes pathogen identification and/or expression analysis by T2MR in a blood sample obtained from a subject followed immediately by AST or detection of resistance markers.
FIGURE 1E is a schematic of a diagnostic method that includes pathogen identification by T2MR in a blood sample obtained from a subject, followed by AST performed using a T2MR-based detection method. In this example, T2MR is performed a second time to quantitatively or semi-quantitatively measure microbial growth, providing more detailed information than standard antimicrobial susceptibility testing.
FIGURE 1F is a schematic of a diagnostic method that includes pathogen identification and expression analysis of key transcripts by T2MR in one or more blood samples obtained from a subject. Expression analysis is performed to monitor expression of inducible antimicrobial resistance genes as well as expression of energy metabolism or other housekeeping genes. This analysis is optionally followed by additional blood draws, and repeat of T2MR-based expression analysis (e.g., real-time PCR or RNA expression analysis of one or more genes that is characteristic of the pathogen) to determine effectiveness of treatment, which may be indicated by the decline in expression of the one or more genes, indicating cell growth arrest or death due to successful antimicrobial therapy. This process may be repeated as necessary to track antimicrobial susceptibility over time. In some embodiments, T2MR-based direct cell detection (e.g., Lee et al., Nature Medicine 14(8):869-874, 2008) may be used to monitor pathogen growth.

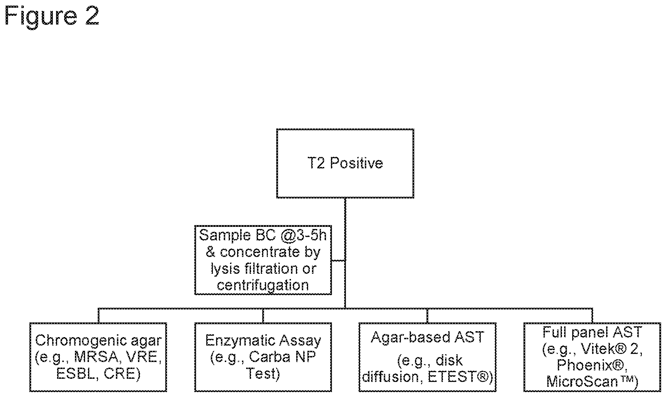

FIGURE 2 is a schematic showing exemplary downstream steps following positive detection and identification of a pathogen by T2MR ("T2 positive") from a blood culture obtained from a subject. Following species identification, a blood culture grown in parallel is sampled at 3-5 hours and the pathogen is concentrated (e.g., by lysis filtration or centrifugation). The concentrated pathogen is then plated to appropriate chromogenic media, enzymatic assays (e.g., a carbapenemase Nordmann-Poirel (Carba NP) test to detect carbapenemase-producing bacteria), an agar-based AST method (e.g., disk diffusion or ETEST®, and/or an automated AST device (e.g., an automated full panel AST device such as VITEK® 2, PHOENIX®, or MICROSCAN™).

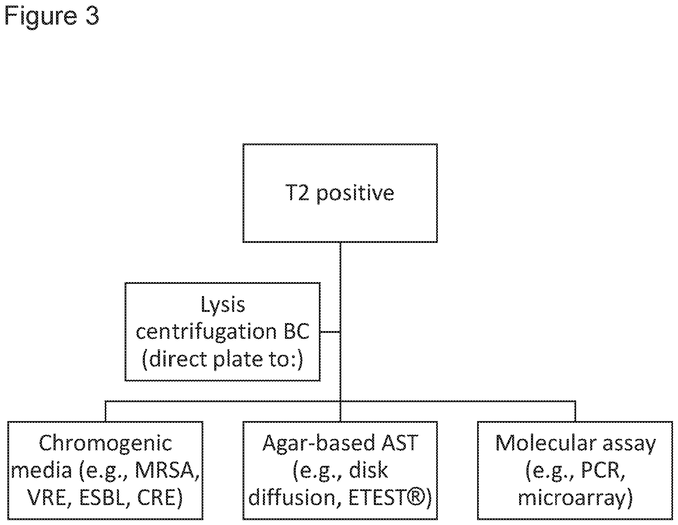

FIGURE 3 is a schematic showing exemplary downstream steps following positive detection and identification of a pathogen by T2MR ("T2 positive") from a blood culture obtained from a subject. Following pathogen identification, a lysis-centrifugation (e.g., ISOLATOR™) blood culture system is used to obtain a pellet containing the pathogen. The pellet is directly plated to appropriate chromogenic media, an agar-based AST method, and/or subjected to molecular assays (e.g., to determine the presence and/or activity of antimicrobial resistance markers).

### DETAILED DESCRIPTION OF EMBODIMENTS OF THE INVENTION

The invention provides methods for rapid and sensitive detection and identification of pathogens (e.g., identification of a genus to which the pathogen belongs, or more specifically, the species) and determination of the pathogen's susceptibility to antimicrobial agents useful in the diagnosis and/or treatment of disease, including bloodstream infections (e.g., bacteremia and fungemia), sepsis, septic shock, septic arthritis, pneumonia, peritonitis, osteomyeletis, meningitis, empyema, urinary tract infection, and systemic inflammatory response syndrome (SIRS). In some embodiments, the invention provides methods for determination of an improved antimicrobial therapy for a subject suffering from an infection based on the susceptibility of the infectious pathogen. The description also provides methods of treatment for disease, including bloodstream infections (e.g., bacteremia and fungemia), sepsis, septic shock, septic arthritis, pneumonia, peritonitis, osteomyelitis, meningitis, empyema, urinary tract infection, and SIRS, that involve administration of an antimicrobial therapy based on the susceptibility of the pathogen present in a biological sample obtained from a subject.

The methods of the invention can be used for rapid detection and identification, along with rapid AST, of any suitable pathogen. In some embodiments, the pathogen is a bacterial pathogen, including Gram-positive bacteria (e.g., Gram-positive anaerobic bacteria), Gram-negative bacteria (e.g., Gram-negative anaerobic bacteria), *Enterobacteriaceae* spp., *Acinetobacter* spp. (e.g., *Acinetobacter baumannii), Enterococcus* spp. (e.g., *Enterococcus faecium* and *Enterococcus faecalis), Klebsiella* spp. (e.g., *Klebsiella pneumoniae), Pseudomonas* spp. (e.g., *Pseudomonas aeruginosa), Staphylococcus* spp. (including, e.g., coagulase-positive species (e.g., *Staphylococcus aureus)* and coagulase-negative (CoNS) species), *Streptococcus* spp. (e.g., β-hemolytic streptococci, *Streptococcus mitis, Streptococcus pneumoniae, Streptococcus agalactiae,* and *Streptococcus pyogenes), Escherichia* spp. (e.g., *Escherichia coli), Stenotrophomonas* spp. (e.g., *Stenotrophomonas maltophilia), Proteus* spp. (e.g., *Proteus mirabilis* and *Proteus vulgaris), Serratia* spp. (e.g., *Serratia marcescens), Citrobacter* spp. (e.g., *Citrobacter freundii), Enterobacter* spp. (e.g., *Enterobacter aerogenes* and *Enterobacter cloacae), Borrelia* spp. (e.g., *Borrelia burgdorferi, Borrelia afzelii,* and *Borrelia garinii), Rickettsia* spp. (e.g., *Rickettsia rickettsii), Anaplasma* spp. (e.g., *Anaplasma phagocytophilum), Coxiella* spp. (e.g., *Coxiella burnetii*), *Ehrlichia* spp. (e.g., *Ehrlichia chaffeensis* and *Ehrlichia ewingii*), *Franciscella* spp. (e.g., *Francisella tularensis), Clostridium* spp. (e.g., *Clostridium botulinum, Clostridium difficile, Clostridium perfringens,* and *Clostridium tetani), Bacteroides* spp. (e.g,. *Bacteroides fragilis),* and *Neisseria* spp. (e.g., *Neisseria meningitides).* In some embodiments, the pathogen is a fungal pathogen, including *Candida* spp. (e.g., *Candida albicans, Candida guilliermondii, Candida glabrata, Candida krusei, Candida lusitaniae, Candida parapsilosis,* and *Candida tropicalis), Saccharomyces* spp., *Aspergillus* spp. (e.g., *Aspergillus fumigatus, Aspergillus clavatus,* and *Aspergillus flavus),* and *Cryptococcus* spp. (e.g., *Cryptococcus neoformans, Cryptococcus laurentii,* and *Cryptococcus albidus).* In some embodiments, the pathogen is a protozoan pathogen, including *Babesia* spp. (e.g., *Babesia microti* and *Babesia divergens).*

In some embodiments, the methods of the invention employ magnetic particles. In some embodiments, the methods employ an NMR unit, optionally one or more magnetic assisted agglomeration (MAA) units, optionally one or more incubation stations at different temperatures, optionally one or more vortexers, optionally one or more centrifuges, optionally a fluidic manipulation station, optionally a robotic system, and optionally one or more modular cartridges, as described in International Patent Application Publication No. WO 2012/054639. In some embodiments, the methods of the invention may employ a conduit-containing device, for example, as described in U.S. Patent Application Publication No. US 2013/0265054. In some embodiments, the methods of the invention may involve detecting cells by using magnetic particles comprising a binding agent that is operative to bind the cell surface of a pathogen and this binding event can lead to a change in measured signal such as a change in the measured T2MR value (see, e.g., International Patent Application Publication No. WO 2012/129281; Skewis et al. Nuclear Magnetic Resonance Nanotechnology: Applications in Clinical Diagnostics and Monitoring. Encyclopedia of Analytical Chemistry, 2013; Kaittanis et al. Nano Lett. 7:380, 2007; Lee et al. Nat. Med. 14:869, 2008; Kulkarni et al. Anal. Chem. 82:7430, 2010; Chung et al. ACS Nano 5:8834, 2011; Liong et al. Bioconjug. Chem. 22:2390, 2011; and Lee et al. Angew. Chem. Int. Ed. 48:5657, 2009). In some embodiments, the methods of the invention are performed (in full or in part) using a fully-automated system, e.g., a T2Dx® instrument (T2 Biosystems, Inc., Lexington, Massachusetts, USA). The methods of the invention can be used to assay a biological sample (e.g., whole blood, erum, plasma, cerebrospinal fluid (CSF), pleural fluid, urine, synovial fluid, breast milk, sweat, tears, saliva, semen, feces, vaginal fluid or tissue, sputum, nasopharyngeal aspirate or swab, lacrimal fluid, mucous, or epithelial swab (buccal swab), and tissues (e.g., tissue homogenates), organs, bones, teeth, among others).

### Definitions

The terms "aggregation," "agglomeration," and "clustering" are used interchangeably in the context of the magnetic particles described herein and mean the binding of two or more magnetic particles to one another, for example, via a multivalent analyte, multimeric form of analyte, antibody, nucleic acid molecule, or other binding molecule or entity. In some instances, magnetic particle agglomeration is reversible. Such aggregation may lead to the formation of "aggregates," which may include amplicons and magnetic particles bearing binding moieties.

The terms "amplification" or "amplify" or derivatives thereof as used herein mean one or more methods known in the art for copying a target or template nucleic acid, thereby increasing the number of copies of a selected nucleic acid sequence. Amplification may be exponential or linear. A target or template nucleic acid may be either DNA or RNA (e.g., mRNA). The sequences amplified in this manner form an "amplified region," "amplified nucleic acid," or "amplicon." Primer probes can be readily designed by those skilled in the art to target a specific template nucleic acid sequence.

By "analyte" is meant a substance or a constituent of a sample to be analyzed. Exemplary analytes include one or more species of one or more of the following: a protein, a peptide, a polypeptide, an amino acid, a nucleic acid, an oligonucleotide, RNA (e.g., mRNA), DNA, an antibody, a carbohydrate, a polysaccharide, glucose, a lipid, a gas (e.g., oxygen or carbon dioxide), an electrolyte (e.g., sodium, potassium, chloride, bicarbonate, blood urea nitrogen (BUN), magnesium, phosphate, calcium, ammonia, lactate), a lipoprotein, cholesterol, a fatty acid, a glycoprotein, a proteoglycan, a lipopolysaccharide, a cell surface marker (e.g., a cell surface protein of a pathogen), a cytoplasmic marker (e.g., CD4/CD8 or CD4/viral load), a therapeutic agent, a metabolite of a therapeutic agent, a marker for the detection of a weapon (e.g., a chemical or biological weapon), an organism, a pathogen, a pathogen byproduct, a parasite (e.g., a protozoan or a helminth), a protist, a fungus (e.g., yeast or mold), a bacterium, an actinomycete, a cell (e.g., a whole cell, a tumor cell, a stem cell, a white blood cell, a T cell (e.g., displaying CD3, CD4, CD8, IL2R, CD35, or other surface markers), or another cell identified with one or more specific markers), a virus, a prion, a plant component, a plant by-product, algae, an algae by-product, plant growth hormone, an insecticide, a man-made toxin, an environmental toxin, an oil component, and components derived therefrom.

A "biological sample" is a sample obtained from a subject including but not limited to whole blood, serum, plasma, cerebrospinal fluid (CSF), pleural fluid, urine, synovial fluid, breast milk, sweat, tears, saliva, semen, feces, vaginal fluid or tissue, sputum, nasopharyngeal aspirate or swab, lacrimal fluid, mucous, or epithelial swab (buccal swab), tissues (e.g., tissue homogenates), organs, bones, teeth, among others.

A "biomarker" is a biological substance that can be used as an indicator of a particular disease state or particular physiological state of an organism, generally a biomarker is a protein or other native compound measured in bodily fluid whose concentration reflects the presence or severity or staging of a disease state or dysfunction, can be used to monitor therapeutic progress of treatment of a disease or disorder or dysfunction, or can be used as a surrogate measure of clinical outcome or progression.

The term "growth" is used in its broadest sense, and includes changes in cell size or metabolic state as well as changes in cell number. "Growth" encompasses the growth of an individual pathogen cell, as well as the growth of a population of pathogen cells. Growth may include cell division of a pathogen cell into two daughter cells, as well as a pathogen increasing in size over time without cell division.

The term "conditions suitable for enhanced growth," as used herein, encompasses conditions (e.g., media, growth temperature, additives, oxygen content, and the like) that lead to enhanced (e.g., increased) growth relative to a reference condition, which may be, for example, a standard condition used for growth of a microbial species when the identity of the microbial species is not known.

The term "antimicrobial agent," as used herein, refers to any compound having an inhibitory (or antagonistic) effect on the growth of microorganisms, that is, agents that are capable of at least reducing the growth rate (e.g., bacteriostatic agents with respect to controlling the growth of bacteria) as well as agents that cause toxic effects (e.g., bactericide agents killing bacteria). An antimicrobial agent may be selected from small organic or inorganic molecules; saccharines; oligosaccharides; polysaccharides; biological macromolecules, e.g., peptides, proteins, and peptide analogs and derivatives; peptidomimetics; antibodies and antigen binding fragments thereof; nucleic acids; nucleic acid analogs and derivatives; glycogens or other sugars; immunogens; antigens; an extract made from biological materials such as bacteria, plants, fungi, or animal cells; animal tissues; naturally occurring or synthetic compositions; and any combinations thereof. As used herein, the term "antimicrobial agent" includes antibacterial agents, antifungal agents, antiviral agents, antiprotozoal agents, antiviral agents, and mixtures thereof.

Exemplary antibacterial agents include, but are not limited to, acrosoxacin, amifioxacin, amikacin, amoxycillin, ampicillin, aspoxicillin, azidocillin, azithromycin, aztreonam, balofloxacin, benzylpenicillin, biapenem, brodimoprim, cefaclor, cefadroxil, cefatrizine, cefcapene, cefdinir, cefetamet, ceftmetazole, cefoxitin, cefprozil, cefroxadine, ceftarolin, ceftazidime, ceftibuten, ceftobiprole, cefuroxime, cephalexin, cephalonium, cephaloridine, cephamandole, cephazolin, cephradine, chlorquinaldol, chlortetracycline, ciclacillin, cinoxacin, ciprofloxacin, clarithromycin, clavulanic acid, clindamycin, clofazimine, cloxacillin, colistin, danofloxacin, dapsone, daptomycin, demeclocycline, dicloxacillin, difloxacin, doripenem, doxycycline, enoxacin, enrofloxacin, erythromycin, fleroxacin, flomoxef, flucloxacillin, flumequine, fosfomycin, gentamycin, isoniazid, imipenem, kanamycin, levofloxacin, linezolid, mandelic acid, mecillinam, meropenem, metronidazole, minocycline, moxalactam, mupirocin, nadifloxacin, nafcillin, nalidixic acid, netilmycin, netromycin, nifuirtoinol, nitrofurantoin, nitroxoline, norfloxacin, ofloxacin, oxacillin, oxytetracycline, panipenem, pefloxacin, phenoxymethylpenicillin, pipemidic acid, piromidic acid, pivampicillin, pivmecillinam, polymixin-b, prulifloxacin, rufloxacin, sparfloxacin, sulbactam, sulfabenzamide, sulfacytine, sulfametopyrazine, sulphacetamide, sulphadiazine, sulphadimidine, sulphamethizole, sulphamethoxazole, sulphanilamide, sulphasomidine, sulphathiazole, teicoplanin, temafioxacin, tetracycline, tetroxoprim, tigecycline, tinidazole, tobramycin, tosufloxacin, trimethoprim, vancomycin, and pharmaceutically acceptable salts or esters thereof.

Exemplary antifungal agents include, but are not limited to, polyenes (e.g., amphotericin B, candicidin, filipin, hamycin, natamycin, nystatin, and rimocidin), azoles (e.g., imidazoles such as bifonazole, butoconazole, clotrimazole, eberconazole, econazole, fenticonazole, flutrimazole, isoconazole, ketoconazole, luliconazole, miconazole, omoconazole, oxiconazole, sertaconazole, sulconazole, and tioconazole; triazoles such as albaconazole, efinaconazole, epoxiconazole, fluconazole, isavuconazole, itraconazole, posaconazole, propiconazole, ravuconazole, terconazole, and voriconazole; and thiazoles such as abafungin), allylamines (e.g., amorolfin, butenafine, naftifine, and terbinafine), echinocandins (e.g., anidulafungin, caspofungin, and micafungin), and other antifungal agents including but not limited to benzoic acid, ciclopirox olamine, 5-flucytosin, griseofulvin, haloprogin, tolnaftate, aminocandin, chlordantoin, chlorphenesin, nifuroxime, undecylenic acid, crystal violet, and pharmaceutically acceptable salts or esters thereof.

Exemplary antiprotozoal agents include, but are not limited to, acetarsol, amphotericin (e.g., liposomal amphotericin, amphotericin B, and amphotericin deoxycholate), arthemether, artsunate, atovaquone, azanidazole, azithromycin, benznidazole, chloroquine, ciprofloxacin, clindamycin, diloxanide, eflornithine, flucytosine, fluconazole, folinic acid, hydroxychloroquine, iodoquinol, lumefantrine, macrolides, mefloquine, melarsoprol, metronidazole, miltefosine, nifuratel, nifurtimox, nimorazole, nitazoxanide, omidazole, paramomycin, pentamidine, primaquine, proguanil, propenidazole, pyrimethamine, quinine, quinidine, secnidazole, sinefungin, sodium stibogluconate, spiramycin, suramin, sulfadiazine, sulfamethoxazole, tenonitrozole, temidazole, tinidazole, trimethoprim, TMP/SMX (co-timoxazole; trimethoprim and sulfamethoxazole in a 1:5 ratio), and pharmaceutically acceptable salts or esters thereof. Additional antiprotozoal agents are described, for example, in Kappagoda et al. Mayo Clin. Proc. 86(6):561-583, 2011.

Exemplary antiviral agents include, but are not limited to, abacavir, acyclovir, adefovir, amantadine, amprenavir, ampligen, arbidol, atazanavir, atripla, balavir, brivudine, cidofovir, combivir, curcumin, darunavir, delavirdine, desciclovir, didanosine, 1-docosanol, dolutegravir, edoxudine, efavirenz, emtricitabine, enfuvirtide, entecavir, ecoliever, famciclovir, fiacitabine, fomivirsen, fosamprenavir, foscarnet, fosfonet, fusion inhibitors, ganciclovir, ibacitabine, idoxuridine, imiquimod, imunovir, indinavir, inosine, integrase inhibitor, interferon (e.g., interferon type I, interferon type II, and interferon type III), lamivudine, lopinavir, loviride, maraviroc, moroxydine, methisazone, nelfinavir, nevirapine, nexavir, nucleoside analogs, novir, oseltamivir, peginterferon alfa-2a, penciclovir, peramivir, pleconaril, podophyllotoxin, protease inhibitors, pyramidine, raltegravir, reverse transcriptase inhibitors, ribavarin, rimantadine, ritonavir, saquinavir, sofosbuvir, stavudine, telaprevir, tenofovir, tenofovir disoproxil, tipranavir, trifluridine, trizivir, tromontadine, truvada, valacyclovir, valganciclovir, vicriviroc, vidarabine, viramidine, zalcitabine, zanamivir, zidovudine, and pharmaceutically acceptable salts or esters thereof.

By an "isolated" nucleic acid molecule is meant a nucleic acid molecule that is removed from the environment in which it naturally occurs. For example, a naturally-occurring nucleic acid molecule present in the genome of cell or as part of a gene bank is not isolated, but the same molecule, separated from the remaining part of the genome, as a result of, e.g., a cloning event, amplification, or enrichment, is "isolated." Typically, an isolated nucleic acid molecule is free from nucleic acid regions (e.g., coding regions) with which it is immediately contiguous, at the 5' or 3' ends, in the naturally occurring genome. Such isolated nucleic acid molecules can be part of a vector or a composition and still be isolated, as such a vector or composition is not part of its natural environment.

As used herein, "linked" means attached or bound by covalent bonds, non-covalent bonds, and/or linked via Van der Waals forces, hydrogen bonds, and/or other intermolecular forces.

The term "magnetic particle" refers to particles including materials of high positive magnetic susceptibility such as paramagnetic compounds, superparamagnetic compounds, and magnetite, gamma ferric oxide, or metallic iron.

As used herein, "nonspecific reversibility" refers to the colloidal stability and robustness of magnetic particles against non-specific aggregation in a liquid sample and can be determined by subjecting the particles to the intended assay conditions in the absence of a specific clustering moiety (i.e., an analyte or an agglomerator). For example, nonspecific reversibility can be determined by measuring the T₂ values of a solution of magnetic particles before and after incubation in a uniform magnetic field (defined as <5000 ppm) at 0.45T for 3 minutes at 37°C. Magnetic particles are deemed to have nonspecific reversibility if the difference in T₂ values before and after subjecting the magnetic particles to the intended assay conditions vary by less than 10% (e.g., vary by less than 9%, 8%, 6%, 4%, 3%, 2%, or 1%). If the difference is greater than 10%, then the particles exhibit irreversibility in the buffer, diluents, and matrix tested, and manipulation of particle and matrix properties (e.g., coating and buffer formulation) may be required to produce a system in which the particles have nonspecific reversibility. In another example, the test can be applied by measuring the T₂ values of a solution of magnetic particles before and after incubation in a gradient magnetic field 1 Gauss/mm-10000 Gauss/mm.

As used herein, the term "NMR relaxation rate" refers to a measuring any of the following in a sample T₁, T₂, T₁/T₂ hybrid, T₁ᵣₕₒ, T₂ᵣₕₒ, and T₂*. The methods of the invention are designed to produce an NMR relaxation rate characteristic of whether an analyte is present in the liquid sample. In some instances the NMR relaxation rate is characteristic of the quantity of analyte present in the liquid sample.

As used herein, the term "T₁/T₂ hybrid" refers to any detection method that combines a T₁ and a T₂ measurement. For example, the value of a T₁/T₂ hybrid can be a composite signal obtained through the combination of, ratio, or difference between two or more different T₁ and T₂ measurements. The T₁/T₂ hybrid can be obtained, for example, by using a pulse sequence in which T₁ and T₂ are alternatively measured or acquired in an interleaved fashion. Additionally, the T₁/T₂ hybrid signal can be acquired with a pulse sequence that measures a relaxation rate that is comprised of both T₁ and T₂ relaxation rates or mechanisms.

A "pathogen" means an agent causing disease or illness to its host, such as an organism or infectious particle, capable of producing a disease in another organism, and includes but is not limited to bacteria, viruses, protozoa, prions, yeast and fungi, or pathogen by-products. "Pathogen by-products" are those biological substances arising from the pathogen that can be deleterious to the host or stimulate an excessive host immune response, for example pathogen antigen(s), metabolic substances, enzymes, biological substances, or toxins. By "pathogen-associated analyte" is meant an analyte characteristic of the presence of a pathogen (e.g., a bacterium, fungus, or virus) in a sample. The pathogen-associated analyte can be a particular substance derived from a pathogen (e.g., a protein, nucleic acid, lipid, polysaccharide, or any other material produced by a pathogen) or a mixture derived from a pathogen (e.g., whole cells, or whole viruses). In certain instances, the pathogen-associated analyte is selected to be characteristic of the genus, species, or specific strain of pathogen being detected. Alternatively, the pathogen-associated analyte is selected to ascertain a property of the pathogen, such as resistance to a particular therapy. In some embodiments, a pathogen-associated analyte may be a target nucleic acid that has been amplified. In other embodiments, a pathogen-associated analyte may be a host antibody or other immune system protein that is expressed in response to an infection by a pathogen (e.g., an IgM antibody, an IgA antibody, an IgG antibody, or a major histocompatibility complex (MHC) protein).

A "genus," as used herein, refers to a grouping of organisms, including pathogens. In some embodiments, a genus may be a taxonomic classification, for instance, a taxonomic domain, a taxonomic kingdom, a taxonomic phylum, a taxonomic class, a taxonomic order, a taxonomic family, or a taxonomic genus. In other embodiments, a genus may be defined by any desired or suitable characteristics such as, for example, resistance to an antimicrobial agent or gram staining. It is to be understood that, in some instances, a pathogen may belong to more than one genus.

The term "species," as used herein, refers to a basic unit of biological classification as well as a taxonomic rank. A skilled artisan appreciates that a species may be defined based on a number of criteria, including, for example, DNA similarity, morphology, and ecological niche. The term encompasses any suitable species concept, including evolutionary species, phylogenetic species, typological species, genetic species, and reproductive species. The term also encompasses subspecies or strains.

By "pulse sequence" or "RF pulse sequence" is meant one or more radio frequency pulses to be applied to a sample and designed to measure, e.g., certain NMR relaxation rates, such as spin echo sequences. A pulse sequence may also include the acquisition of a signal following one or more pulses to minimize noise and improve accuracy in the resulting signal value.

As used herein, the term "signal" refers to an NMR relaxation rate, frequency shift, susceptibility measurement, diffusion measurement, or correlation measurements.

As used herein, reference to the "size" of a magnetic particle refers to the average diameter for a mixture of the magnetic particles as determined by microscopy, light scattering, or other methods.

A "subject" is an animal, preferably a mammal (including, for example, rodents (e.g., mice or rats), farm animals (e.g., cows, sheep, horses, and donkeys), pets (e.g., cats and dogs), or primates (e.g., non-human primates and humans)). In particular embodiments, the subject is a human. A subject may be a patient (e.g., a patient having or suspected of having a disease associated with or caused by a pathogen).

As used herein, the term "substantially monodisperse" refers to a mixture of magnetic particles having a polydispersity in size distribution as determined by the shape of the distribution curve of particle size in light scattering measurements. The FWHM (full width half max) of the particle distribution curve less than 25% of the peak position is considered substantially monodisperse. In addition, only one peak should be observed in the light scattering experiments and the peak position should be within one standard deviation of a population of known monodisperse particles.

By "T₂ relaxivity per particle" is meant the average T₂ relaxivity per particle in a population of magnetic particles.

As used herein, "unfractionated" refers to an assay in which none of the components of the sample being tested are removed following the addition of magnetic particles to the sample and prior to the NMR relaxation measurement.

It is contemplated that methods of the claimed invention encompass variations and adaptations developed using information from the embodiments described herein. Throughout the description, where units and systems are described as having, including, or including specific components, or where processes and methods are described as having, including, or including specific steps, it is contemplated that, additionally, there are units and systems that consist essentially of, or consist of, the recited components, and that there are processes and methods according to the present invention that consist essentially of, or consist of, the recited processing steps. It should be understood that the order of steps or order for performing certain actions is immaterial, unless otherwise specified, so long as the invention remains operable. Moreover, in many instances two or more steps or actions may be conducted simultaneously.

### Magnetic Particles and NMR-based Detection

The methods of the invention may involve use of magnetic particles and NMR. The magnetic particles can be coated with a binding moiety (e.g., oligonucleotide, antibody, etc.) such that in the presence of analyte, or multivalent binding agent, aggregates are formed. Aggregation depletes portions of the sample from the microscopic magnetic non-uniformities that disrupt the solvent's T₂ signal, leading to an increase in T₂ relaxation (see, e.g., Figure 3 of International Patent Application Publication No. WO 2012/054639).

The T₂ measurement is a single measure of all spins in the ensemble, measurements lasting typically 1-10 seconds, which allows the solvent to travel hundreds of microns, a long distance relative to the microscopic non-uniformities in the liquid sample. Each solvent molecule samples a volume in the liquid sample and the T₂ signal is an average (net total signal) of all (nuclear spins) on solvent molecules in the sample; in other words, the T₂ measurement is a net measurement of the entire environment experienced by a solvent molecule, and is an average measurement of all microscopic non-uniformities in the sample.

The observed T₂ relaxation rate for the solvent molecules in the liquid sample is dominated by the magnetic particles, which in the presence of a magnetic field form high magnetic dipole moments. In the absence of magnetic particles, the observed T₂ relaxation rates for a liquid sample are typically long (i.e., T₂ (water) = approximately 2000 ms, T₂ (blood) = approximately 1500 ms). As particle concentration increases, the microscopic non-uniformities in the sample increase and the diffusion of solvent through these microscopic non-uniformities leads to an increase in spin decoherence and a decrease in the T₂ value. The observed T₂ value depends upon the particle concentration in a non-linear fashion, and on the relaxivity per particle parameter.

In the aggregation assays, the number of magnetic particles, and if present the number of agglomerant particles, remain constant during the assay. The spatial distribution of the particles changes when the particles cluster. Aggregation changes the average "experience" of a solvent molecule because particle localization into clusters is promoted rather than more even particle distributions. At a high degree of aggregation, many solvent molecules do not experience microscopic non-uniformities created by magnetic particles and the T₂ approaches that of solvent. As the fraction of aggregated magnetic particles increases in a liquid sample, the observed T₂ is the average of the non-uniform suspension of aggregated and single (unaggregated) magnetic particles. The assays are designed to maximize the change in T₂ with aggregation to increase the sensitivity of the assay to the presence of analytes, and to differences in analyte concentration.

In some embodiments, the methods of the invention involve contacting a solution (e.g., a biological sample) with between from 1×10⁶ to 1×10¹³ magnetic particles per milliliter of the liquid sample (e.g., from 1×10⁶to 1×10⁸, 1×10⁷ to 1×10⁸, 1×10⁷ to 1×10⁹, 1×10⁸ to 1×10¹⁰, 1×10⁹ to 1×10¹¹, or 1×10¹⁰ to 1×10¹³ magnetic particles per milliliter).

In some embodiments, the magnetic particles used in the methods of the invention have a mean diameter of from 150 nm to 1200 nm (e.g., from 150 nm to 250 nm, 200 nm to 350 nm, 250 nm to 450 nm, 300 nm to 500 nm, 450 nm to 650 nm, 500 nm to 700 nm, 700 nm to 850 nm, 800 nm to 950 nm, 900 nm to 1050 nm, or from 1000 nm to 1200 nm). For example, in some embodiments, the magnetic particles used in the methods of the invention may have a mean diameter of from 150 nm to 699 nm (e.g., from 150 nm to 250 nm, 200 nm to 350 nm, 250 nm to 450 nm, 300 nm to 500 nm, 450 nm to 650 nm, or from 500 nm to 699 nm). In other embodiments, the magnetic particles used in the methods of the invention may have a mean diameter of from 700 nm to 1200 nm (e.g., from 700 nm to 850 nm, 800 nm to 950 nm, 900 nm to 1050 nm, or from 1000 nm to 1200 nm). In particular embodiments, the magnetic particles may have a mean diameter of from 700 nm to 950 nm (e.g., from 700 nm to 750 nm, 700 nm to 800 nm, 700 nm to 850 nm, or from 700 nm to 900 nm).

In some embodiments, the magnetic particles used in the methods of the invention may have a T₂ relaxivity per particle of from 1×10⁸ to 1×10¹² mM⁻¹s⁻¹ (e.g., from 1×10⁸ to 1×10⁹ mM⁻¹s⁻¹, 1×10⁸ to 1×10¹⁰ mM⁻¹s⁻¹, 1×10⁹ to 1×10¹⁰ mM⁻¹s⁻¹, 1×10⁹ to 1×10¹¹ mM⁻¹s⁻¹, or from 1×10¹⁰ to 1×10¹² mM⁻¹s⁻¹). In some embodiments, the magnetic particles have a T₂ relaxivity per particle of from 1×10⁹ to 1×10¹² mM⁻¹s⁻¹ (e.g., from 1×10⁹ to 1×10¹⁰ mM⁻¹s⁻¹, 1×10⁹ to 1×10¹¹ mM⁻¹s⁻¹, or from 1×10¹⁰ to 1×10¹² mM⁻¹s⁻¹).

In some embodiments, the magnetic particles may be substantially monodisperse. In some embodiments, the magnetic particles in a liquid sample (e.g., a biological sample such as whole blood) may exhibit nonspecific reversibility in the absence of the one or more analytes and/or multivalent binding agent. In some embodiments, the magnetic particles may further include a surface decorated with a blocking agent selected from albumin, fish skin gelatin, gamma globulin, lysozyme, casein, peptidase, and an amine-bearing moiety (e.g., amino polyethyleneglycol, glycine, ethylenediamine, or amino dextran.

### Medical conditions

The methods of the invention can also be used to monitor and diagnose diseases and other medical conditions and to identify improved therapeutic regimens based on the diagnosis, for instance, based on AST results. In several embodiments, the methods of the invention may be used to monitor and diagnose disease in a multiplexed, automated, no sample preparation system.

The methods of the invention can be used to identify and monitor the pathogenesis of disease in a subject, to select therapeutic interventions, and to monitor the effectiveness of the selected treatment. For example, for a patient having or at risk of an infectious disease, for example, bloodstream infection (e.g., bacteremia or fungemia) and/or sepsis, the methods of the invention can be used to identify the infectious pathogen, pathogen load, and to monitor white blood cell count and/or biomarkers indicative of the status of the infection. The identity of the pathogen (e.g., a genus to which the pathogen belongs or, more specifically, the species) can be used to select an appropriate therapy, for example, using AST, using methods described herein and/or known in the art. Administering a therapeutic agent may follow monitoring or diagnosing an infectious disease. The therapeutic intervention (e.g., a particular antimicrobial agent) can be monitored as well to correlate the treatment regimen to the circulating concentration of antimicrobial agent and pathogen load to ensure that the patient is responding to treatment. In some embodiments, antimicrobial resistance markers (e.g., antimicrobial resistance genes) may be monitored following therapeutic intervention.

Exemplary diseases that can be diagnosed and/or monitored by the methods of the invention include diseases caused by or associated with microbial pathogens (e.g., bacterial infection, fungal infection, viral infection, protozoan infection, Lyme disease, bloodstream infection (e.g., bacteremia, fungemia, or viremia), pneumonia, peritonitis, osteomyeletis, meningitis, empyema, urinary tract infection, sepsis, septic shock, and septic arthritis) and diseases that may manifest with similar symptoms to diseases caused by or associated with microbial pathogens (e.g., SIRS). For example, the methods of the invention may be used to diagnose and/or monitor a disease caused by the following pathogens.

In some embodiments, the disease is caused by a bacterial pathogen, including Gram-positive bacteria (e.g., Gram-positive anaerobic bacteria), Gram-negative bacteria (e.g., Gram-negative anaerobic bacteria), *Enterobacteriaceae* spp., *Acinetobacter* spp. (e.g., *Acinetobacter baumannii), Enterococcus* spp. (e.g., *Enterococcus faecium* and *Enterococcus faecalis), Klebsiella* spp. (e.g., *Klebsiella pneumoniae), Pseudomonas* spp. (e.g., *Pseudomonas aeruginosa), Staphylococcus* spp. (including, e.g., coagulase-positive species (e.g., *Staphylococcus aureus)* and coagulase-negative (CoNS) species), *Streptococcus* spp. (e.g., β-hemolytic streptococci, *Streptococcus mitis, Streptococcus pneumoniae, Streptococcus agalactiae,* and *Streptococcus pyogenes), Escherichia* spp. (e.g., *Escherichia coli), Stenotrophomonas* spp. (e.g., *Stenotrophomonas maltophilia), Proteus* spp. (e.g., *Proteus mirabilis* and *Proteus vulgaris), Serratia* spp. (e.g., *Serratia marcescens), Citrobacter* spp. (e.g., *Citrobacter freundii), Enterobacter* spp. (e.g., *Enterobacter aerogenes* and *Enterobacter cloacae), Borrelia* spp. (e.g., *Borrelia burgdorferi, Borrelia afzelii,* and *Borrelia garinii), Rickettsia* spp. (e.g., *Rickettsia rickettsii), Anaplasma* spp. (e.g., *Anaplasma phagocytophilum), Coxiella* spp. (e.g., *Coxiella burnetii*), *Ehrlichia* spp. (e.g., *Ehrlichia chaffeensis* and *Ehrlichia ewingii*), *Franciscella* spp. (e.g., *Francisella tularensis), Clostridium* spp. (e.g., *Clostridium botulinum, Clostridium difficile, Clostridium perfringens,* and *Clostridium tetani), Bacteroides* spp. (e.g., *Bacteroides fragilis),* and *Neisseria* spp. (e.g., *Neisseria meningitides).* In other embodiments, the disease is caused by a fungal pathogen, including *Candida* spp. (e.g., *Candida albicans, Candida guilliermondii, Candida glabrata, Candida krusei, Candida lusitaniae, Candida parapsilosis,* and *Candida tropicalis), Saccharomyces* spp., *Aspergillus* spp. (e.g., *Aspergillus fumigatus, Aspergillus clavatus,* and *Aspergillus flavus),* and *Cryptococcus* spp. (e.g., *Cryptococcus neoformans, Cryptococcus laurentii,* and *Cryptococcus albidus).* In yet other embodiments, the disease is caused by a protozoan pathogen, including *Babesia* spp. (e.g., *Babesia microti* and *Babesia divergens).* In some embodiments, the disease is caused by a pathogen described in Pien et al. Am. J. Med. 123:819-829, 2010.

### Analytes

Embodiments of the invention include methods for detecting and/or measuring the concentration of one or more analytes. In several embodiments, the analyte may be a nucleic acid derived from an organism (e.g., DNA or RNA (e.g., mRNA)). In some embodiments, the nucleic acid is DNA. In other embodiments, the nucleic acid is RNA (e.g., mRNA). In some embodiments, the nucleic acid is a target nucleic acid derived from the organism that has been amplified to form an amplicon.

In some embodiments, the analyte may be derived from a microbial pathogen. For instance, pathogen-associated analytes may include or be derived from a bacterial pathogen including Gram-positive bacteria (e.g., Gram-positive anaerobic bacteria), Gram-negative bacteria (e.g., Gram-negative anaerobic bacteria), *Enterobacteriaceae* spp., *Acinetobacter* spp. (e.g., *Acinetobacter baumannii), Enterococcus* spp. (e.g., *Enterococcus faecium* and *Enterococcus faecalis), Klebsiella* spp. (e.g., *Klebsiella pneumoniae), Pseudomonas* spp. (e.g., *Pseudomonas aeruginosa), Staphylococcus* spp. (including, e.g., coagulase-positive species (e.g., *Staphylococcus aureus)* and coagulase-negative (CoNS) species), *Streptococcus* spp. (e.g., β-hemolytic streptococci, *Streptococcus mitis, Streptococcus pneumoniae, Streptococcus agalactiae,* and *Streptococcus pyogenes), Escherichia* spp. (e.g., *Escherichia coli), Stenotrophomonas* spp. (e.g., *Stenotrophomonas maltophilia), Proteus* spp. (e.g., *Proteus mirabilis* and *Proteus vulgaris), Serratia* spp. (e.g., *Serratia marcescens), Citrobacter* spp. (e.g., *Citrobacter freundii), Enterobacter* spp. (e.g., *Enterobacter aerogenes* and *Enterobacter cloacae), Borrelia* spp. (e.g., *Borrelia burgdorferi, Borrelia afzelii,* and *Borrelia garinii), Rickettsia* spp. (e.g., *Rickettsia rickettsii), Anaplasma* spp. (e.g., *Anaplasma phagocytophilum), Coxiella* spp. (e.g., *Coxiella burnetii*), *Ehrlichia* spp. (e.g., *Ehrlichia chaffeensis* and *Ehrlichia ewingii*), *Franciscella* spp. (e.g., *Francisella tularensis), Clostridium* spp. (e.g., *Clostridium botulinum, Clostridium difficile, Clostridium perfringens,* and *Clostridium tetani), Bacteroides* spp. (e.g., *Bacteroides fragilis),* and *Neisseria* spp. (e.g., *Neisseria meningitides).* In other embodiments, pathogen-associated analytes may include or be derived from a fungal pathogen, including *Candida* spp. (e.g., *Candida albicans, Candida guilliermondii, Candida glabrata, Candida krusei, Candida lusitaniae, Candida parapsilosis,* and *Candida tropicalis), Saccharomyces* spp., *Aspergillus* spp. (e.g., *Aspergillus fumigatus, Aspergillus clavatus,* and *Aspergillus flavus),* and *Cryptococcus* spp. (e.g., *Cryptococcus neoformans, Cryptococcus laurentii,* and *Cryptococcus albidus).* In yet other embodiments, pathogen-associated analytes may include or be derived from a protozoan pathogen, including *Babesia* spp. (e.g., *Babesia microti* and *Babesia divergens).* In still further embodiments, pathogen-associated analytes may include or be derived from a viral pathogen. In some embodiments, pathogen-associated analytes may include or be derived from a pathogen associated with bloodstream infection (e.g., bacteremia or fungemia), invasive bacterial infection, pneumonia, peritonitis, osteomyeletis, meningitis, empyema, urinary tract infection, sepsis, septic shock, septic arthritis, SIRS, or Lyme disease. In some embodiments, the pathogen-associated analyte includes or is derived from a pathogen described in Pien et al. Am. *J. Med. supra.*

In some embodiments, the analyte is characteristic of a genus of pathogens. For instance, in some embodiments, the analyte may be characteristic of bacterial pathogens, fungal pathogens, viral pathogens, or protozoan pathogens. In another example, in the case of bacterial pathogens, the analyte may be characteristic of Gram-positive bacteria, Gram-negative bacteria, *Enterobacteriaceae* spp., *Acinetobacter* spp., *Enterococcus* spp., *Klebsiella* spp., *Pseudomonas* spp., *Staphylococcus* spp., *Streptococcus* spp., *Escherichia* spp., *Stenotrophomonas* spp., *Proteus* spp., *Serratia* spp., *Citrobacter* spp., *Enterobacter* spp., *Borrelia* spp., *Rickettsia* spp., *Anaplasma* spp., *Coxiella* spp., *Ehrlichia* spp., *Franciscella* spp., *Clostridium* spp., *Bacteroides* spp. *Neisseria* spp., or other groupings of bacterial pathogens recognized in the art, for instance, β-hemolytic streptococci, fastidious Gram-negative bacteria, and the like.

In other instances, the analyte is characteristic of a particular pathogen species. For example, in some embodiments, the analyte may be characteristic of any of the pathogen species described above or that is known in the art (e.g., any species listed in Pien et al., *supra).*

In some embodiments, a pathogen-associated analyte may be a nucleic acid derived from any of the organisms described above. In some embodiments, the nucleic acid is a target nucleic acid derived from the organism that has been amplified to form an amplicon. In some embodiments, the nucleic acid is DNA. In other embodiments, the nucleic acid is RNA (e.g., mRNA). In some embodiments, the target nucleic acid may be a multi-copy locus. Use of a target nucleic acid derived from a multi-copy locus, in particular in methods involving amplification, may lead to an increase in sensitivity in the assay. Exemplary multi-copy loci may include, for example, ribosomal DNA (rDNA) operons and multi-copy plasmids. In other embodiments, the target nucleic acid may be a single-copy locus. In particular embodiments, the target nucleic acid may be derived from an essential locus, for example, an essential house-keeping gene. In particular embodiments, the target nucleic acid may be derived from a locus that is involved in virulence (e.g., a virulence gene). In some instances, the target nucleic acid may be derived from an antibiotic resistance gene (e.g., an inducible antibiotic resistance gene). Any suitable antibiotic resistance gene known in the art or described herein may be used. In any of the above embodiments, a locus may include a gene and/or an intragenic region, for example, an internally transcribed sequence (ITS) between rRNA genes (e.g., ITS1, between the 16S and 23S rRNA genes, or ITS2, between the 5S and 23S rRNA genes).

In some embodiments, a target nucleic acid may be (a) genus-specific, (b) genus-inclusive (in other words, present in all species in a given genus), (c) compatible with an amplification/detection protocol, and/or (d) present in multiple copies). In some embodiments, a target nucleic acid may be (a) species-specific, (b) species-inclusive (in other words, present in all strains or subspecies of a given species), (c) compatible with an amplification/detection protocol, and/or (d) present in multiple copies. In particular embodiments, a target nucleic acid is chromosomally-encoded, which can help avoid loss by, for example, plasmid exchange and plasmid curing/transduction events.

In some embodiments, a target nucleic acid may allow for identification of a genus to which the pathogen belongs without allowing for identification of the particular species. In other embodiments, a target nucleic acid allows for identification of the particular species of the pathogen, which will typically identify the genus as well.

In some embodiments, a target nucleic acid may be a control nucleic acid. Such a control nucleic acid may serve as a reference for detection of a pathogen.

### Acinetobacter target nucleic acids

In some embodiments, a target nucleic acid may include sequence elements that are specific for an *Acinetobacter* spp., for example, *Acinetobacter baumannii.* For example, in some embodiments, an *Acinetobacter baumannii* target nucleic acid may be amplified in the presence of a forward primer and a reverse primer which are specific to *Acinetobacter baumannii,* as described below. Detection of such a target nucleic acid in a sample would typically indicate that an *Acinetobacter baumannii* bacterium was present in the sample. In other embodiments, a target nucleic acid may include sequence elements that are common to all *Acinetobacter* spp. For example, in some embodiments, an *Acinetobacter* spp. target nucleic acid may be amplified in the presence of a forward primer and a reverse primer, each of which is universal to all *Acinetobacter* spp. Detection of such a target nucleic acid in a sample typically would indicate that an *Acinetobacter* spp. bacterium was present in the sample. In yet other embodiments, these approaches may be combined.

In some embodiments, an *Acinetobacter* spp. target nucleic acid may be derived from a linear chromosome or a linear or circular plasmid (e.g., a single-, low-, or multi-copy plasmid). In some embodiments, an *Acinetobacter* spp. target nucleic acid may be derived from an essential locus (e.g., an essential housekeeping gene) or a locus involved in virulence (e.g., a gene essential for virulence). In some embodiments, an *Acinetobacter* spp. target nucleic acid may be derived from a multi-copy locus. In other embodiments, an *Acinetobacter* spp. target nucleic acid may be derived from a multi-copy plasmid.

In some embodiments, an *Acinetobacter baumannii* target nucleic acid is derived from a region that includes parts or all of the internally transcribed sequence (ITS) between the 5S and 23S rRNA genes (i.e., the ITS2 region). In particular embodiments, an *Acinetobacter baumannii* target nucleic acid may be amplified in the presence of a forward primer that includes the oligonucleotide sequence 5'-CGT TTT CCA AAT CTG TAA CAG ACT GGG-3' (SEQ ID NO: 1) or 5'-GGA AGG GAT CAG GTG GTT CAC TCT T-3' (SEQ ID NO: 69) and a reverse primer that includes the oligonucleotide sequence 5'- AGG ACG TTG ATA GG TTG GAT GTG GA-3' (SEQ ID NO: 2). For example, in particular embodiments, an *Acinetobacter baumannii* target nucleic acid may be amplified in the presence of a forward primer that includes the oligonucleotide sequence 5'-GGA AGG GAT CAG GTG GTT CAC TCT T-3' (SEQ ID NO: 69) and a reverse primer that includes the oligonucleotide sequence 5'- AGG ACG TTG ATA GG TTG GAT GTG GA-3' (SEQ ID NO: 2). In some embodiments, an amplicon produced using these primers is detected by hybridization using a 5' capture probe that includes the oligonucleotide sequence 5'-TGA GGC TTG ACT ATA CAA CAC C-3' (SEQ ID NO: 15) and/or a 3' capture probe that includes the oligonucleotide sequence 5'- CTA AAA TGA ACA GAT AAA GTA AGA TTC AA-3' (SEQ ID NO: 16) to detect the presence of *Acinetobacter baumannii* in a biological sample. In some embodiments, an amplicon produced using these primers is detected by hybridization using a 5' capture probe that includes the oligonucleotide sequence of SEQ ID NO: 17 and/or a 3' capture probe that includes the oligonucleotide sequence of SEQ ID NO: 18 to detect the presence of *Acinetobacter baumannii* in a biological sample. In some embodiments, the 5' capture probe and/or the 3' capture probe is conjugated to a magnetic nanoparticle.

In some embodiments, a control target nucleic acid for A. *baumannii* may comprise the nucleic acid sequence of SEQ ID NO: 45.

### Enterococcus target nucleic acids

In some embodiments, a target nucleic acid may include sequence elements that are specific for an *Enterococcus* spp., for example, *Enterococcus faecium* or *Enterococcus faecalis.* For example, in some embodiments, an *Enterococcus faecium* target nucleic acid may be amplified in the presence of a forward primer and a reverse primer which are specific to *Enterococcus faecium.* Detection of such a target nucleic acid in a sample would typically indicate that an *Enterococcus faecium* bacterium was present in the sample. In other embodiments, a target nucleic acid may include sequence elements that are specific for multiple (e.g., 2, 3, 4, or 5) *Enterococcus* spp. For example, in some embodiments, a target nucleic acid may include sequence elements that are specific for *Enterococcus faecium* and *Enterococcus faecalis,* as described below. In other embodiments, a target nucleic acid may include sequence elements that are common to all *Enterococcus* spp. For example, in some embodiments, an *Enterococcus* spp. target nucleic acid may be amplified in the presence of a forward primer and a reverse primer, each of which is universal to all *Enterococcus* spp. Detection of such a target nucleic acid in a sample typically would indicate that an *Enterococcus* spp. bacterium was present in the sample. In yet other embodiments, these approaches may be combined.

In some embodiments, an *Enterococcus* spp. target nucleic acid may be derived from a linear chromosome or a linear or circular plasmid (e.g., a single-, low-, or multi-copy plasmid). In some embodiments, an *Enterococcus* spp. target nucleic acid may be derived from an essential locus (e.g., an essential housekeeping gene) or a locus involved in virulence (e.g., a gene essential for virulence). In some embodiments, an *Enterococcus* spp. target nucleic acid may be derived from a multi-copy locus. In particular embodiments, an *Enterococcus* spp. target nucleic acid may be derived from a multi-copy plasmid.

In some embodiments, an *Enterococcus* spp. target nucleic acid is derived from a region that includes parts or all of the ITS between the 23S and 5S rRNA genes. In particular embodiments, an target nucleic acid that is specific for *Enterococcus faecium* and *Enterococcus faecalis* may be amplified in the presence of a forward primer that includes the oligonucleotide sequence 5'-GGT AGC TAT GTA GGG AAG GGA TAA ACG CTG A-3' (SEQ ID NO: 3) and a reverse primer that includes the oligonucleotide sequence 5'-GCG CTA AGG AGC TTA ACT TCT GTG TTC G-3' (SEQ ID NO: 4). In some embodiments, an amplicon produced using these primers is detected by hybridization using a 5' capture probe that includes the oligonucleotide sequence 5'-AAA ACT TAT ATG ACT TCA AAT CCA GTT TT-3' (SEQ ID NO: 19) or 5'-AAA ACT TAT GTG ACT TCA AAT CCA GTT TT-3' (SEQ ID NO: 70) and/or a 3' capture probe that includes the oligonucleotide sequence 5'-TTT ACT CAA TAA AAG ATA ACA CCA CAG-3' (SEQ ID NO: 20) or 5'-TTT ACT CAA TAA AAG ATA ACA CCA CAG T-3' (SEQ ID NO 71) to detect the presence of *Enterococcus faecium* in a biological sample. In particular embodiments, an amplicon produced using these primers is detected by hybridization using a 5' capture probe that includes the oligonucleotide sequence 5'-AAA ACT TAT GTG ACT TCA AAT CCA GTT TT-3' (SEQ ID NO: 70) and/or a 3' capture probe that includes the oligonucleotide sequence 5'-TTT ACT CAA TAA AAG ATA ACA CCA CAG T-3' (SEQ ID NO: 71) to detect the presence of *Enterococcus faecium* in a biological sample. In some embodiments, an amplicon produced using these primers is detected by hybridization using a 5' capture probe that includes the oligonucleotide sequence of SEQ ID NO: 21 and/or a 3' capture probe that includes the oligonucleotide sequence of SEQ ID NO: 22 to detect the presence of *Enterococcus faecium* in a biological sample. In some embodiments, an amplicon produced using these primers is detected by hybridization using a 5' capture probe that includes the oligonucleotide sequence 5'-TGG ATA AGT AAA AGC AAC TTG GTT-3' (SEQ ID NO: 23) and/or a 3' capture probe that includes the oligonucleotide sequence 5'-AAT GAA GAT TCA ACT CAA TAA GAA ACA ACA-3' (SEQ ID NO: 24) to detect the presence of *Enterococcus faecalis* in a biological sample. In some embodiments, an amplicon produced using these primers is detected by hybridization using a 5' capture probe that includes the oligonucleotide sequence of SEQ ID NO: 25 and/or a 3' capture probe that includes the oligonucleotide sequence of SEQ ID NO: 26 to detect the presence of *Enterococcus faecalis* in a biological sample. In some embodiments, the 5' capture probe and/or the 3' capture probe is conjugated to a magnetic nanoparticle.

In some embodiments, a control target nucleic acid for *Enterococcus faecium* may comprise the nucleic acid sequence of SEQ ID NO: 46. In other embodiments, a control target nucleic acid for *Enterococcus faecium* may comprise the nucleic acid sequence of SEQ ID NO: 77. In some embodiments, a control target nucleic acid for *Enterococcus faecalis* may comprise the nucleic acid sequence of SEQ ID NO: 47.

### Klebsiella target nucleic acids

In some embodiments, a target nucleic acid may include sequence elements that are specific for a *Klebsiella* spp., for example, *Klebsiella pneumoniae.* For example, in some embodiments, a *Klebsiella pneumoniae* target nucleic acid may be amplified in the presence of a forward primer and a reverse primer which are specific to *Klebsiella pneumoniae,* as described below. Detection of such a target nucleic acid in a sample would typically indicate that a *Klebsiella pneumoniae* bacterium was present in the sample. In other embodiments, a target nucleic acid may include sequence elements that are common to all *Klebsiella* spp. For example, in some embodiments, a *Klebsiella* spp. target nucleic acid may be amplified in the presence of a forward primer and a reverse primer, each of which is universal to all *Klebsiella* spp. Detection of such a target nucleic acid in a sample typically would indicate that a *Klebsiella* spp. bacterium was present in the sample. In yet other embodiments, these approaches may be combined.

In some embodiments, a *Klebsiella* spp. target nucleic acid may be derived from a linear chromosome or a linear or circular plasmid (e.g., a single-, low-, or multi-copy plasmid). In some embodiments, a *Klebsiella* spp. target nucleic acid may be derived from an essential locus (e.g., an essential housekeeping gene) or a locus involved in virulence (e.g., a gene essential for virulence). In some embodiments, a *Klebsiella* spp. target nucleic acid may be derived from a multi-copy locus. In particular embodiments, a *Klebsiella* spp. target nucleic acid may be derived from a multi-copy plasmid.

In some embodiments, a *Klebsiella pneumoniae* target nucleic acid is derived from a 23S rRNA gene. In particular embodiments, a *Klebsiella pneumoniae* target nucleic acid may be amplified in the presence of a forward primer that includes the oligonucleotide sequence 5'-GAC GGT TGT CCC GGT TTA AGC A-3' (SEQ ID NO: 5) and a reverse primer that includes the oligonucleotide sequence 5'-GCT GGT ATC TTC GAC TGG TCT-3' (SEQ ID NO: 6). In some embodiments, an amplicon produced using these primers is detected by hybridization using a 5' capture probe that includes the oligonucleotide sequence 5'-TAC CAA GGC GCT TGA GAG AAC TC-3' (SEQ ID NO: 27) and/or a 3' capture probe that includes the oligonucleotide sequence 5'-CTG GTG TGT AGG TGA AGT C-3' (SEQ ID NO: 28) to detect the presence of *Klebsiella pneumoniae* in a biological sample. In some embodiments, an amplicon produced using these primers is detected by hybridization using a 5' capture probe that includes the oligonucleotide sequence of SEQ ID NO: 29 and/or a 3' capture probe that includes the oligonucleotide sequence of SEQ ID NO: 30 to detect the presence of *Klebsiella pneumoniae* in a biological sample. In some embodiments, the 5' capture probe and/or the 3' capture probe is conjugated to a magnetic nanoparticle.

In some embodiments, a control target nucleic acid for *Klebsiella pneumoniae* may comprise the nucleic acid sequence of SEQ ID NO: 48.

### Pseudomonas target nucleic acids

In some embodiments, a target nucleic acid may include sequence elements that are specific for a *Pseudomonas* spp., for example, *Pseudomonas aeruginosa.* For example, in some embodiments, a *Pseudomonas aeruginosa* target nucleic acid may be amplified in the presence of a forward primer and a reverse primer which are specific to *Pseudomonas aeruginosa,* as described below. Detection of such a target nucleic acid in a sample would typically indicate that a *Pseudomonas aeruginosa* bacterium was present in the sample. In other embodiments, a target nucleic acid may include sequence elements that are common to all *Pseudomonas* spp. For example, in some embodiments, a *Pseudomonas* spp. target nucleic acid may be amplified in the presence of a forward primer and a reverse primer, each of which is universal to all *Pseudomonas* spp. Detection of such a target nucleic acid in a sample typically would indicate that a *Pseudomonas* spp. bacterium was present in the sample. In yet other embodiments, these approaches may be combined.

In some embodiments, a *Pseudomonas* spp. target nucleic acid may be derived from a linear chromosome or a linear or circular plasmid (e.g., a single-, low-, or multi-copy plasmid). In some embodiments, a *Pseudomonas* spp. target nucleic acid may be derived from an essential locus (e.g., an essential housekeeping gene) or a locus involved in virulence (e.g., a gene essential for virulence). In some embodiments, a *Pseudomonas* spp. target nucleic acid may be derived from a multi-copy locus. In particular embodiments, a *Pseudomonas* spp. target nucleic acid may be derived from a multi-copy plasmid.

In some embodiments, a *Pseudomonas aeruginosa* target nucleic acid is derived from a region that includes parts or all of the ITS between the 23S and 5S rRNA genes. In particular embodiments, a *Pseudomonas aeruginosa* target nucleic acid may be amplified in the presence of a forward primer that includes the oligonucleotide sequence 5'-AGG CTG GGT GTG TAA GCG TTG T-3' (SEQ ID NO: 7) and a reverse primer that includes the oligonucleotide sequence 5'-CAA GCA ATT CGG TTG GAT ATC CGT T-3' (SEQ ID NO: 8). In some embodiments, an amplicon produced using these primers is detected by hybridization using a 5' capture probe that includes the oligonucleotide sequence 5'-GTG TGT TGT AGG GTG AAG TCG AC-3' (SEQ ID NO: 31) or 5'-TCT GAC GAT TGT GTG TTG TAA GG-3' (SEQ ID NO: 73) and/or a 3' capture probe that includes the oligonucleotide sequence 5'-CAC CTT GAA ATC ACA TAC CTG A-3' (SEQ ID NO: 32) or 5'-GGA TAG ACG TAA GCC CAA GC-3' (SEQ ID NO: 74) to detect the presence of *Pseudomonas aeruginosa* in a biological sample. In particular embodiments, an amplicon produced using these primers is detected by hybridization using a 5' capture probe that includes the oligonucleotide sequence 5'-TCT GAC GAT TGT GTG TTG TAA GG-3' (SEQ ID NO: 73) and/or a 3' capture probe that includes the oligonucleotide 5'-GGA TAG ACG TAA GCC CAA GC-3' (SEQ ID NO: 74) to detect the presence of *Pseudomonas aeruginosa* in a biological sample. In some embodiments, an amplicon produced using these primers is detected by hybridization using a 5' capture probe that includes the oligonucleotide sequence of SEQ ID NO: 33 and/or a 3' capture probe that includes the oligonucleotide sequence of SEQ ID NO: 34 to detect the presence of *Pseudomonas aeruginosa* in a biological sample. In some embodiments, the 5' capture probe and/or the 3' capture probe is conjugated to a magnetic nanoparticle.

In some embodiments, a control target nucleic acid for *Pseudomonas aeruginosa* may comprise the nucleic acid sequence of SEQ ID NO: 49.

### Staphylococcus target nucleic acids

In some embodiments, a target nucleic acid may include sequence elements that are specific for a *Staphylococcus* spp., for example, *Staphylococcus aureus.* For example, in some embodiments, a *Staphylococcus aureus* target nucleic acid may be amplified in the presence of a forward primer and a reverse primer which are specific to *Staphylococcus aureus,* as described below. Detection of such a target nucleic acid in a sample would typically indicate that a *Staphylococcus aureus* bacterium was present in the sample. In other embodiments, a target nucleic acid may include sequence elements that are common to all *Staphylococcus* spp. For example, in some embodiments, a *Staphylococcus* spp. target nucleic acid may be amplified in the presence of a forward primer and a reverse primer, each of which is universal to all *Staphylococcus* spp. Detection of such a target nucleic acid in a sample typically would indicate that a *Staphylococcus* spp. bacterium was present in the sample. In yet other embodiments, these approaches may be combined.

In some embodiments, a *Staphylococcus* spp. target nucleic acid may be derived from a linear chromosome or a linear or circular plasmid (e.g., a single-, low-, or multi-copy plasmid). In some embodiments, a *Staphylococcus* spp. target nucleic acid may be derived from an essential locus (e.g., an essential housekeeping gene), a locus involved in virulence (e.g., a gene essential for virulence), or a gene involved in antibiotic resistance (e.g., femA and femB). In some embodiments, a *Staphylococcus* spp. target nucleic acid may be derived from a multi-copy locus. In particular embodiments, a *Staphylococcus* spp. target nucleic acid may be derived from a multi-copy plasmid.

In some embodiments, a *Staphylococcus aureus* target nucleic acid is derived from the femAB operon. The femAB operon codes for two nearly identical approximately 50 kDa proteins involved in the formation of the Staphylococcal pentaglycine interpeptide bridge in peptidoglycan. These chromosomally-encoded proteins are considered as factors that influence the level of methicillin resistance and as essential housekeeping genes. femB is one gene in the femA/B operon, also referred to as *graR,* the two component response regulator of methicillin resistance. femB encodes a aminoacyltransferase, whereas femA encodes a regulatory factor that is essential for expression of femB and therefore methicillin resistance expression. In some embodiments, a *Staphylococcus aureus* target nucleic acid is derived from the *femA* gene. For example, in particular embodiments, a *Staphylococcus aureus* target nucleic acid may be amplified in the presence of a forward primer that includes the oligonucleotide sequence 5'-GGT AAT GAATTA CCT /i6diPr/TC TCT GCT GGTTTC TTC TT-3' (SEQ ID NO: 9) and a reverse primer that includes the oligonucleotide sequence 5'-ACC AGC ATC TTC /i6diPr/GC ATC TTC TGT AAA-3' (SEQ ID NO: 10). Note that "/i6diPr/" indicates 2,6-Diaminopurine. In some embodiments, an amplicon produced using these primers is detected by hybridization using a 5' capture probe that includes the oligonucleotide sequence 5'-CCA TTT GAA GTT GTT TAT TAT GC-3' (SEQ ID NO: 35) and/or a 3' capture probe that includes the oligonucleotide sequence 5'-GGG AAA TGA TTA ATT ATG CAT TAA ATC-3' (SEQ ID NO: 36) to detect the presence of *Staphylococcus aureus* in a biological sample. In some embodiments, an amplicon produced using these primers is detected by hybridization using a 5' capture probe that includes the oligonucleotide sequence of SEQ ID NO: 37 and/or a 3' capture probe that includes the oligonucleotide sequence of SEQ ID NO: 38 to detect the presence of *Staphylococcus aureus* in a biological sample. In some embodiments, the 5' capture probe and/or the 3' capture probe is conjugated to a magnetic nanoparticle.

In some embodiments, a *Staphylococcus aureus* target nucleic acid is derived from the femB gene. For example, in other particular embodiments, a *Staphylococcus aureus* target nucleic acid may be amplified in the presence of a forward primer that includes the oligonucleotide sequence 5'-GAA GTT ATG TTT /i6diPr/CT ATT CGA ATC GTG GTC CAGT-3' (SEQ ID NO: 11) and a reverse primer that includes the oligonucleotide sequence 5'-GTT GTA AAG CCA TGA TGC TCG TAA CCA-3' (SEQ ID NO: 12). In some embodiments, an amplicon produced using these primers is detected by hybridization using a 5' capture probe that includes the oligonucleotide sequence 5'-TT TTT CAG ATT TAG GAT TAG TTG ATT-3' (SEQ ID NO: 39) and/or a 3' capture probe that includes the oligonucleotide sequence 5'-GAT CCG TAT TGG TTA TAT CAT C-3' (SEQ ID NO: 40) to detect the presence of *Staphylococcus aureus* in a biological sample. In some embodiments, an amplicon produced using these primers is detected by hybridization using a 5' capture probe that includes the oligonucleotide sequence of SEQ ID NO: 41 and/or a 3' capture probe that includes the oligonucleotide sequence of SEQ ID NO: 42 to detect the presence of *Staphylococcus aureus* in a biological sample. In some embodiments, the 5' capture probe and/or the 3' capture probe is conjugated to a magnetic nanoparticle.

In some embodiments, a *Staphylococcus aureus* target nucleic acid includes all or a portion of both the *femA* gene and the femB gene.

In some embodiments, a control target nucleic acid for *Staphylococcus aureus femA* may comprise the nucleic acid sequence of SEQ ID NO: 50. In some embodiments, a control target nucleicacid for *Staphylococcus aureus* femB may comprise the nucleic acid sequence of SEQ ID NO: 51.

### Escherichia target nucleic acids

In some embodiments, a target nucleic acid may include sequence elements that are specific for an *Escherichia* spp., for example, *Escherichia coli.* For example, in some embodiments, an *Escherichia coli* target nucleic acid may be amplified in the presence of a forward primer and a reverse primer which are specific to *Escherichia coli,* as described below. Detection of such a target nucleic acid in a sample would typically indicate that an *Escherichia coli* bacterium was present in the sample. In other embodiments, a target nucleic acid may include sequence elements that are common to all *Staphylococcus* spp. For example, in some embodiments, an *Escherichia* spp. target nucleic acid may be amplified in the presence of a forward primer and a reverse primer, each of which is universal to all *Escherichia* spp. Detection of such a target nucleic acid in a sample typically would indicate that a *Escherichia* spp. bacterium was present in the sample. In yet other embodiments, these approaches may be combined.

In some embodiments, an *Escherichia* spp. target nucleic acid may be derived from a linear chromosome or a linear or circular plasmid (e.g., a single-, low-, or multi-copy plasmid). In some embodiments, an *Escherichia* spp. target nucleic acid may be derived from an essential locus (e.g., an essential housekeeping gene), a locus involved in virulence (e.g., a gene essential for virulence), or a gene involved in antibiotic resistance. In some embodiments, an *Escherichia* spp. target nucleic acid may be derived from a multi-copy locus. In particular embodiments, an *Escherichia* spp. target nucleic acid may be derived from a multi-copy plasmid.

In particular embodiments, an *Escherichia coli* target nucleic acid is derived from the yfcL gene. The yfcL gene is within an E. coli-specific Chaperone-Usher Fimbriae gene cluster (see, e.g., Wurpelet al. PLoS One Vol 8, e52835, 2013). For example, in other particular embodiments, *Escherichia coli* yfcL may be amplified in the presence of a forward primer that includes the oligonucleotide sequence 5'-GCA TTA ATC GAC GGT ATG GTT GAC C-3' (SEQ ID NO: 52) and a reverse primer that includes the oligonucleotide sequence 5'-CCT GCT GAA ACA GGT TTT CCC ACA TA-3' (SEQ ID NO: 53). In some embodiments, an amplicon produced using these primers is detected by hybridization using a 5' capture probe that includes the oligonucleotide sequence 5'-AGT GAT GAT GAG TTG TTT GCC AGT G-3' (SEQ ID NO: 54) and/or a 3' capture probe that includes the oligonucleotide sequence 5'-TGA ATT GTC GCC GCG TGA CCA G-3' (SEQ ID NO: 55) to detect the presence of *Escherichia coli* in a biological sample. In some embodiments, the 5' capture probe and/or the 3' capture probe is conjugated to a magnetic nanoparticle.

### Candida target nucleic acids

In some embodiments, a target nucleic acid may include sequence elements that are specific for a *Candida* spp. (e.g., *Candida albicans, Candida guilliermondii, Candida glabrata, Candida krusei, Candida lusitaniae, Candida parapsilosis,* and *Candida tropicalis*). For example, in some embodiments, a *Candida albicans* target nucleic acid may be amplified in the presence of a forward primer and a reverse primer which are specific to *Candida albicans.* Detection of such a target nucleic acid in a sample would typically indicate that a *Candida albicans* cell was present in the sample. In other embodiments, a target nucleic acid may include sequence elements that are common to all *Candida* spp. For example, in some embodiments, a *Candida* spp. target nucleic acid may be amplified in the presence of a forward primer and a reverse primer, each of which is universal to all *Candida* spp., as described below. Detection of such a target nucleic acid in a sample typically would indicate that a *Candida* spp. cell was present in the sample. In yet other embodiments, these approaches may be combined.

In some embodiments, a *Candida* spp. target nucleic acid may be derived from a linear chromosome or a linear or circular plasmid (e.g., a single-, low-, or multi-copy plasmid). In some embodiments, a *Candida* spp. target nucleic acid may be derived from an essential locus (e.g., an essential housekeeping gene) or a locus involved in virulence (e.g., a gene essential for virulence). In some embodiments, a *Candida* spp. target nucleic acid may be derived from a multi-copy locus. For example, in some embodiments, a *Candida* spp. target nucleic acid may be derived from a ribosomal DNA operon.

Detection of a *Candida* species can be performed as described, for example, in International Patent Application Publication No. WO 2012/054639. In particular embodiments, a *Candida* spp. target nucleic acid may be amplified in the presence of a forward primer that includes the oligonucleotide sequence 5'-GGC ATG CCT GTT TGA GCG TC-3' (SEQ ID NO: 13) and a reverse primer that includes the oligonucleotide sequence 5'-GCT TAT TGA TAT GCT TAA GTT CAG CGG GT-3' (SEQ ID NO: 14). The capture probes listed in Table 1 can be used for detection of an amplicon produced by these primers to identify the presence of the indicated *Candida* species.

**Table 1: Capture Probes for Detection of Candida spp.**

| *Candida* Capture Probes | Sequence |
|---|---|
| *Candida albicans* Probe **#1** | ACC CAG CGG TTT GAG GGA GAA AC (SEQ ID NO: 56) |
| *Candida albicans* Probe **#2** | AAA GTT TGA AGA TAT ACG TGG TGG ACG TTA (SEQ ID NO: 57) |
| *Candida krusei* Probe **#1** | CGC ACG CGC AAG ATG GAA ACG (SEQ ID NO: 58) |
| *Candida krusei* Probe **#2** | AAG TTC AGC GGG TAT TCC TAC CT (SEQ ID NO: 59) |
| *Candida krusei* probe | AGC TTT TTG TTG TCT CGC AAC ACT CGC (SEQ ID NO: 60) |
| *Candida glabrata* Probe **#1** | CTA CCA AAC ACA ATG TGT TTG AGA AG (SEQ ID NO: 61) |
| *Candida glabrata* Probe **#2** | CCT GAT TTG AGG TCA AAC TTA AAG ACG TCT G (SEQ ID NO: 62) |
| *Candida parapsilosis*/*tropicalis* Probe **#1** | AGT CCT ACC TGA TTT GAG GTC Nitlnd¹AA (SEQ ID NO: 63) |
| *Candida parapsilosis*/*tropicalis* Probe **#2** | CCG Nitlnd¹GG GTT TGA GGG AGA AAT (SEQ ID NO: 64) |
| *Candida tropicalis* | AAA GTT ATG AAATAA ATT GTG GTG GCC ACT AGC (SEQ ID NO: 65) |
| *Candida tropicalis* | ACC CGG GGGTTT GAG GGA GAA A (SEQ ID NO: 66) |
| *Candida parapsilosis* | AGT CCT ACC TGA TTT GAG GTC GAA (SEQ ID NO: 67) |
| *Candida parapsilosis* | CCG AGG GTT TGA GGG AGA AAT (SEQ ID NO: 68) |

| | |
|---|---|
| 1. Nitlnd is 5' 5-Nitroindole, a base that is capable of annealing with any of the four DNA bases. | |

In some methods, a *Candida* amplicon produced by amplification of a *Candida* target nucleic acid in the presence of a forward primer comprising the oligonucleotide sequence 5'-GGC ATG CCT GTT TGA GCG TC-3' (SEQ ID NO: 13) and a reverse primer that includes the oligonucleotide sequence 5'-GCT TAT TGA TAT GCT TAA GTT CAG CGG GT-3' (SEQ ID NO: 14) is detected by hybridization a first nucleic acid probe and a second nucleic acid probe conjugated to one or more populations of magnetic particles. For example, certain embodiments, (i) the *Candida* species is *Candida albicans,* the first probe includes the oligonucleotide sequence 5'-ACC CAG CGG TTT GAG GGA GAA AC-3' (SEQ ID NO: 56), and the second probe includes the oligonucleotide sequence 5'-AAA GTT TGA AGA TAT ACG TGG TGG ACG TTA-3' (SEQ ID NO: 57); (ii) the *Candida* species is *Candida krusei* and the first probe and the second probe include an oligonucleotide sequence selected from: 5'-CGC ACG CGC AAG ATG GAA ACG-3' (SEQ ID NO: 58), 5'-AAG TTC AGC GGG TAT TCC TAC CT-3' (SEQ ID NO: 59), and 5'-AGC TTT TTG TTG TCT CGC AAC ACT CGC-3' (SEQ ID NO: 60); (iii) the *Candida* species is *Candida glabrata,* the first probe includes the oligonucleotide sequence: 5'-CTA CCA AAC ACA ATG TGT TTG AGA AG-3' (SEQ ID NO: 61), and the second probe includes the oligonucleotide sequence: 5'-CCT GAT TTG AGG TCA AAC TTA AAG ACG TCT G-3' (SEQ ID NO: 62); and (iv) the Candida species is *Candida parapsilosis* or *Candida tropicalis* and the first probe and the second probe include an oligonucleotide sequence selected from: 5'-AGT CCT ACC TGA TTT GAG GTCNitlndAA-3' (SEQ ID NO: 63), 5'-CCG NitlndGG GTT TGA GGG AGA AAT-3' (SEQ ID NO: 64), 5'-AAA GTT ATG AAATAA ATT GTG GTG GCC ACT AGC-3' (SEQ ID NO: 65), 5'-ACC CGG GGGTTT GAG GGA GAA A-3' (SEQ ID NO: 66), 5'-AGT CCT ACC TGA TTT GAG GTC GAA-3' (SEQ ID NO: 67), and 5'-CCG AGG GTT TGA GGG AGA AAT-3' (SEQ ID NO: 68). In some embodiments, the first probe comprises the oligonucleotide sequence of SEQ ID NO: 43 and the second probe comprises the oligonucleotide sequence of SEQ ID NO: 44.

### Antimicrobial Susceptibility Testing

The invention provides methods that involve determining the susceptibility of a pathogen to one or more antimicrobial agents (e.g., antimicrobial susceptibility testing (AST)) that are based, at least in part, on the rapid and sensitive detection of the presence and identity of a pathogen associated with an infection (e.g., the causative pathogen) by the methods of the invention. In some embodiments, the methods of the invention identify a genus to which the pathogen belongs. In other embodiments, the methods of the invention identify both the genus and the species to which the pathogen belongs. In other embodiments, the methods of the invention identify a genus to which the pathogen belongs without identifying the particular species. These methods are facilitated both by the sensitivity (e.g. 1 CFU/mL sensitivity, or 95% hit rate at 1 CFU/mL), as well as the fast turnaround time (typically about 2-8 h, e.g., about 2 h, about 3h, about 4 h, about 5 h, about 6 h, about 7h, or about 8 h) of the detection methods of the invention. The rapid detection and identification of the pathogen by the methods of the invention in turn facilitates rapid and directed determination of susceptibility of the pathogen to antimicrobial agents.

Following the detection and identification of a pathogen (e.g., at the genus and/or species level) by the methods of the invention, a sample (e.g., a pellet obtained from a biological sample or a culture thereof) of the pathogen is typically processed such that organisms in a sample can be inoculated onto media (e.g., chromogenic media) and/or an AST test system (e.g., an automated system) to provide phenotypic AST results. In some embodiments, AST involves comparing the growth rate of the pathogen in the presence of the antimicrobial agent to the growth rate of the pathogen in the absence of the antimicrobial agent. In other embodiments, AST may involve comparing one or more functions of the pathogen in the presence of the antimicrobial agent compared to the absence of the antimicrobial agent. For example, the function of the pathogen may be the expression of a gene associated with the pathogen (e.g., an antimicrobial resistance gene), an enzymatic activity (e.g., the activity of a protein encoded by an antimicrobial resistance gene, such as carbapenemase), production of a metabolite or toxin, and the like. In some embodiments, AST may involve measuring properties of blood and/or growth media that are affected by the presence of the pathogen, such as electrochemical methods that measure a change in impedance, resistance, capacitance, or voltage in the sample, as the pathogen cultures may be associated with a change in ionic strength, bulk susceptibility, bulk capacitance, or resistance; methods such as those capable of measuring metabolites such as gas chromatography or mass spectrometry that measure changes in the amount or distribution of one or more small molecule or protein metabolites; optical methods that detect cell growth by measuring changes in the light transmittance of the sample; or labeled methods that detect cell growth by means of a labeling moiety such as a fluorescently-labeled nucleic acid, peptide nucleic acid (PNA), antibody, aptamer, or other targeting moiety that allows measurement of the cells directly.

For example, in some instances, the methods of the invention may involve determining the presence and identity of a pathogen (e.g., at the genus and/or species level) in a first portion of the biological sample after having obtained a biological sample from a subject (e.g., a patient suspected to be suffering from a BSI). In some instances, a second portion of the biological sample is incubated in parallel in order to culture the pathogen for downstream AST testing. For example, in embodiments where the biological sample is blood, the method may involve determining the presence and identity of the pathogen (e.g., at the genus and/or species level) in one portion of a blood sample, and in parallel inoculating one or more blood culture bottles to form a pathogen culture for AST testing. Once the presence and identity of the pathogen is determined by the methods of the invention, the blood culture bottles may be sampled immediately for AST testing, or a subculture can be inoculated in a growth media that is more favorable for growth of the pathogen. As one non-limiting example, if the pathogen is identified as a *Candida* spp., the method of the invention may include inoculating a fungal blood culture bottle (e.g., BACTEC™ Myco/F Lytic, BD) and/or use of a lysis centrifugation system (e.g., the ISOLATOR™ lysis centrifugation system, Wampole Laboratories, Cranbury, N.J.) followed by plating onto chromogenic media (e.g., CHROMAGAR™ Candida, Chromagar, Paris, France), chocolate agar, and/or Samouraud Glucose media to encourage growth of *Candida* spp. and early growth for the isolated colonies. The isolated colonies may be subjected to AST using any of the methods described herein. In some embodiments, the inoculated agar plates are used for AST by placing E-TEST® strips or disk diffusion tests on the media.

In other instances, an additional biological sample has been obtained from the subject following determination of the presence and identity of the pathogen (e.g., at the genus and/or species level) in a first biological sample. In some instances, AST may be performed directly in the additional biological sample. In other instances, the additional biological sample is incubated in media suitable for enhanced growth of the identified pathogen. For example, if the identified pathogen is a *Candida* species, the method may involve obtaining another blood draw from the subject and inoculating a fungal blood culture bottle and/or use of a lysis centrifugation system (e.g., the ISOLATOR™ lysis centrifugation system, Wampole Laboratories, Cranbury, NJ) followed by plating onto chromogenic media (e.g., CHROMAGAR™ Candida, Chromagar, Paris, France), chocolate agar and/or Samouraud Glucose media to encourage growth of *Candida* species and early growth of the isolated colonies. The isolated colonies may be subjected to AST using any of the methods described herein. In some embodiments, the inoculated agar plates are used for AST by placing ETEST® strips or disk diffusion tests on the media.

In some embodiments, the methods of the invention may involve addition of an additive to the growth medium of a culture (e.g., a blood culture) of the pathogen in order to enhance growth. For example, in some embodiments, the presence of free heme in a blood sample may retard growth of a microbial pathogen. In some embodiments, a heme detoxicification agent may be added to a blood sample or a subculture thereof, thereby enhancing growth of a pathogen present. In some embodiments, a heme detoxification agent may be an antioxidant, a heme polymerase, a reducing agent, a buffering agent, a free radical scavenger, or an agent that inactivates or eliminates one or more antimicrobial agents. In some embodiments, the method of the invention may involve removal of a component of a growth medium of a culture of the pathogen in order to enhance growth.

In some instances, any of the methods described herein may involve centrifugation, filtration, lysis centrifugation, and/or lysis concentration, which may be used, for example, to concentrate pathogens. In some instances, magnetic separation may be used to concentrate pathogens (see, e.g., Aprodu et al. Int. J. Food. Microbiol. 145(1):S61-S65, 2011). Any suitable approach for lysis centrifugation may be used, including using an ISOLATOR™ lysis centrifugation system. In general, lysis centrifugation may include obtaining a biological sample (e.g., a blood sample) from a subject, placing the sample in a culture tube that includes a lysis agent (e.g., saponin or others described herein) under conditions suitable for lysis of cells in the sample (e.g., red and white blood cells), centrifuging the culture tube to obtain pellets containing a pathogen, removing the supernatant, and placing the pellet on one or more media plates, which may be selected based on the genus and/or species of the pathogen. The media plates may then be incubated under conditions suitable for growth (including enhanced growth) in order to obtain biomass (e.g., colonies), which can be used in AST analysis. In some instances, the one or more media plates may be used for AST, for example, by placing ETEST® strips or disk diffusion tests on the media plates. A number of lysis centrifugation approaches are known in the art (see, e.g., Biéler et al. Acta Tropica 121(2):135-140, 2012 Rossmanith et al. J. Microbiol. Methods 69(3):504-511, 2007; Trovato et al. Clin. Microbiol. Infect. 18:E63-E65, 2012; and Idelevich et al. J. Clin. Microbiol. 55(1):97-100, 2017), and any suitable approach may be used in the context of the invention.

Any suitable method of AST can be used in the methods of the invention. For example, any method of AST described in Jorgensen et al. Clinical Infectious Diseases 49:1749-1755, 2009, may be used in the methods of the invention. For example, in some instances, the methods of the invention may include a broth dilution test, a disk diffusion test, an antimicrobial gradient test, growth on chromogenic media, an enzyme activity assay, and/or an automated instrument. In some instances, the broth diffusion test may include macrobroth or microdilution trays. In some instances, the antimicrobial gradient test may include use of an epsilometer test (e.g., an ETEST®, bioMérieux SA, Marcy-l'Étoile, France).

In some instances, the methods of the invention include growth of the pathogen on chromogenic media. Any suitable chromogenic media may be used. In some embodiments, the chromogenic media is selected based on the identity of the pathogen (e.g., at the genus and/or species level). For example, suitable chromogenic media for *Staphylococcus aureus* (particularly methicillin-resistant S. *aureus,* MRSA) may include BRILLIANCE™ MRSA agar (Oxoid, Basingstoke, United Kingdom), CHROMID™ (bioMérieux), MRSASELECT™ (Bio-Rad, Hercules, CA), CHROMAGAR™ MRSA (Chromagar), and BBL™ CHROMAGAR™ (BD Diagnostics). Suitable chromogenic media for *Enterococcus* spp. (e.g., vancomycin resistant *Enterococci,* VRE) may include BRILLIANCE™ VRE agar (Oxoid), CHROMID™ VRE (bioMérieux), and CHROMAGAR™ VRE (Chromagar). Suitable chromogenic media for gram negative *Bacilli* expressing extended spectrum β-Lactamase (ESBL) may include BRILLIANCE™ ESBL agar (Oxoid), CHROMID™ESBL (bioMérieux), and HARDYCHROM™ ESBL agar (Hardy Diagnostics, Santa Maria, CA). Suitable chromogenic media for carbapenem-resistant Enterobacteriaceae (CRE) may include BRILLIANCE™ CRE agar (Oxoid); HARDYCHROM™ CRE agar (Hardy Diagnostics); CHROMID™CARBA SMART (bioMérieux); and CHROMAGAR™ KPC (Chromagar).

In some embodiments, the methods of the invention may involve AST using automated systems, including full-range automated AST devices. A number of automated systems are known in the art. Non-limiting examples include the VITEK® 2 (bioMérieux), MICROSCAN® (Beckman Coulter, Brea, CA), and PHOENIX™ (BD Diagnostics) systems. In some embodiments, the methods of the invention may include obtaining a pellet of the pathogen from blood culture or a subculture thereof, for example, using lysis centrifugation (e.g., SEPSITYPER™, Bruker Corporation, Billerica, MA), lysis filtration, or centrifugation alone, followed by inoculation of an automated system.

In some embodiments, the methods of the invention may involve T2MR-based AST. In some embodiments, T2MR is used to determine the growth of pathogen cells present in a biological sample or subculture thereof containing antimicrobial agents or controls, for example, in aliquots taken at time intervals. In some embodiments, growth is determined by determining the level of an analyte characteristic of the pathogen (e.g., a nucleic acid (e.g., DNA or RNA (e.g., mRNA), protein, small molecule, metabolite, growth media component, or cellular biproduct) over time. In other embodiments, growth is determined by direct cell detection of pathogens, for example, as described in International Patent Application Publication No. WO 2012/129281; Skewis et al. Nuclear Magnetic Resonance Nanotechnology: Applications in Clinical Diagnostics and Monitoring. Encyclopedia of Analytical Chemistry, 2013; Kaittanis et al. Nano Lett. 7:380, 2007; Lee et al. Nat. Med. 14:869, 2008; Kulkarni et al. Anal. Chem. 82:7430, 2010; Chung et al. ACS Nano 5:8834, 2011; Liong et al. Bioconjug. Chem. 22:2390, 2011; and Lee et al. Angew. Chem. Int. Ed. 48:5657, 2009. In some embodiments, direct cell detection is achieved using magnetic particles that include binding moieties operative to bind to the cell surface of the pathogen. For example, in some embodiments, the binding moieties are operative to bind to a surface-exposed protein (e.g., protein A of *Staphylococcus aureus* or protein G of *Streptococci)* or a cell wall component (e.g., D-alanyl-D-alanine or lipopolysaccharide (LPS)). Binding of magnetic particles to the pathogen or pathogen-associated analyte can lead to a change in the T2MR signal that can be used to indicate, detect, and/or monitor cell growth.

Any suitable number of antimicrobial agents may be tested in the methods of the invention. For example, in some embodiments, about 1 to about 30 (e.g., about 1, about 2, about 3, about 4, about 5, about 6, about 7, about 8, about 9, about 10, about 11, about 12, about 13, about 14, about 15, about 16, about 17, about 18, about 19, about 20, about 25, or about 30) or more antimicrobial agents are tested in the methods of the invention. The antimicrobial agents for testing are typically selected based on the identity (e.g., at the genus and/or species level) of the pathogen detected and identified by the methods of the invention. In some embodiments, the antimicrobial agents for testing are based on local resistance patterns. In some embodiments, the antimicrobial agents for testing are based on both on the identity of the pathogen species detected and identified by the methods of the invention and on local resistance patterns. The susceptibility or resistance of a pathogen to a given antimicrobial agent may refer to a direct interaction between the antimicrobial agent and the pathogen (which can be measured in vitro) or the likelihood that the subject will respond to treatment (which may depend in part on dosing, dose schedule, site of infection, pharmacokinetics (PK) of the antimicrobial agent), and/or host defenses).

As a non-limiting example, if the pathogen is identified as an *Enterobacteriaceae* (e.g., *Escherichia* spp. *(e.g., E. coli), Klebsiella* spp. (e.g., *K. pneumoniae),* or *Enterobacter* spp.), the methods of the invention may include testing from about 1 to about 20 (e.g., about 1, about 2, about 3, about 4, about 5, about 6, about 7, about 8, about 9, about 10, about 11, about 12, about 13, about 14, about 15, a out 16, about 17, about 18, about 19, or about 20) antimicrobial agents. In some embodiments, the antimicrobial agent is selected from one or more (e.g., 1, 2, 3, 4, 5, 6, 7, 8, 9, or 10) of the following: amp/sulbactam, cefepime, cefotaxime, ceftazidime, ceftriaxone, ciprofloxacin, gentamicin, imipenem, meropenem, and/or piperacillin/tazobactam. In some embodiments, the method involves testing amp/sulbactam, cefepime, cefotaxime, ceftazidime, ceftriaxone, ciprofloxacin, gentamicin, imipenem, meropenem, and piperacillin/tazobactam.

In another example, if the pathogen is identified as a *Pseudomonas* spp. (e.g., *P. aeruginosa)* or an *Acinetobacter* spp. (e.g., A. *baumannii),* the methods of the invention may involve testing from about 1 to about 20 (e.g., about 1, about 2, about 3, about 4, about 5, about 6, about 7, about 8, about 9, about 10, about 11, about 12, about 13, about 14, about 15, a out 16, about 17, about 18, about 19, or about 20) antimicrobial agents. In some embodiments, the antimicrobial agent is selected from one or more (e.g., 1, 2, 3, 4, 5, 6, 7, 8, 9, or 10) of the following: aztreonam, cefepime, ceftazidime, ciprofloxacin, gentamicin, amikacin, tobramycin, imipenem, meropenem, doxycycline (especially for *Acinetobacter),* piperacillin/tazobactam, and/or colistin. In some embodiments, the method involves testing aztreonam, cefepime, ceftazidime, ciprofloxacin, gentamicin, amikacin, tobramycin, imipenem, meropenem, doxycycline, piperacillin/tazobactam, and colistin.

In yet another example, if the pathogen is identified as a *Staphylococcus* (e.g., S. *aureus*)*,* the methods of the invention may involve testing from about 1 to about 12 (e.g., about 1, about 2, about 3, about 4, about 5, about 6, about 7, about 8, about 9, about 10, about 11, about 12) antimicrobial agents. In some embodiments, the antimicrobial agent is selected from one or more (e.g., 1, 2, 3, 4, 5, or 6) of the following: oxacillin, vancomycin, trimethoprim-sulfamethoxazole (BACTRIM™), doxycycline, daptomycin, and/or linezolid. In some embodiments, the method involves testing oxacillin, vancomycin, trimethoprim/sulfa, doxycycline, daptomycin, and linezolid.

In a still further example, if the pathogen is identified as an *Enterococcus* (e.g., *Enterococcus faecalis* or *Enterococcus faecium*)*,* the methods of the invention may involve testing from about 1 to about 12 (e.g., about 1, about 2, about 3, about 4, about 5, about 6, about 7, about 8, about 9, about 10, about 11, about 12) antimicrobial agents. In some embodiments, the antimicrobial agent is selected from one or more (e.g., 1, 2, 3, or 4) of the following: ampicillin, vancomycin, linezolid, and/or high level aminoglycoside. In some embodiments, the method involves testing ampicillin, vancomycin, linezolid, and high level aminoglycoside.

In some embodiments, antimicrobial agents are tested at breakpoint concentrations. See, for example, Turnidge et al. Clinical Microbiology Reviews 20(3):391-408, 2007. Breakpoint concentrations are also referred to as interpretive criteria, and may assist a clinician in interpreting AST results to classify a pathogen as susceptible, intermediate, or resistant to a given antimicrobial agent. For example, in some embodiments, a breakpoint concentration may be the minimum inhibitory concentration (MIC) for any given antimicrobial agent that distinguishes wild-type populations of the pathogen from those with acquired or selected resistance mechanisms (also known in the art as a "wild-type" breakpoint). In other embodiments, a breakpoint concentration may refer to a "clinical breakpoint," which refers to concentrations that separate pathogen strains where there is an increased likelihood of treatment success from pathogen strains where there is an increased likelihood of treatment failure. In yet other embodiments, the breakpoint concentration may be a concentration of antimicrobial agent calculated from knowledge of a pharmacodynamic (PD) parameter and the dimension of that parameter that predicts efficacy in vivo (e.g., a pharmacokinetic/PD (PK/PD) breakpoint, where data generated in an animal model may be extrapolated to humans using mathematical techniques). Breakpoint concentrations can be determined using methods known in the art (see, e.g., Turnidge et al. *supra*)*.* Additionally, breakpoint concentrations for antimicrobial agents are published in guidelines from the Clinical and Laboratory Standards Institute (CLSI), the European Union Committee on Antimicrobial Susceptibility Testing (EUCAST), and the U.S. Food and Drug Administration (FDA). It is to be understood that while breakpoints set by different organizations may differ in some aspects, a skilled artisan is able to determine appropriate breakpoint concentrations for a particular pathogen species and a particular antimicrobial agent using approaches known in the art.

The results of AST performed in the methods of the invention may be classified or interpreted using any suitable approaches known in the art. For example, a pathogen may be classified as "susceptible," "intermediate," or "resistant" to an antimicrobial agent based on any suitable method. One set of classifications that may be used with the methods of the invention is provided by CLSI (Performance Standards for Antimicrobial Susceptibility Testing; Twenty-Fifth Informational Supplement (M100-S25), 2015). For example, a susceptible category may include pathogen isolates that are inhibited by the usually achievable concentrations of antimicrobial agent when the recommended dosage is used for that site of infection. An intermediate category may include pathogen isolates with antimicrobial agent MICs that approach usually attainable blood and tissue levels and for which response rates may be lower than those for susceptible pathogen isolates. The resistant category may include pathogen isolates that are not inhibited by the usually achievable concentrations of the microbial agent with normal dosage schedules and/or demonstrate MICs that fall in the range where specific microbial resistance mechanisms are likely and that clinical efficacy against the pathogen has not been shown reliably in treatment studies. Other classification approaches beyond these exemplary classification approaches are known in the art and may be used in the methods of the invention.

In some embodiments, the methods of the invention may involve detecting the presence and/or activity of antimicrobial resistance factors (e.g., acquired antimicrobial resistance genes, including AmpC β-lactamases (Bla_{Ampc}), extended-spectrum β-lactamases (ESBLs), *erm*B*,* mefA, *mec*A*, fol*P*, van*A*,* and *van*B*;* see, e.g., Thabit et al. Expert Opin. Pharmacother. 16(2):159-177 for additional antimicrobial resistance factors). For example, in some embodiments, a sample of the identified pathogen may be subjected to a PCR-based (see, e.g., Aminov et al. Methods Mol. Biol. 268:3-13, 2004 and Ingram et al. J. Med. Microbiol. 60:715-721, 2011) or microarray analysis (see, e.g., Call et al. Antimicrob. Agents Chemother. 47(10):3290-3295, 2003) to detect the presence of resistance genes. In other embodiments, non-agar based chromogenic tests for detection of resistance genes, e.g., ESBL/AMPC/CRE, may be used. The results from these assays may be analyzed in addition to phenotypic AST results to determine whether an antimicrobial agent is likely to be effective to treat an infection by a pathogen identified by the methods of the invention.

In some embodiments, any of the methods of the invention may involve taking additional blood draws from the subject. In some embodiments, the additional blood draws are based on the titer level of the pathogen in the biological sample. For example, if quantitative or semi-quantitative T2MR results indicate that there is a low titer level of the pathogen, one may take multiple draws from the patient (in the event of low titer level) or smaller draws (in the event of a high titer level). These additional blood draws may be used, for example, for AST or expression analysis (e.g., real-time PCR or microarray) of genes characteristic of the pathogen, such as inducible antimicrobial resistance genes, housekeeping genes, or other genes. In some embodiments, expression analysis involves measurement of RNA (e.g., mRNA) levels.

### Treatment (not claimed)

After the methods of the invention have been carried out, a therapeutic agent may be administered to a subject following a diagnosis. Typically, the identification of a particular pathogen by the methods of the invention will guide the selection of the appropriate therapeutic agent. By the methods of the invention, a therapeutic agent to which the pathogen identified in a patient sample has been determined to be susceptible is identified as to be administered. The therapeutic agent may be an antimicrobial agent, e.g., an antibiotic, an antifungal agent, an antiprotozoal agent, an antiviral agent, or any other therapeutic agent suitable for treatment and/or prophylaxis of a disease associated with infection by a pathogen.

For example, for a bacterial infection (e.g., bacteremia), a therapy may include an antibiotic. In some instances, an antibiotic may be administered orally. In other instances, the antibiotic may be administered intravenously. Exemplary non-limiting antibiotics that may be used in the methods of the invention include acrosoxacin, amifioxacin, amikacin, amoxycillin, ampicillin, aspoxicillin, azidocillin, azithromycin, aztreonam, balofloxacin, benzylpenicillin, biapenem, brodimoprim, cefaclor, cefadroxil, cefatrizine, cefcapene, cefdinir, cefetamet, ceftmetazole, cefoxitin, cefprozil, cefroxadine, ceftarolin, ceftazidime, ceftibuten, ceftobiprole, cefuroxime, cephalexin, cephalonium, cephaloridine, cephamandole, cephazolin, cephradine, chlorquinaldol, chlortetracycline, ciclacillin, cinoxacin, ciprofloxacin, clarithromycin, clavulanic acid, clindamycin, clofazimine, cloxacillin, colistin, danofloxacin, dapsone, daptomycin, demeclocycline, dicloxacillin, difloxacin, doripenem, doxycycline, enoxacin, enrofloxacin, erythromycin, fleroxacin, flomoxef, flucloxacillin, flumequine, fosfomycin, gentamycin, isoniazid, imipenem, kanamycin, levofloxacin, linezolid, mandelic acid, mecillinam, meropenem, metronidazole, minocycline, moxalactam, mupirocin, nadifloxacin, nafcillin, nalidixic acid, netilmycin, netromycin, nifuirtoinol, nitrofurantoin, nitroxoline, norfloxacin, ofloxacin, oxacillin, oxytetracycline, panipenem, pefloxacin, phenoxymethylpenicillin, pipemidic acid, piromidic acid, pivampicillin, pivmecillinam, polymixin-b, prulifloxacin, rufloxacin, sparfloxacin, sulbactam, sulfabenzamide, sulfacytine, sulfametopyrazine, sulphacetamide, sulphadiazine, sulphadimidine, sulphamethizole, sulphamethoxazole, sulphanilamide, sulphasomidine, sulphathiazole, teicoplanin, temafioxacin, tetracycline, tetroxoprim, tigecycline, tinidazole, tobramycin, tosufloxacin, trimethoprim, vancomycin, and pharmaceutically acceptable salts or esters thereof.

The treatment method (not claimed) may involve administration of an antifungal agent, for example, for treatment of fungemia (e.g., Candidemia). Exemplary antifungal agents suitable for use in the invention include, but are not limited to, polyenes (e.g., amphotericin B, candicidin, filipin, hamycin, natamycin, nystatin, and rimocidin), azoles (e.g., imidazoles such as bifonazole, butoconazole, clotrimazole, eberconazole, econazole, fenticonazole, flutrimazole, isoconazole, ketoconazole, luliconazole, miconazole, omoconazole, oxiconazole, sertaconazole, sulconazole, tioconazole; triazoles such as albaconazole, efinaconazole, epoxiconazole, fluconazole, isavuconazole, itraconazole, posaconazole, propiconazole, ravuconazole, terconazole, voriconazole; and thiazoles such as abafungin), allylamines (e.g., amorolfin, butenafine, naftifine, and terbinafine), echinocandins (e.g., anidulafungin, caspofungin, and micafungin), and other antifungal agents including but not limited to benzoic acid, ciclopirox olamine, 5-flucytosin, griseofulvin, haloprogin, tolnaftate, aminocandin, chlordantoin, chlorphenesin, nifuroxime, undecylenic acid, crystal violet and pharmaceutically acceptable salts or esters thereof.

The treatment method (not claimed) may involve administration of an antiprotozoal agent, for example, for treatment of infection by *Babesia microti.* Exemplary antiprotozoal agents suitable for use in the invention include, but are not limited to, acetarsol, amphotericin (e.g., liposomal amphotericin, amphotericin B, and amphotericin deoxycholate), arthemether, artsunate, atovaquone, azanidazole, azithromycin, benznidazole, chloroquine, ciprofloxacin, clindamycin, diloxanide, eflornithine, flucytosine, fluconazole, folinic acid, hydroxychloroquine, iodoquinol, lumefantrine, macrolides, mefloquine, melarsoprol, metronidazole, miltefosine, nifuratel, nifurtimox, nimorazole, nitazoxanide, omidazole, paramomycin, pentamidine, primaquine, proguanil, propenidazole, pyrimethamine, quinine, quinidine, secnidazole, sinefungin, sodium stibogluconate, spiramycin, suramin, sulfadiazine, sulfamethoxazole, tenonitrozole, temidazole, tinidazole, trimethoprim, TMP/SMX, and pharmaceutically acceptable salts or esters thereof.

The treatment method (not claimed) may involve administration of an antiviral agent, for example, for treatment of viremia. Exemplary antiviral agents suitable for use in the invention include, but are not limited to, abacavir, acyclovir, acyclovir, adefovir, amantadine, amprenavir, ampligen, arbidol, atazanavir, atripla, balavir, brivudine, cidofovir, combivir, curcumin, darunavir, delavirdine, desciclovir, didanosine, 1-docosanol, dolutegravir, edoxudine, efavirenz, emtricitabine, enfuvirtide, entecavir, ecoliever, famciclovir, fiacitabine, fomivirsen, fosamprenavir, foscarnet, fosfonet, fusion inhibitors, ganciclovir, ibacitabine, idoxuridine, imiquimod, imunovir, indinavir, inosine, integrase inhibitor, interferon (e.g., interferon type I, interferon type II, and interferon type III), lamivudine, lopinavir, loviride, maraviroc, moroxydine, methisazone, nelfinavir, nevirapine, nexavir, nucleoside analogs, novir, oseltamivir, peginterferon alfa-2a, penciclovir, peramivir, pleconaril, podophyllotoxin, protease inhibitors, pyramidine, raltegravir, reverse transcriptase inhibitors, ribavarin, rimantadine, ritonavir, saquinavir, sofosbuvir, stavudine, telaprevir, tenofovir, tenofovir disoproxil, tipranavir, trifluridine, trizivir, tromontadine, truvada, valacyclovir, valganciclovir, vicriviroc, vidarabine, viramidine, zalcitabine, zanamivir, zidovudine, and pharmaceutically acceptable salts or esters thereof.

An antimicrobial agent or any other therapeutic agent may be administered by any suitable route. An antimicrobial agent or any other therapeutic agent, or a pharmaceutical composition thereof, may be administered by one or more of a variety of routes, including parenteral (e.g., subcutaneous, intracutaneous, intravenous, intraperitoneal, intramuscular, intraarticular, intraarterial, intrasynovial, intrasternal, intrathecal, intralesional, or intracranial injection, as well as any suitable infusion technique), oral, trans- or intra-dermal, interdermal, rectal, intravaginal, topical (e.g.. by powders, ointments, creams, gels, lotions, and/or drops), mucosal, nasal, buccal, enteral, vitreal, intratumoral, sublingual, intranasal; by intratracheal instillation, bronchial instillation, and/or inhalation; as an oral spray and/or powder, nasal spray, and/or aerosol, and/or through a portal vein catheter. A composition may be administered intravenously, intramuscularly, intradermally, intra-arterially, intratumorally, subcutaneously, or by inhalation. However, the present disclosure encompasses the delivery of compositions by any appropriate route taking into consideration likely advances in the sciences of drug delivery. In general, the most appropriate route of administration will depend upon a variety of factors including the nature of the pharmaceutical composition (e.g., its stability in various bodily environments such as the bloodstream and gastrointestinal tract), and the condition of the patient (e.g., whether the patient is able to tolerate particular routes of administration).

A dose of an antimicrobial agent or any other therapeutic agent may be administered at any suitable frequency, in the same or a different amount, to obtain a desired drug concentration and/or effect (e.g., a therapeutic effect). The desired dosage may be delivered, for example, three times a day, two times a day, once a day, every other day, every third day, every week, every two weeks, every three weeks, or every four weeks. The desired dosage may be delivered using multiple administrations (e.g., two, three, four, five, six, seven, eight, nine, ten, eleven, twelve, thirteen, fourteen, or more administrations). The specific therapeutically effective, prophylactically effective, or otherwise appropriate dose level for any particular subject will depend upon a variety of factors including the severity and identify of a disorder being treated, if any; the antimicrobial agent and/or other therapeutic agent employed; the specific composition employed; the age, body weight, general health, sex, and diet of the patient; the time of administration, route of administration, and rate of excretion of the specific pharmaceutical composition employed; the duration of the treatment; drugs used in combination or coincidental with the specific pharmaceutical composition employed; and like factors well known in the medical arts.

An antimicrobial agent or other therapeutic agent, or a pharmaceutical composition thereof, may be administered in combination with another agent, for example, another therapeutic agent, a prophylactic agent, and/or a diagnostic agent. By "in combination with," it is not intended to imply that the agents must be administered at the same time and/or formulated for delivery together, although these methods of delivery are within the scope of the present disclosure. For example, one or more compositions including one or more different antimicrobial agents may be administered in combination. Compositions can be administered concurrently with, prior to, or subsequent to, one or more other desired therapeutics or medical procedures. In general, each agent will be administered at a dose and/or on a time schedule determined for that agent. The present disclosure encompasses the delivery of compositions, or imaging, diagnostic, or prophylactic compositions thereof in combination with agents that improve their bioavailability, reduce and/or modify their metabolism, inhibit their excretion, and/or modify their distribution within the body.

### Assay reagents

The methods described herein may include any suitable reagents, for example, surfactants, buffer components, additives, chelating agents, and the like. The surfactant may be selected from a wide variety of soluble non-ionic surface active agents including surfactants that are generally commercially available under the IGEPAL® trade name from GAF Company. The IGEPAL® liquid non-ionic surfactants are polyethylene glycol p-isooctylphenyl ether compounds and are available in various molecular weight designations, for example, IGEPAL® CA720, IGEPAL® CA630, and IGEPAL® CA890. Other suitable non-ionic surfactants include those available under the trade name TETRONIC® 909 from BASF Corporation. This material is a tetra-functional block copolymer surfactant terminating in primary hydroxyl groups. Suitable non-ionic surfactants are also available under the ALPHONIC® trade name from Vista Chemical Company and such materials are ethoxylates that are non-ionic biodegradables derived from linear primary alcohol blends of various molecular weights. The surfactant may also be selected from poloxamers, such as polyoxyethylene-polyoxypropylene block copolymers, such as those available under the trade names SYNPERONIC® PE series (ICI), PLURONIC® series (BASF), Supronic, MONOLAN®, PLURACARE®, and PLURODAC®, polysorbate surfactants, such as TWEEN® 20 (PEG-20 sorbitan monolaurate), and glycols such as ethylene glycol and propylene glycol.

Such non-ionic surfactants may be selected to provide an appropriate amount of detergency for an assay without having a deleterious effect on assay reactions. In particular, surfactants may be included in a reaction mixture for the purpose of suppressing non-specific interactions among various ingredients of the aggregation assays. The non-ionic surfactants are typically added to the liquid sample prior in an amount from 0.01% (w/w) to 5% (w/w).

The non-ionic surfactants may be used in combination with one or more proteins (e.g., albumin, fish skin gelatin, lysozyme, or transferrin) also added to the liquid sample prior in an amount from 0.01% (w/w) to 5% (w/w).

Furthermore, the methods of the invention can include additional suitable buffer components (e.g., Tris base, selected to provide a pH of about 7.8 to 8.2 in the reaction milieu); and chelating agents to scavenge cations (e.g., ethylene diamine tetraacetic acid (EDTA), EDTA disodium, citric acid, tartaric acid, glucuronic acid, saccharic acid or suitable salts thereof).

### Sample Preparation and Cell Lysis

The methods of the invention may involve sample preparation and/or cell lysis. For example, a pathogen present in a biological sample may be lysed prior to amplification of a target nucleic acid. Suitable lysis methods for lysing pathogen cells in a biological sample (e.g., whole blood, urine, and tissue homogenates) include, for example, mechanical lysis (e.g., beadbeating and sonication), heat lysis, and alkaline lysis. In some embodiments, beadbeating may be performed by adding glass beads (e.g., 0.5 mm glass beads) to a biological sample to form a mixture and agitating the mixture. As an example, the sample preparation and cell lysis (e.g., beadbeating) may be performed using any of the approaches and methods described in WO 2012/054639.

In some embodiments, the methods of the invention involve detection of one or more pathogen-associated analytes in a whole blood sample. In some embodiments, the methods may involve disruption of red blood cells (erythrocytes). In some embodiments, the disruption of the red blood cells can be carried out using an erythrocyte lysis agent (i.e., a lysis buffer, or a nonionic detergent). Erythrocyte lysis buffers which can be used in the methods of the invention include, without limitation, isotonic solutions of ammonium chloride (optionally including carbonate buffer and/or EDTA), and hypotonic solutions. Alternatively, the erythrocyte lysis agent can be an aqueous solution of nonionic detergents (e.g., nonyl phenoxypolyethoxylethanol (NP-40), 4-octylphenol polyethoxylate (TRITON™ X-100), BRIJ® 58, or related nonionic surfactants, and mixtures thereof). The erythrocyte lysis agent disrupts at least some of the red blood cells, allowing a large fraction of certain components of whole blood (e.g., certain whole blood proteins) to be separated (e.g., as supernatant following centrifugation) from the white blood cells or other cells (e.g., pathogen cells (e.g., bacterial cells and/or fungal cells)) present in the whole blood sample. Following erythrocyte lysis and centrifugation, the resulting pellet may be lysed, for example, as described above. In some embodiments, the resulting pellet is lysed without any additional washes or erythrocyte lysis steps.

In some embodiments, the methods of the invention may include (a) providing a whole blood sample from a subject; (b) mixing the whole blood sample with an erythrocyte lysis agent solution to produce disrupted red blood cells; (c) following step (b), centrifuging the sample to form a supernatant and a pellet, discarding some or all of the supernatant, and resuspending the pellet to form an extract, (d) lysing cells of the extract (which may include white blood cells and/or pathogen cells) to form a lysate. In some embodiments, the method further comprises amplifying one or more target nucleic acids in the lysate. In some embodiments, the sample of whole blood is from about 0.5 to about 10 mL of whole blood, for example, 0.5 mL, 1 mL, 2 mL, 3 mL, 4 mL, 5 mL, 6 mL, 7 mL, 8 mL, 9 mL, or 10 mL of whole blood. In some embodiments, the method may include washing the pellet (e.g., with a buffer such as TE buffer) prior to resuspending the pellet and optionally repeating step (c). In some embodiments, the method may include 1, 2, 3, 4, 5, or more wash steps. In other embodiments, the method is performed without performing any wash step. In some embodiments, the amplifying is in the presence of whole blood proteins, non-target nucleic acids, or both. In some embodiments, the amplifying may be in the presence of from 0.5 µg to 60 µg (e.g., 0.5 µg, 1 µg, 5 µg, 10 µg, 15 µg, 20 µg, 25 µg, 30 µg, 35 µg, 40 µg, 45 µg, 50 µg, 55 µg, or 60 µg) of subject DNA. In some embodiments, the subject DNA is from white blood cells of the subject.

### Amplification and Detection of Nucleic Acids from Complex Samples

In several embodiments, the methods of the invention involve amplification of one or more nucleic acids. Amplification may be exponential or linear. A target or template nucleic acid may be either DNA or RNA (e.g., mRNA). The sequences amplified in this manner form an "amplified region" or "amplicon." Primer probes can be readily designed by those skilled in the art to target a specific template nucleic acid sequence. In certain preferred embodiments, resulting amplicons are short to allow for rapid cycling and generation of copies. The size of the amplicon can vary as needed, for example, to provide the ability to discriminate target nucleic acids from non-target nucleic acids. For example, amplicons can be less than about 1,000 nucleotides in length. Desirably the amplicons are from 100 to 500 nucleotides in length (e.g., 100 to 200, 150 to 250, 300 to 400, 350 to 450, or 400 to 500 nucleotides in length). In some embodiments, more than one (e.g., 1, 2, 3, 4, 5, 6, 7, 8, 9, 10, or more than 10) target nucleic acids may be amplified in one reaction. In other embodiments, a single target nucleic acid may be amplified in one reaction. In some embodiments, the invention provides amplification-based nucleic acid detection assays conducted in complex samples (e.g., whole blood).

Sample preparation typically involves removing or providing resistance for common PCR inhibitors found in complex samples (e.g., body fluids and tissue homogenates). Common inhibitors are listed in Table 2 (see also Wilson, Appl. Environ. Microbiol., 63:3741 (1997)). Inhibitors typically act by either prevention of cell lysis, degradation or sequestering a target nucleic acid, and/or inhibition of a polymerase activity. The most commonly employed polymerase, Taq, is inhibited by the presence of 0.1% blood in a reaction. Mutant Taq polymerases have been engineered that are resistant to common inhibitors (e.g., hemoglobin and/or humic acid) found in blood (Kermekchiev et al., Nucl. Acid. Res., 37(5): e40, (2009)). Manufacturer recommendations indicate these mutations enable direct amplification from up to 20% blood. Despite resistance afforded by the mutations, accurate real-time PCR detection is complicated due to fluorescence quenching observed in the presence of blood sample (Kermekchiev et al., Nucl. Acid. Res., 37:e40 (2009)).

**Table 2. PCR inhibitors and facilitators/methods for overcoming inhibition**

| **Substrate** | **Target** | **Inhibitor** | **Facilitator** |
|---|---|---|---|
| feces | Escherichia coli | >10³ bacterial cells | ion-exchange column |
| CSF | Treponema pallidum | Cellular debris causing nonspecific amplification | nested primers |
| whole blood | mammalian tissue | >4 µl of blood/100-ml reaction mix (hemoglobin) | 1-2% blood per reaction |
| feces | Rotavirus | unknown dilution | cellulose fiber |
| clinical specimens | Cytomegalovirus | unidentified components | glass bead extraction |
| human blood and tissue | human genes | DNA binding proteins | thermophilic protease from Thermus strain rt44A |
| mammalian tissue | Mammalian tissue genetics | thermal cycler variations | formamide |
| mammalian tissue | Mammalian tissue genetics | thermal cycler variations | DMSO, glycerol, PEG, organic solvents |
| clinical specimens | Treponema pallidum | unknown factors | Various substrate-specific physicochemical methods |
| forensic semen samples | Sperm | Genotyping errors; selective/total PCR inhibition by vaginal microorganisms | |
| feces | Salmonella enterica | various body fluids | immunomagnetic separation |
| feces | Various enteric viruses | unknown | size exclusion chromatography, physicochemical extraction |
| clinical specimens | Herpes simplex virus | endogenous inhibitors, random effects | repurification, coamplified positive control |
| feces | Escherichia coli | nonspecific inhibitors, urea, hemoglobin, heparin, phenol, SDS | additional primers and reaction cyclers, booster PCR |
| tissue culture | Cytomegalovirus HIV | glove powder | |
| suspensions, skin biopsies | Mycobacterium leprae | mercury-based fixatives, neutral buffered formaline | reduced fixation times, ethanol fixation |
| clinical specimens | Mycobacterium tuberculosis | unknown inhibitors in pus, tissue biopsies, sputum, pleural fluid | physicochemical extraction |
| mammalian tissue | mammalian tissue genetics | unknown contaminant of reverse transcriptase | additional DNA |
| formalin-fixed paraffin tissue | Hepatitis C virus | ribonucleotide vanadyl complexes | phenol/chloroform extraction |
| nasopharyngea I aspirates and swabs | Bordetella pertussis | unknown inhibitors | phenol/chloroform extraction |
| human mononuclear blood cells | HIV type I | detergents | mineral oil |
| bloodstain | human mitochondrial DNA | unidentified heme compound, hemin | BSA |
| blood | various | heparin | alternative polymerases and buffers, chelex, spermine, [Mg2+], glycerol, BSA, heparinase |
| sputa | Mycoplasma pneumonia | N-acetyl-L-cysteine, dithiothreitol, mucolytic agents | |
| human tissue | HLA-DRB1 genotyping | pollen, glove powder, impure DNA, heparin, hemoglobin | |
| clinical specimens | Mycobacterium tuberculosis | unknown | competitive internal control |
| dental plaque | many | unknown | diatomaceous earth, guanidium isothiocyanate, ethanol, acetone |
| ancient mammalian tissues | Cytochrome b gene | unknown | ammonium acetate, ethidium bromide |

Polymerase chain reaction amplification of DNA or cDNA is a tried and trusted methodology; however, as discussed above, polymerases are inhibited by agents contained in crude samples, including but not limited to commonly used anticoagulants and hemoglobin. Recently mutant Taq polymerases have been engineered to harbor resistance to common inhibitors found in blood and soil. Currently available polymerases, e.g., HemoKlenTaq™ (New England BioLabs, Inc., Ipswich, MA) as well as OmniTaq™ and OmniKlenTaq™ (DNA Polymerase Technology, Inc., St. Louis, MO) are mutant (e.g., N-terminal truncation and/or point mutations) Taq polymerase that render them capable of amplifying DNA in the presence of up to 10%, 20% or 25% whole blood, depending on the product and reaction conditions (See, e.g., Kermekchiev et al. Nucl. Acids Res. 31:6139 (2003); and Kermekchiev et al., Nucl. Acid. Res. 37:e40 (2009); and see U.S. Patent No. 7,462,475). Additionally, PHUSION® Blood Direct PCR Kits (Finnzymes Oy, Espoo, Finland), include a unique fusion DNA polymerase enzyme engineered to incorporate a double-stranded DNA binding domain, which allows amplification under conditions which are typically inhibitory to conventional polymerases such as Taq or Pfu, and allow for amplification of DNA in the presence of up to about 40% whole blood under certain reaction conditions. See Wang et al., Nucl. Acids Res. 32:1197 (2004); and see U.S. Patent Nos. 5,352,778 and 5,500,363. Furthermore, Kapa Blood PCR Mixes (Kapa Biosystems, Woburn, MA), provide a genetically engineered DNA polymerase enzyme which allows for direct amplification of whole blood at up to about 20% of the reaction volume under certain reaction conditions. Despite these breakthroughs, direct optical detection of generated amplicons is not possible with existing methods since fluorescence, absorbance, and other light based methods yield signals that are quenched by the presence of blood. See Kermekchiev et al., Nucl. Acid. Res. 37:e40 (2009).

A variety of impurities and components of whole blood can be inhibitory to the polymerase and primer annealing. These inhibitors can lead to generation of false positives and low sensitivities. To reduce the generation of false positives and low sensitivities when amplifying and detecting nucleic acids in complex samples, it is desirable to utilize a thermal stable polymerase not inhibited by whole blood samples, for example as described above, and include one or more internal PCR assay controls (see Rosenstraus et al. J. Clin Microbiol. 36:191 (1998) and Hoofar et al., J. Clin. Microbiol. 42:1863 (2004)).

For example, the assay can include an internal control nucleic acid that contains primer binding regions identical to those of the target sequence to assure that clinical specimens are successfully amplified and detected. In some embodiments, the target nucleic acid and internal control can be selected such that each has a unique probe binding region that differentiates the internal control from the target nucleic acid. The internal control is, optionally, employed in combination with a processing positive control, a processing negative control, and a reagent control for the safe and accurate determination and identification of an infecting organism in, e.g., a whole blood clinical sample. The internal control can be an inhibition control that is designed to co-amplify with the nucleic acid target being detected. Failure of the internal inhibition control to be amplified is evidence of a reagent failure or process error. Universal primers can be designed such that the target sequence and the internal control sequence are amplified in the same reaction tube. Thus, using this format, if the target DNA is amplified but the internal control is not it is then assumed that the target DNA is present in a proportionally greater amount than the internal control and the positive result is valid as the internal control amplification is unnecessary. If, on the other hand, neither the internal control nor the target is amplified it is then assumed that inhibition of the PCR reaction has occurred and the test for that particular sample is not valid.

The assays can include one or more positive processing controls in which one or more target nucleic acids is included in the assay (e.g., each included with one or more cartridges) at 3x to 5x the limit of detection. The measured T₂ for each of the positive processing controls must be above the predetermined threshold indicating the presence of the target nucleic acid. The positive processing controls can detect all reagent failures in each step of the process (e.g., lysis, PCR, and T₂ detection), and can be used for quality control of the system. The assays can include one or more negative processing controls consisting of a solution free of target nucleic acid (e.g., buffer alone). The T₂ measurements for the negative processing control should be below the threshold indicating a negative result while the T₂ measured for the internal control is above the decision threshold indicating an internal control positive result. The purpose of the negative control is to detect carry-over contamination and/or reagent contamination. The assays can include one or more reagent controls. The reagent control will detect reagent failures in the PCR stage of the reaction (i.e. incomplete transfer of master mix to the PCR tubes). The reagent controls can also detect gross failures in reagent transfer prior to T₂ detection.

In some embodiments, complex biological samples, which may be a liquid sample (including whole blood, cerebrospinal fluid, urine, synovial fluid, and tissue biopsy homogenates (e.g., skin biopsies) can be directly amplified using about 5%, about 10%, about 20%, about 25%, about 30%, about 25%, about 40%, and about 45% or more complex liquid sample in amplification reactions, and that the resulting amplicons can be directly detected from amplification reaction using magnetic resonance (MR) relaxation measurements upon the addition of conjugated magnetic particles bound to oligonucleotides complementary to the target nucleic acid sequence. Alternatively, the magnetic particles can be added to the sample prior to amplification. Thus, provided are methods for the use of nucleic acid amplification in a complex dirty sample, hybridization of the resulting amplicon to paramagnetic particles, followed by direct detection of hybridized magnetic particle conjugate and target amplicons using magnetic particle based detection systems. In particular embodiments, direct detection of hybridized magnetic particle conjugates and amplicons is via MR relaxation measurements (e.g., T₂, T₁, T₁/T₂ hybrid, T₂*, etc). Further provided are methods which are kinetic, in order to quantify the original nucleic acid copy number within the sample (e.g., sampling and nucleic acid detection at pre-defined cycle numbers, comparison of endogenous internal control nucleic acid, use of exogenous spiked homologous competitive control nucleic acid).

While the exemplary methods described hereinafter relate to amplification using polymerase chain reaction ("PCR"), numerous other methods are known in the art for amplification of nucleic acids (e.g., isothermal methods, rolling circle methods, etc.). Those skilled in the art will understand that these other methods may be used either in place of, or together with, PCR methods. See, e.g., Saiki, "Amplification of Genomic DNA" in PCR Protocols, Innis et al., Eds., Academic Press, San Diego, Calif., pp 13-20 (1990); Wharam et al., Nucleic Acids Res. 29:E54 (2001); Hafner et al., Biotechniques 30:852 (2001). Further amplification methods suitable for use with the present methods include, for example, reverse transcription PCR (RT-PCR), ligase chain reaction (LCR), transcription based amplification system (TAS), transcription mediated amplification (TMA), nucleic acid sequence based amplification (NASBA) method, the strand displacement amplification (SDA) method, the loop mediated isothermal amplification (LAMP) method, the isothermal and chimeric primer-initiated amplification of nucleic acid (ICAN) method, and the smart amplification system (SMAP) method. These methods, as well as others are well known in the art and can be adapted for use in conjunction with provided methods of detection of amplified nucleic acid.

The PCR method is a technique for making many copies of a specific template DNA sequence. The PCR process is disclosed in U.S. Patent Nos. 4,683,195; 4,683,202; and 4,965,188. One set of primers complementary to a template DNA are designed, and a region flanked by the primers is amplified by DNA polymerase in a reaction including multiple amplification cycles. Each amplification cycle includes an initial denaturation, and up to 50 cycles of annealing, strand elongation (or extension) and strand separation (denaturation). In each cycle of the reaction, the DNA sequence between the primers is copied. Primers can bind to the copied DNA as well as the original template sequence, so the total number of copies increases exponentially with time. PCR can be performed as according to Whelan et al., Journal of Clinical Microbiology, 33:556 (1995). Various modified PCR methods are available and well known in the art. Various modifications such as the "RT-PCR" method, in which DNA is synthesized from RNA using a reverse transcriptase before performing PCR; and the "TaqMan® PCR" method, in which only a specific allele is amplified and detected using a fluorescently labeled TaqMan® probe, and Taq DNA polymerase, are known to those skilled in the art. RT-PCR and variations thereof have been described, for example, in U.S. Patent Nos. 5,804,383; 5,407,800; 5,322,770; and 5,310,652, and references described therein; and TaqMan® PCR and related reagents for use in the method have been described, for example, in U.S .Patent Nos. 5,210,015; 5,876,930; 5,538,848; 6,030,787; and 6,258,569.

In some embodiments, asymmetric PCR is performed to preferentially amplify one strand of a double-stranded DNA template. Asymmetric PCR typically involves addition of an excess of the primer for the strand targeted for amplification. An exemplary asymmetric PCR condition is 300 nM of the excess primer and 75nM of the limiting primer to favor single strand amplification. In other embodiments, 400 nM of the excess primer and 100 nM of the limiting primer may be used to favor single strand amplification.

In some embodiments, including embodiments that employ multiplexed PCR reactions, hot start PCR conditions may be used to reduce mis-priming, primer-dimer formation, improve yield, and/or and ensure high PCR specificity and sensitivity. A variety of approaches may be employed to achieve hot start PCR conditions, including hot start DNA polymerases (e.g., hot start DNA polymerases with aptamer-based inhibitors or with mutations that limit activity at lower temperatures) as well as hot start dNTPs (e.g., CLEANAMP™ dNTPs, TriLink Biotechnologies).

In some embodiments, a PCR reaction may include from about 20 cycles to about 55 cycles or more (e.g., about 20, 25, 30, 35, 40, 45, 50, or 55 cycles).

LCR is a method of DNA amplification similar to PCR, except that it uses four primers instead of two and uses the enzyme ligase to ligate or join two segments of DNA. Amplification can be performed in a thermal cycler (e.g., LCx of Abbott Labs, North Chicago, IL). LCR can be performed for example, as according to Moore et al., Journal of Clinical Microbiology 36:1028 (1998). LCR methods and variations have been described, for example, in European Patent Application Publication No. EP0320308, and U.S. Patent No. 5,427,930.

The TAS method is a method for specifically amplifying a target RNA in which a transcript is obtained from a template RNA by a cDNA synthesis step and an RNA transcription step. In the cDNA synthesis step, a sequence recognized by a DNA-dependent RNA polymerase (i.e., a polymerase-binding sequence or PBS) is inserted into the cDNA copy downstream of the target or marker sequence to be amplified using a two-domain oligonucleotide primer. In the second step, an RNA polymerase is used to synthesize multiple copies of RNA from the cDNA template. Amplification using TAS requires only a few cycles because DNA-dependent RNA transcription can result in 10-1000 copies for each copy of cDNA template. TAS can be performed according to Kwoh et al., Proc. Natl. Acad. Sci. USA 86:1173 (1989). The TAS method has been described, for example, in International Patent Application Publication No. WO1988/010315.

Transcription mediated amplification (TMA) is a transcription-based isothermal amplification reaction that uses RNA transcription by RNA polymerase and DNA transcription by reverse transcriptase to produce an RNA amplicon from target nucleic acid. TMA methods are advantageous in that they can produce 100 to 1000 copies of amplicon per amplification cycle, as opposed to PCR or LCR methods that produce only 2 copies per cycle. TMA has been described, for example, in U.S. Patent No. 5,399,491. NASBA is a transcription-based method which for specifically amplifying a target RNA from either an RNA or DNA template. NASBA is a method used for the continuous amplification of nucleic acids in a single mixture at one temperature. A transcript is obtained from a template RNA by a DNA-dependent RNA polymerase using a forward primer having a sequence identical to a target RNA and a reverse primer having a sequence complementary to the target RNA a on the 3' side and a promoter sequence that recognizes T7 RNA polymerase on the 5' side. A transcript is further synthesized using the obtained transcript as template. This method can be performed as according to Heim, et al., Nucleic Acids Res. 26:2250 (1998). The NASBA method has been described in U.S. Patent No. 5,130,238.

The SDA method is an isothermal nucleic acid amplification method in which target DNA is amplified using a DNA strand substituted with a strand synthesized by a strand substitution type DNA polymerase lacking 5' - >3' exonuclease activity by a single stranded nick generated by a restriction enzyme as a template of the next replication. A primer containing a restriction site is annealed to template, and then amplification primers are annealed to 5' adjacent sequences (forming a nick). Amplification is initiated at a fixed temperature. Newly synthesized DNA strands are nicked by a restriction enzyme and the polymerase amplification begins again, displacing the newly synthesized strands. SDA can be performed according to Walker, et al., Proc. Natl. Acad. Sci. USA 89:392 (1992). SDA methods have been described in U.S. Patent Nos. 5,455,166 and 5,457,027.

The LAMP method is an isothermal amplification method in which a loop is always formed at the 3' end of a synthesized DNA, primers are annealed within the loop, and specific amplification of the target DNA is performed isothermally. LAMP can be performed according to Nagamine et al., Clinical Chemistry 47:1742 (2001). LAMP methods have been described in U.S. Patent Nos. 6,410,278; 6,974,670; and 7,175,985.

The ICAN method is anisothermal amplification method in which specific amplification of a target DNA is performed isothermally by a strand substitution reaction, a template exchange reaction, and a nick introduction reaction, using a chimeric primer including RNA-DNA and DNA polymerase having a strand substitution activity and RNase H. ICAN can be performed according to Mukai et al., J. Biochem. 142: 273 (2007). The ICAN method has been described in U.S. Patent No. 6,951,722.

The SMAP (MITANI) method is a method in which a target nucleic acid is continuously synthesized under isothermal conditions using a primer set including two kinds of primers and DNA or RNA as a template. The first primer included in the primer set includes, in the 3' end region thereof, a sequence (Ac') hybridizable with a sequence (A) in the 3' end region of a target nucleic acid sequence as well as, on the 5' side of the above-mentioned sequence (Ac'), a sequence (B') hybridizable with a sequence (Bc) complementary to a sequence (B) existing on the 5' side of the above-mentioned sequence (A) in the above-mentioned target nucleic acid sequence. The second primer includes, in the 3' end region thereof, a sequence (Cc') hybridizable with a sequence (C) in the 3' end region of a sequence complementary to the above-mentioned target nucleic acid sequence as well as a loopback sequence (D-Dc') including two nucleic acid sequences hybridizable with each other on an identical strand on the 5' side of the above-mentioned sequence (Cc'). SMAP can be performed according to Mitani et al., Nat. Methods 4(3): 257 (2007). SMAP methods have been described in U.S. Patent Application Publication Nos. 2006/0160084, 2007/0190531 and 2009/0042197.

The amplification reaction can be designed to produce a specific type of amplified product, such as nucleic acids that are double stranded; single stranded; double stranded with 3' or 5' overhangs; or double stranded with chemical ligands on the 5' and 3' ends. The amplified PCR product can be detected by: (i) hybridization of the amplified product to magnetic particle bound complementary oligonucleotides, where two different oligonucleotides are used that hybridize to the amplified product such that the nucleic acid serves as an interparticle tether promoting particle agglomeration; (ii) hybridization mediated detection where the DNA of the amplified product must first be denatured; (iii) hybridization mediated detection where the particles hybridize to 5' and 3' overhangs of the amplified product; (iv) binding of the particles to the chemical or biochemical ligands on the termini of the amplified product, such as streptavidin functionalized particles binding to biotin functionalized amplified product.

The methods of the invention can be used to perform real time PCR and provide quantitative information about the amount of target nucleic acid present in a sample (see, e.g., Figure 52 and Example 18 of WO 2012/054639). Methods for conducting quantitative real time PCR are provided in the literature (see for example: RT-PCR Protocols. Methods in Molecular Biology, Vol. 193. Joe O'Connell, ed. Totowa, NJ: Humana Press, 2002, 378 pp. ISBN 0-89603-875-0.). Example 18 of WO 2012/054639 describes use of the methods of the invention for real time PCR analysis of a whole blood sample.

The methods of the invention can be used to perform real time PCR directly in opaque samples, such as whole blood, using magnetic nanoparticles modified with capture probes and magnetic separation. Using real-time PCR allows for the quantification of a target nucleic acid without opening the reaction tube after the PCR reaction has commenced.

In one approach, biotin or avidin labeled primers can be used to perform real-time PCR. These labels would have corresponding binding moieties on the magnetic particles that could have very fast binding times. This allows for a double stranded product to be generated and allows for much faster particle binding times, decreasing the overall turnaround time. The binding chemistry would be reversible, preventing the primers from remaining particle bound. In order to reverse the binding, the sample can be heated or the pH adjusted.

In another approach, the real-time PCR can be accomplished through the generation of duplex DNA with overhangs that can hybridize to the superparamagnetic particles. Additionally, LNA and/or fluorinated capture probes may speed up the hybridization times.

In still another approach, the particles are designed to have a hairpin that buries the binding site to the amplicon. Heating the particles to a higher melt temperature would expose the binding site of the hairpin to allow binding to the target.

In another approach, a probe that hybridizes to an amplicon is tethering two (or more) particles. The reaction would be conducted in the presence of a polymerase with 5' exonuclease activity, resulting in the cleavage of the inter-particle tether and a subsequent change in T₂. The polymerase is selected to have exonuclease activity and compatibility with the matrix of choice (e.g. blood). In this approach, smaller particles (e.g., 30 nm CLIO) can be used to reduce steric hindrance of the hybridization to target or subsequent enzymatic digestion during polymerization (see, e.g., Heid et al. Genome Research 1996 6: 986-994).

In another approach, two particle populations can be synthesized to bear complementary capture probes. In the absence of amplicon, the capture probes hybridize promoting particle clustering. Upon generation of amplicon, the amplicon can compete, hybridize, and displace the capture probes leading to particle declustering. The method can be conducted in the presence or absence of nanoparticles. The particles free in solution will cluster and decluster due to the thermocycling (because, e.g., the Tm can be below 95°C). The Tm of the amplicon binding to one of the particle-immobilized capture probes can be designed such that that binding interaction is more favorable than the particle-to-particle binding interaction (by, e.g., engineering point mutations within the capture probes to thermodynamically destabilize the duplexes). In this embodiment, the particle concentration can be kept at, e.g., low or high levels.

Previous work showed that in some cases the presence of particles in the PCR reaction could inhibit PCR. For these inhibitory particles, it is envisioned that the particles could be pulled to the side of the tube (or other location within the container) to keep them out of solution during the PCR reaction. Methods can be used to release the particles back into suspension to allow them to hybridize to the PCR product and then pull them back out of solution.

In certain embodiments, the invention features the use of enzymes compatible with whole blood, including but not limited to NEB HemoKlenTaq™, DNAP OmniKlenTaq™, Kapa Biosystems whole blood enzyme, and Thermo-Fisher Finnzymes PHUSION® enzyme.

The invention also features quantitative asymmetric PCR. In any of the real-time PCR methods of the invention, the method can involve the following steps:
1. aliquoting whole blood into a prepared PCR mastermix containing superparamagnetic particles;
2. prior to the first PCR cycle, closing the tube until PCR cycling is completed;
3. loading the tube onto thermal cycler;
4. running "n" cycles of standard PCR thermal cycling;
5. conducting a T₂ detection (the exact time duration and steps for this vary depending on the biochemical and particle design approach described below); and
6. repeating steps 4 and 5 until enough T₂ readings have been taken for an accurate quantification of initial target concentration.

The above methods can be used with any of the following categories of detection of aggregation or disaggregation described herein, including those described in Table 3.

**Table 3: Categories of Detection of Aggregation or Disaggregation**

| Name | Description |
|---|---|
| Clustering-based detection and magnetic separation | • Particles >100 nm or magnetic-separation compatible. |
| | • Particles removed from solution during PCR |
| | • T₂ goes up with amplicon generation |
| | • Agitation during step 5 |
| Clustering-based detection with particles >100 nm | • Particles >100 nm |
| | • Particles do not inhibit PCR |
| | • T₂ goes up with amplicon generation |
| | • Agitation during step 5 |
| De-clustering-based detection and magnetic separation | • Particles >100 nm |
| | • Particles on the side of the tube during PCR |
| | • T₂ goes down with amplicon generation |
| | • Agitation during step 5 |
| De-clustering-based detection with particles >100 nm | • Particles >100 nm |
| | • Particles do not inhibit PCR |
| | • T₂ goes down with amplicon generation |
| | • Agitation during step 5 |
| Clustering-based detection with particles <100 nm | • Particles <100 nm (e.g., 30 nm particles) |
| | • T₂ goes down with amplicon appearance (at least for initial cycles, T₂ may subsequently increase as cluster size increases) |
| | • Has potential for much more rapid hybridization times |
| | • No agitation required to keep particles suspended |
| | • Particle concentration in nM range |
| De-clustering-based detection with particles <100 nm | • Particles <100 nm (e.g., 30 nm particles) |
| | • T₂ goes up with amplicon appearance |
| | • T₂ could decrease as the cluster size increase above 100nm |
| | • No agitation required to keep particles suspended |
| | • Has potential for most rapid detection times |
| | • Particle concentration in nM range |

### Contamination control

One potential problem in the use of PCR as an analytical tool is the risk of having new reactions contaminated with old, amplified products. Potential sources of contamination include a) large numbers of target organisms in clinical specimens that may result in cross-contamination, b) plasmid clones derived from organisms that have been previously analyzed and that may be present in larger numbers in the laboratory environment, and c) repeated amplification of the same target sequence leading to accumulation of amplification products in the laboratory environment. A common source of the accumulation of the PCR amplicon is aerosolization of the product. Typically, if uncontrolled aerosolization occurs, the amplicon will contaminate laboratory reagents, equipment, and ventilation systems. When this happens, all reactions will be positive, and it is not possible to distinguish between amplified products from the contamination or a true, positive sample. In addition to taking precautions to avoid or control this carry-over of old products, preferred embodiments include a blank reference reaction in every PCR experiment to check for carry-over. For example, carry-over contamination will be visible on the agarose gel as faint bands or fluorescent signal when TaqMan® probes, molecular beacons, or intercalating dyes, among others, are employed as detection mechanisms. Furthermore, it is preferred to include a positive sample. As an example, in some embodiments, contamination control is performed using any of the approaches and methods described in WO 2012/054639. In some embodiments, a bleach solution is used to neutralize potential amplicons, for example, in a reaction tube of a T2Dx® device being used to perform a method of the invention.

### Systems

The description provides systems for carrying out the methods of the invention, which may include one or more NMR units, MAA units, cartridge units, and agitation units, as described in WO 2012/054639. The system may also include an AST unit for determination of susceptibility of a pathogen to a selected number of antimicrobial agents. Such systems may further include other components for carrying out an automated assay, such as a PCR unit for the detection of oligonucleotides; a centrifuge, a robotic arm for delivery an liquid sample from unit to unit within the system; one or more incubation units; a fluid transfer unit (i.e., pipetting device) for combining assay reagents and a biological sample to form the liquid sample; a computer with a programmable processor for storing data, processing data, and for controlling the activation and deactivation of the various units according to a one or more preset protocols; and a cartridge insertion system for delivering pre-filled cartridges to the system, optionally with instructions to the computer identifying the reagents and protocol to be used in conjunction with the cartridge. Figure 42 of WO 2012/054639 depicts an exemplary system for carrying out the methods of the invention.

The systems can provide an effective means for high throughput and real-time detection of analytes present in a bodily fluid from a subject. The detection methods may be used in a wide variety of circumstances including, without limitation, identification and/or quantification of analytes that are associated with specific biological processes, physiological conditions, disorders or stages of disorders. As such, the systems have a broad spectrum of utility in, for example, disease diagnosis, parental and forensic identification, disease onset and recurrence, individual response to treatment versus population bases, and monitoring of therapy. The devices and systems can provide a flexible system for personalized medicine. The system can be changed or interchanged along with a protocol or instructions to a programmable processor of the system to perform a wide variety of assays as described herein. The systems may offer many advantages of a laboratory setting contained in a desk-top or smaller size automated instrument.

The systems can be used to simultaneously assay analytes that are present in the same liquid sample over a wide concentration range, and can be used to monitor the rate of change of an analyte concentration and/or or concentration of PD or PK markers over a period of time in a single subject, or used for performing trend analysis on the concentration, or markers of PD, or PK, whether they are concentrations of drugs or their metabolites. Thus, the data generated with the use of the subject fluidic devices and systems can be utilized for performing a trend analysis on the concentration of an analyte in a subject.

For example, a subject (e.g., a patient having or suspected of having a disease caused by or associated with a microbial pathogen) may be provided with a plurality of cartridge units to be used for detecting a variety of analytes, such as analytes sampled from different tissues, and at predetermined times. A subject may, for example, use different cartridge units on different days of the week. The software on the system may be designed to recognize an identifier on the cartridge instructing the system computer to run a particular protocol for running the assay and/or processing the data. The protocols on the system can be updated through an external interface, such as an USB drive or an Ethernet connection, or the entire protocol can be recorded in the barcode attached to the cartridge. The protocol can be optimized as needed by prompting the user for various inputs (i.e., for changing the dilution of the sample, the amount of reagent provided to the liquid sample, altering an incubation time or MAA time, or altering the NMR relaxation collection parameters).

A multiplexed assay can be performed using a variety of system designs. For example, a multiplexed assay can performed using any of the following configurations:
(i) a spatially-based detection array can be used to direct magnetic particles to a particular region of a tube (i.e., without aggregation) and immobilize the particles in different locations according to the particular analyte being detected. The immobilized particles are detected by monitoring their local effect on the relaxation effect at the site of immobilization. The particles can be spatially separated by gravimetric separation in flow (i.e., larger particles settling faster along with a slow flow perpendicular to gravity to provide spatial separation based on particle size with different magnetic particle size populations being labeled with different targets). Alternatively, of capture probes can be used to locate magnetic particles in a particular region of a tube (i.e., without aggregation) and immobilize the particles in different locations (i.e., on a functionalized surface, foam, or gel). Optionally, the array is flow through system with multiple coils and magnets, each coil being a separate detector that has the appropriate particles immobilized within it, and the presence of the analyte detected with signal changes arising from clustering in the presence of the analyte. Optionally, once the particles are spatially separated, each individual analyte in the multiplexed assay can be detected by sliding a coil across the sample to read out the now spatially separated particles.
(ii) A microfluidic tube where the sample is physically split amongst many branches and a separate signal is detected in each branch, each branch configured for detection of a separate analyte in the multiplexed assay.
(iii) An array of 96 wells (or less or more) where each well has its own coil and magnet, and each well is configured for detection of a separate analyte in the multiplexed assay.
(iv) A sipper or flow through device with multiple independently addressable coils inside one magnet or inside multiple mini magnets that can be used for sequential readings, each reading being a separate reaction for detection of a separate analyte in the multiplexed assay.
(v) A sipper or flow through device with multiple independently addressable wells on a plate inside one magnet or inside multiple mini magnets that can be used for sequential readings using a single sided coil that can be traversed along the plate, each reading being a separate reaction for detection of a separate analyte in the multiplexed assay.
(vi) A tube containing two compartments read simultaneously, resulting in one relaxation curve which is then fit using bi-exponential fitting to produce the separate readings for the multiplexed array.
(vii) A microfluidics system where each droplet of liquid is moved around individually, to produce readings for the multiplexed array.
(viii) Sequential measurements using magnetic separation and resuspension requires novel binding probes or the ability to turn them on and off. This method would be used for nucleic acid analytes in which turn on/off mechanism is based mostly on melting temperature (at higher temperatures hairpin loops relax, denaturation of double strand binding), and hybridization will occur at different temperatures.
(ix) Individual capillaries, each equipped with dried particles within them, allow for small volume rapid multiplexing of one small aliquot. The dried particles are spatially separated, and this spatial separation permits the MR Reader to read each capillary tube independently.
(x) Binding moieties conjugated to nanoparticles are placed in a gel or other viscous material forming a region and analyte specific viscous solution. The gel or viscous solution enhances spatial separation of more than one analyte in the starting sample because after the sample is allowed to interact with the gel, the target analyte can readily diffuse through the gel and specifically bind to a conjugated moiety on the gel or viscous solution held nanoparticle. The clustering or aggregation of the specific analyte, optionally enhanced via one of the described magnetic assisted agglomeration methods, and detection of analyte specific clusters can be performed by using a specific location NMR reader. In this way a spatial array of nanoparticles, and can be designed, for example, as a 2d array.
(xi) Magnetic particles can be spotted and dried into multiple locations in a tube and then each location measured separately. For example, one type of particle can be bound to a surface and a second particle suspended in solution, both of which hybridize to the analyte to be detected. Clusters can be formed at the surface where hybridization reactions occur, each surface being separately detectable.
(xii) A spotted array of nucleic acids can be created within a sample tube, each configured to hybridize to a first portion of an array of target nucleic acids. Magnetic particles can be designed with probes to hybridize to a second portion of the target nucleic acid. Each location can be measured separately. Alternatively, any generic beacon or detection method could be used to produce output from the nucleic acid array.
(xiii) An array of magnetic particles for detecting an array of targets can be included in a single sample, each configured (e.g., by size, or relaxation properties) to provide a distinct NMR relaxation signature with aggregate formation. For example, each of the particles can be selected to produce distinct T₂ relaxation times (e.g., one set of particles covers 10-200 ms, a second set from 250-500 ms, a third set from 550-1100 ms, and so on). Each can be measured as a separate band of relaxation rates.
(xiv) For detection of analytes of various size or magnetic particles, or aggregates of various size, a single sample with multiple analytes and magnetic particles can undergo separation in the presence of a magnetic or electric field (i.e., electrophoretic separation of magnetic particles coated with analytes), the separate magnetic particles and/or aggregates reaching the site of a detector at different times, accordingly.
(xv) The detection tube could be separated into two (or more) chambers that each contain a different nanoparticle for detection. The tube could be read using the reader and through fitting a multiple exponential curve such as A*exp(T_{2_}1) + B*exp(T_{2_}2), the response of each analyte could be determined by looking at the relative size of the constants A and B and T_{2_}1 and T_{2_}2.
(xvi) Gradient magnetic fields can be shimmed to form narrow fields. Shim pulses or other RF based Shimming within a specific field can be performed to pulse and receive signals within a specific region. In this way one could envision a stratification of the RF pulse within a shim and specific resonance signals could be received from the specific shim. While this method relies on shimming the gradient magnetic field, multiplexing would include then, to rely on one of the other methods described to get different nanoparticles and the clusters to reside in these different shims. Thus there would be two dimensions, one provided by magnetic field shims and a second dimension provided by varying nanoparticle binding to more than one analyte. Nanoparticles having two distinct NMR relaxation signals upon clustering with an analyte may be employed in a multiplexed assay. In this method, the observation that small particles (30-200 nm) cause a decrease in T₂ with clustering whereas large particles (>800 nm) cause an increase with clustering. The reaction assay is designed as a competitive reaction, so that with the addition of the target it changes the equilibrium relaxation signal. For example, if the T₂ relaxation time is shorter, clusters forming of analyte with small particles are forming. If on the other hand, the T₂ relaxation becomes longer, clusters of analyte with larger particles are forming. It's probably useful to change the density/viscosity of the solution with additives such as trehalose or glucose or glycerol to make sure the big particles stay in solution. One nanoparticle having binding moieties to a specific analyte for whose T₂ signal is decreased on clustering may be combined with a second nanoparticle having a second binding moiety to a second analyte for whose T₂ signal is increased on clustering. In the case for which the sample is suspected to have both analytes and the clustering reaction may cancel each other out (the increased clustering cancels the decreased clustering), one could envision an ordering of the analysis, i.e. addition of competitive binding agents to detect a competitive binding and thus T₂ signal that would be related to the presence/absence of the analyte of interest in the sample. Alternatively, if the increased clustering cancels the decreased clustering in this multiplexing format, one could envision use of different relaxation pulse sequences or relaxation determinants to identify the presence/absence or concentration of analyte in the sample.
(xvii) Precipitation measurement of particles. In this method, multiple types of particles designed to capture different target sequences of nucleic acid are designed So that the particle size is small enough that the particles bound with analyte remain suspended in solution. Sequential addition of an "initiator" sequence that is complementary to a nucleic acid sequence conjugated to a second set of particles (a larger particle, not necessarily having magnetic properties) and contains a complementary sequence to the captured target DNA sequence. After hybridization, clusters will form if the target DNA sequence is present, e.g. the magnetic nanoparticle conjugated with probe anneals to one specific sequence on the target analyte and the other particle binds to another sequence on the target nucleic acid sequence. These clusters will be big enough to precipitate (this step may require a centrifugation step). In the same reaction, and simultaneously, one could design an additional magnetic particle, second particle set to anneal with a second nucleic acid sequence for which formation of the magnetic nanoparticle-analyte-second particle clusters do not precipitate. In this way sequential addition of particles can result in differential signaling.
(xvii) One possible different detection technique includes phase separated signals, which would stem from differing RF coil pulse sequences that are optimized for the conjugated nanoparticle-analyte interaction. Optimally, this could be achieved with multiple coils in an array that would optimize the ability of the different RF pulses and relaxation signal detection to be mapped and differentiated to ascertain the presence/absence of more than one analyte. Multiplexing may also employ the unique characteristic of the nanoparticle-analyte clustering reaction and subsequent detection of water solvent in the sample, the ability of the clusters to form various "pockets" and these coordinated clusters to have varying porosity. For example, linkers having varying length or conformational structures can be employed to conjugate the binding moiety to the magnetic nanoparticle. In this way, more than one type of cluster formed in the presence of an analyte could be designed having the ability of differing solvent water flow, and thus relaxation signal differences, through the aggregated nanoparticle-analyte-nanoparticle formation. In this way, two or more linker/binding moiety designs would then allow for detection of more than one analyte in the same sample.
(xviii) The methods of the invention can include a fluorinated oil/aqueous mixture for capturing particles in an emulsion. In this design one hydrophobic capture particle set and an aqueous capture set are used, the hydrophobic capture particle set is designed to bind and aggregate more readily in an hydrophobic environment, whereas the aqueous capture particle set is designed to bind and aggregate in an aqueous environment. Introduction of an analyte containing sample having specific analytes that will bind to either the hydrophobic or aqueous particle, and subsequent mixing in the detection tube having both hydrophobic and aqueous solvents, binding and clustering would then result in a physical separation of analytes to either the aqueous or hydrophobic phase. The relaxation signal could be detected in either solution phase. In the event that the analytes and nanoparticles designed in this manner are physically found in an emulsion created by the mixing of the hydrophobic /aqueous phases, relaxation curves would be distinguishable in the emulsion phase. The detection tube may have a capsular design to enhance the ability to move the capsules through an MR detector to read out the signal. Further, additional use of a fluorescent tag to read out probe identity may be employed, i.e., in the case of two different analytes in the same aqueous or hydrophobic phase, the addition of a fluorescent tag can assist determination of the identity of the analyte. This method is amenable in samples for which limited isolation or purification of the target analyte away from the other material in the sample because the described resonance signals are independent of sample quality. Further, the addition of the fluorescent tag can be added in much higher concentrations that usually added in typical fluorescent studies because these tags will never interfere with the relaxation measurements. In this method, oligonucleotide capture probes that are conjugated to the magnetic nanoparticles are designed so that specific restriction endonuclease sites are located within the annealed section. After hybridization with the sample forming nanoparticle-analyte clusters, a relaxation measurement then provides a base signal. Introduction of a specific restriction endonuclease to the detection tube and incubation will result in a specific reduction of the nanoparticle/analyte cluster after restriction digestion has occurred. After a subsequent relaxation measurement, the pattern of signal and restriction enzyme digestion, one can deduce the target.
(xix) In a combined method, a magnetic nanoparticle is conjugated with two separate and distinct binding moieties, i.e. an oligonucleotide and an antibody. This nanoparticle when incubated with a sample having both types of analytes in the sample will form nanoparticle-analyte complexes, and a baseline T₂ relaxation signal will be detectable. Subsequent addition of a known concentration of one of the analytes can be added to reduce the clustering formed by that specific analyte from the sample. After known analyte addition a subsequent T₂ relaxation signal is detected and the presence/absence of the sample analyte can be surmised. Further, a second analyte can be added to compete with the analyte in the sample to form clusters. Again, after a subsequent T₂ relaxation signal detection the presence/absence of the second sample analyte can be surmised. This can be repeated.

Broadly, a multiplexed assay employing the methods of this invention can be designed so that the use of one non-superparamagnetic nanoparticle to generate clusters with analyte from a sample, will reduce the overall Fe²⁺ in assay detection vessel and will extend the dynamic range so that multiple reactions can be measured in the same detection vessel.

Multiplexing nucleic acid detection can make use of differing hybridization qualities of the conjugated magnetic nanoparticle and the target nucleic acid analyte. For example, capture probes conjugated to magnetic nanoparticles can be designed so that annealing the magnetic nanoparticle to the target nucleic acid sequence is different for more than one nucleic acid target sequence. Factors for the design of these different probe-target sequences include G-C content (time to form hybrids), varying salt concentration, hybridization temperatures, and/or combinations of these factors. This method then would entail allowing various nucleic acid conjugated magnetic nanoparticles to interact with a sample suspected of having more than one target nucleic acid analyte. Relaxation times detected after various treatments, i.e. heating, addition of salt, hybridization timing, would allow for the ability to surmise which suspected nucleic acid sequence is present or absent in the sample.

Use complimentary amplicons to block one reaction and allow serial hybridizations. In this method, universal amplification primers are used to amplify more than one specific nucleic acid sequence in the starting sample, forming an amplicon pool. Specific oligonucleotides conjugated to magnetic nanoparticles are added to the sample and a relaxation measurement is taken. The sample is then exposed to a temperature to melt the oligonucleotide-analyte interaction and addition of a oligonucleotide that is not attached to a magnetic nanoparticle is added to compete away any analyte binding to the magnetic nanoparticle. A second magnetic nanoparticle having a second oligonucleotide conjugated to it is then added to form clusters with a second specific target nucleic acid analyte. Alternatively, the method could have a step prior to the addition of the second magnetic nanoparticle that would effectively sequester the first magnetic nanoparticle from the reaction vessel, i.e. exposing the reaction vessel to a magnetic field to move the particles to an area that would not be available to the second, or subsequent reaction.

Each of the multiplexing methods above can employ a step of freezing the sample to slow diffusion and clustering time and thus alter the measurement of the relaxation time. Slowing the diffusion and clustering of the method may enhance the ability to separate and detect more than one relaxation time. Each of the multiplexing methods above can make use of sequential addition of conjugated nanoparticles followed by relaxation detection after each addition. After each sequential addition, the subsequent relaxation baseline becomes the new baseline from the last addition and can be used to assist in correlating the relaxation time with presence/absence of the analyte or analyte concentration in the sample.

In some embodiments, the method of multiplexing may involve hidden capture probes. In this method of multiplexing, oligonucleotides conjugated to the magnetic nanoparticles are designed so that secondary structure or a complementary probe on the surface of the particle hides or covers the sequence for hybridization initially in the reaction vessel. These hidden hybridization sequences are then exposed or revealed in the sample vessel spatially or temporally during the assay. For example, as mentioned above, hybridization can be affected by salt, temperature and time to hybridize. Thus, in one form of this method, secondary or complementary structures on the oligonucleotide probe conjugated to the magnetic nanoparticle can be reduced or relaxed to then expose or reveal the sequence to hybridize to the target nucleic acid sample. Further, secondary structures could be reduced or relaxed using a chemical compound, e.g., DMSO. Another method to selectively reveal or expose a sequence for hybridization of the oligonucleotide conjugated nanoparticle with the target analyte is to design stem-loop structures having a site for a restriction endonuclease; subsequent digestion with a restriction endonuclease would relax the stem-loop structure and allow for hybridization to occur. Alternatively, a chemical cut of the stem-loop structure, releasing one end could make the sequence free to then hybridize to the target nucleic acid sequence.

Where the multiplexed array is configured to detect a target nucleic acid, the assay can include a multiplexed PCR to generate different amplicons and then serially detect the different reactions.

The multiplexed assay optionally includes a logical array in which the targets are set up by binary search to reduce the number of assays required (e.g., gram positive or negative leads to different species based tests that only would be conducted for one group or the other).

The systems can run a variety of assays, regardless of the analyte being detected from a bodily fluid sample. A protocol dependent on the identity of the cartridge unit being used can be stored on the system computer. In some embodiments, the cartridge unit has an identifier (ID) that is detected or read by the system computer, or a bar code (1D or 2D) on a card that then supplies assay specific or patient or subject specific information needed to be tracked or accessed with the analysis information (e.g., calibration curves, protocols, previous analyte concentrations or levels). Where desired, the cartridge unit identifier is used to select a protocol stored on the system computer, or to identify the location of various assay reagents in the cartridge unit. The protocol to be run on the system may include instructions to the controller of the system to perform the protocol, including but not limited to a particular assay to be run and a detection method to be performed. Once the assay is performed by the system, data indicative of an analyte in the biological sample is generated and communicated to a communications assembly, where it can either be transmitted to the external device for processing, including without limitation, calculation of the analyte concentration in the sample, or processed by the system computer and the result presented on a display readout.

For example, the identifier may be a bar code identifier with a series of black and white lines, which can be read by a bar code reader (or another type of detector) upon insertion of the cartridge unit. Other identifiers could be used, such as a series of alphanumerical values, colors, raised bumps, RFID, or any other identifier which can be located on a cartridge unit and be detected or read by the system computer. The detector may also be an LED that emits light which can interact with an identifier which reflects light and is measured by the system computer to determine the identity of a particular cartridge unit. The system may include a storage or memory device with the cartridge unit or the detector for transmitting information to the system computer.

Thus, the systems can include an operating program to carry out different assays, and cartridges encoded to: (i) report to the operating program which pre-programmed assay was being employed; (ii) report to the operating program the configuration of the cartridges; (iii) inform the operating system the order of steps for carrying out the assay; (iv) inform the system which pre-programmed routine to employ; (v) prompt input from the user with respect to certain assay variables; (vi) record a patient identification number (the patient identification number can also be included on the vacutainer holding the blood sample); (vii) record certain cartridge information (e.g., lot number, calibration data, assays on the cartridge, analytic data range, expiration date, storage requirements, acceptable sample specifics); or (viii) report to the operating program assay upgrades or revisions (i.e., so that newer versions of the assay would occur on cartridge upgrades only and not to the larger, more costly system).

The systems can include one or more fluid transfer units configured to adhere to a robotic arm (see, e.g., Figures 43A-43C of WO 2012/054639). The fluid transfer unit can be a pipette, such as an air-displacement, liquid backed, or syringe pipette. For example, a fluid transfer unit can further include a motor in communication with a programmable processor of the system computer and the motor can move the plurality of heads based on a protocol from the programmable processor. Thus, the programmable processor of a system can include instructions or commands and can operate a fluid transfer unit according to the instructions to transfer liquid samples by either withdrawing (for drawing liquid in) or extending (for expelling liquid) a piston into a closed air space. Both the volume of air moved and the speed of movement can be precisely controlled, for example, by the programmable processor. Mixing of samples (or reagents) with diluents (or other reagents) can be achieved by aspirating components to be mixed into a common tube and then repeatedly aspirating a significant fraction of the combined liquid volume up and down into a tip. Dissolution of reagents dried into a tube can be done is similar fashion.

A system can include one or more incubation units for heating the liquid sample and/or for control of the assay temperature. Heat can be used in the incubation step of an assay reaction to promote the reaction and shorten the duration necessary for the incubation step. A system can include a heating block configured to receive a liquid sample for a predetermined time at a predetermined temperature. The heating block can be configured to receive a plurality of samples.

The system temperature can be carefully regulated. For example, the system includes a casing kept at a predetermined temperature (e.g., 37°C) using stirred temperature controlled air. Waste heat from each of the units will exceed what can be passively dissipated by simple enclosure by conduction and convection to air. To eliminate waste heat, the system can include two compartments separated by an insulated floor. The upper compartment includes those portions of the components needed for the manipulation and measurement of the liquid samples, while the lower compartment includes the heat generating elements of the individual units (e.g., the motor for the centrifuge, the motors for the agitation units, the electronics for each of the separate units, and the heating blocks for the incubation units). The lower floor is then vented and forced air cooling is used to carry heat away from the system. See, e.g., Figures 44A and 44B of WO 2012/054639.

The MR unit may require more closely controlled temperature (e.g., ± 0.1°C), and so may optionally include a separate casing into which air heated at a predetermined temperature is blown. The casing can include an opening through which the liquid sample is inserted and removed, and out of which the heated air is allowed to escape. See, e.g., Figures 45A and 45B of WO 2012/054639. Other temperature control approaches may also be utilized.

A system described herein may further include an AST unit configured for testing antimicrobial susceptibility of a pathogen. The AST unit may include, for example, a tray comprising a plurality of wells each of which is suitable for holding a pathogen culture. Aliquots of a pathogen culture may be automatically dispensed into the plurality of wells by a dispenser. A suitable panel of antimicrobial agents may be individually added to the plurality of wells by a dispenser. The tray may be incubated under suitable conditions for growth of the pathogen (e.g., temperature, oxygen content, and the like). The AST unit may further include a detection module to detect the growth of the pathogen in the wells of the tray. The detection module may be configured for turbidometric, fluorometric, or spectrometric detection of growth. The results of the AST testing may be analyzed and stored using a computer.

### Cartridge Units

The invention provides methods that may involve one or more cartridge units to provide a convenient method for placing all of the assay reagents and consumables onto a system. For example, the system may be customized to perform a specific function, or adapted to perform more than one function, e.g., via changeable cartridge units containing arrays of micro wells with customized magnetic particles contained therein. The system can include a replaceable and/or interchangeable cartridge containing an array of wells pre-loaded with magnetic particles, and designed for detection and/or concentration measurement of a particular analyte. Alternatively, the system may be usable with different cartridges, each designed for detection and/or concentration measurements of different analytes, or configured with separate cartridge modules for reagent and detection for a given assay. The cartridge may be sized to facilitate insertion into and ejection from a housing for the preparation of a liquid sample which is transferred to other units in the system (e.g., a magnetic assisted agglomeration unit, or an NMR unit). The cartridge unit itself could potentially interface directly with manipulation stations as well as with the MR reader(s). The cartridge unit can be a modular cartridge having an inlet module that can be sterilized independent of the reagent module.

For handling biological samples, such as blood samples, there are numerous competing requirements for the cartridge design, including the need for sterility for the inlet module to prevent cross contamination and false positive test results, and the need to include reagents in the package which cannot be easily sterilized using standard terminal sterilization techniques like irradiation. An inlet module for sample aliquoting can be designed to interface with uncapped vacutainer tubes, and to aliquot two a sample volume that can be used to perform, for example, an assay to detect a pathogen (see Figures 7D-7F of WO 2012/054639). The vacutainer permits a partial or full fill. The inlet module has two hard plastic parts, that get ultrasonically welded together and foil sealed to form a network of channels to allow a flow path to form into the first well overflow to the second sample well. A soft vacutainer seal part is used to for a seal with the vacutainer, and includes a port for sample flow, and a venting port. To overcome the flow resistance once the vacutainer is loaded and inverted, some hydrostatic pressure is needed. Every time sample is removed from a sample well, the well will get replenished by flow from the vacutainer.

A modular cartridge can provide a simple means for cross contamination control during certain assays, including but not limited to distribution of PCR products into multiple detection aliquots. In addition, a modular cartridge can be compatible with automated fluid dispensing, and provides a way to hold reagents at very small volumes for long periods of time (in excess of a year). Finally, pre-dispensing these reagents allows concentration and volumetric accuracy to be set by the manufacturing process and provides for a point of care use instrument that is more convenient as it can require much less precise pipetting.

The modular cartridge may be a cartridge that is separated into modules that can be packaged and if necessary sterilized separately. They can also be handled and stored separately, if for example the reagent module requires refrigeration but the detection module does not. Figure 6 of WO 2012/054639 shows a representative cartridge with an inlet module, a reagent module and a detection module that are snapped together. The inlet module may be packaged separately in a sterile package and the reagent and detection modules would be pre-assembled and packaged together.

During storage, the reagent module could be stored in a refrigerator while the inlet module could be stored in dry storage. This provides the additional advantage that only a very small amount of refrigerator or freezer space is required to store many assays. At time of use, the operator would retrieve a detection module and open the package, potentially using sterile technique to prevent contamination with skin flora if required by the assay. The Vacutainer tube is then decapped and the inverted inlet module is placed onto the tube as shown in Figure 7A of WO 2012/054639. This module has been designed to be easily moldable using single draw tooling as shown in Figures 7B and 7C of WO 2012/054639 and the top and bottom of the cartridge are sealed with foil to prevent contamination and also to close the channels. Once the tube has been re-sealed using the inlet module, the assembly is turned right side up and snapped onto the remainder of the cartridge. The inlet section includes a well with an overflow that allows sample tubes with between 2 and 6 ml of blood to be used and still provide a constant depth interface to the system automation. It accomplishes this by means of the overflow shown in Figure 8 of WO 2012/054639, where blood that overflows the sampling well simply falls into the cartridge body, preventing contamination.

Figures 9A-9C of WO 2012/054639 show the means of storing precisely pipetted small volume reagents. The reagents are kept in pipette tips that are shown in Figure 9C of WO 2012/054639. These are filled by manufacturing automation and then are placed into the cartridge to seal their tips in tight fitting wells which are shown in a cutaway view Figure 9B of WO 2012/054639. Finally, foil seals are placed on the back of the tips to provide a complete water vapor proof seal. It is also possible to seal the whole module with a seal that will be removed by the operator, either in place of or in addition to the aforementioned foils. This module also provides storage for empty reaction vessels and pipette tips for use by the instrument while the detection module provides storage for capped 200 µl PCR vials used by the instrument to make final measurements from.

Figures 10-13C of WO 2012/054639 show an alternative detection module of the cartridge which is design to provide for contamination control during, for example, pipetting of post-PCR (polymerase chain reaction) products. This is required because the billion fold amplification produced by PCR presents a great risk of cross contamination and false positives. However, it is desirable to be able to aliquot this mixture safely, because low frequency analytes will have been amplified up and can be distributed for separate detection or identification. There are three ways in which this portion of the cartridge aids in contamination control during this aliquoting operation.

First, the cartridge contains a recessed well to perform the transfer operations in as shown in Figures 10A and 10B of WO 2012/054639. Second, the machine provides airflow through this well and down into the cartridge through holes in the bottom of the well, as shown in Figure 11 of WO 2012/054639. The depth of the well is such that a pipette tip will remain in the airflow and prevent any aerosol from escaping. Figure 12 of WO 2012/054639 depicts a bottom view of the detection module, showing the bottom of the detection tubes and the two holes used to ensure airflow. An optional filter can be inserted here to capture any liquid aerosol and prevent it from entering the machine. This filter could also be a sheet of a hydrophobic material like Gore-tex that will allow air but not liquids to escape. Finally, there is a special seal cap on each 200ul tube to provide a make then break seal for each pipette tip as it enters the vessel, as shown in Figures 13A-13C of WO 2012/054639. It is contemplated that the pipette tip used for aliquoting be stored in this well at all, thus making it possible for the tip never to leave the controlled air flow region.

Alternatively, the modular cartridge is designed for a multiplexed assay. The challenge in multiplexing assays is combining multiple assays which have incompatible assay requirements (i.e., different incubation times and/or temperatures) on one cartridge. The cartridge format depicted in Figures 14A-14C of WO 2012/054639 allows for the combination of different assays with dramatically different assay requirements. The cartridge features two main components: (i) a reagent module (i.e., the reagent strip portion) that contains all of the individual reagents required for the full assay panel (for example, a panel as described below), and (ii) the detection module. A cartridge may be configured to detect from 2 to 24 or more pathogens (e.g., 2, 3, 4, 5, 6, 7, 8, 9, 10, 11, 12, 13, 14, 15, 16, 17, 18, 19, 20, 21, 22, 23, 24, or more pathogens). The detection modules contain only the parts of the cartridge that carry through the incubation, and can carry single assays or several assays, as needed. The detection module depicted in Figure 14B of WO 2012/054639 includes two detection chambers for a single assay, the first detection chamber as the control and the second detection chamber for the sample. This cartridge format is expandable in that additional assays can be added by including reagents and an additional detection module.

The operation of the module begins when the user inserts the entire or a portion of the cartridge into the instrument. The instruments performs the assay actuation, aliquoting the assays into the separate detection chambers. These individual detection chambers are then disconnected from the reagent strip and from each other, and progress through the system separately. Because the reagent module is separated and discarded, the smallest possible sample unit travels through the instrument, conserving internal instrument space. By splitting up each assay into its own unit, different incubation times and temperatures are possible as each multiplexed assay is physically removed from the others and each sample is individually manipulated.

The cartridge units can include one or more populations of magnetic particles, either as a liquid suspension or dried magnetic particles which are reconstituted prior to use. For example, the cartridge units can include a compartment including from 1×10⁶ to 1×10¹³magnetic particles (e.g., from 1×10⁶ to 1×10⁸, 1×10⁷ to 1×10⁹, 1×10⁸ to 1×10¹⁰, 1×10⁹ to 1×10¹¹, 1×10¹⁰ to 1×10¹², 1×10¹¹ to 1×10¹³, or from 1×10⁷ to 5×10⁸ magnetic particles) for assaying a single liquid sample.

### Kits

The description provides kits configured for T2MR-based detection and identification of pathogens (e.g., genus and/or species) and AST. For example, a kit may include one or more populations of magnetic particles and one or more antimicrobial agents. The kit may include any suitable number of populations of magnetic particles (e.g., 1, 2, 3, 4, 5, 6, 7, 8, 9, 10, 11, 12, 13, 14, 15, 16, 17, 18, or more, populations). The kit may include any suitable number of antimicrobial agents (e.g., 1, 2, 3, 4, 5, 6, 7, 8, 9, 10, 11, 12, 13, 14, 15, 16, 17, 18, 19, 20, or more antimicrobial agents). The magnetic particles may be a liquid suspension or dried magnetic particles which are reconstituted prior to use. The magnetic particles may be conjugated to a binding moiety configured for detection of a pathogen-associated analyte. In some examples, the binding moiety is a nucleic acid probe. In other examples, the binding moiety may be an antibody. The kit may further include a device, for example, as described herein (e.g., a T2Dx® device).

The kit may include one or more populations of magnetic particles conjugated to a nucleic acid probe as described herein, for example, a nucleic acid probe selected from any one of SEQ ID NOs: 15-42, 54-68, or 70-76.

### Panels

The methods of the invention can be configured to detect a predetermined panel of pathogens. The panel may be a bacterial pathogen panel configured to individually detect between 1 and 18 (e.g., 1, 2, 3, 4, 5, 6, 7, 8, 9, 10, 11, 12, 13, 14, 15, 16, 17, or 18) pathogens selected from the following: Gram-positive bacteria (e.g., Gram-positive anaerobic bacteria), Gram-negative bacteria (e.g., Gram-negative anaerobic bacteria), *Enterobacteriaceae* spp., *Acinetobacter* spp. (including *Acinetobacter baumannii*)*, Enterococcus* spp. (including *Enterococcus faecium* and *Enterococcus faecalis), Klebsiella* spp. (including *Klebsiella pneumoniae), Pseudomonas* spp. (including *Pseudomonas aeruginosa), Staphylococcus* spp. (including coagulase-positive species (e.g., *Staphylococcus aureus)* and coagulase-negative (CoNS) species), *Streptococcus* spp. (including β-hemotytic streptococci, *Streptococcus mitis, Streptococcus pneumoniae, Streptococcus agalactiae,* and *Streptococcus pyogenes), Escherichia* spp. (including *Escherichia coli),* a *Stenotrophomonas* spp. (including *Stenotrophomonas maltophilia), Proteus* spp. (including *Proteus mirabilis* and *Proteus vulgaris), Serratia* spp. (including *Serratia marcescens), Citrobacter* spp. (including *Citrobacter freundii*)*, Enterobacter* spp. (including *Enterobacter aerogenes* and *Enterobacter cloacae), Borrelia* spp. (e.g., *Borrelia burgdorferi, Borrelia afzelii,* and *Borrelia garinii*)*, Rickettsia* spp. (e.g., *Rickettsia rickettsii*)*, Anaplasma* spp. (e.g., *Anaplasma phagocytophilum), Coxiella* spp. (e.g., *Coxiella burnetii*)*, Ehrlichia* spp. (e.g., *Ehrlichia chaffeensis* and *Ehrlichia ewingii*)*, Franciscella* spp. (e.g., *Francisella tularensis), Clostridium* spp. (e.g., *Clostridium botulinum, Clostridium difficile, Clostridium perfringens,* and *Clostridium tetani*)*, Bacteroides* spp. (e.g., *Bacteroides fragilis),* and *Candida* spp., (including *Candida albicans, Candida guilliermondii, Candida glabrata, Candida krusei, Candida lusitaniae, Candida parapsilosis,* and *Candida tropicalis).*

A pathogen panel may be configured to detect *Candida* spp., including one or more (e.g., 1, 2, 3, 4, 5, 6, or 7) of the following: *Candida albicans, Candida guilliermondii, Candida glabrata, Candida krusei, Candida lusitaniae, Candida parapsilosis,* and *Candida tropicalis).* In cases where multiple species of a genus are detected, the species may be detected using individual target nucleic acids or using target nucleic acids that are universal to all of the species, for example, target nucleic acids amplified using universal primers. The panel be detected using the exemplary primers and probes described herein.

The panel may be configured to detect one or more genus of pathogens, one or more species of pathogens, or a combination thereof.

The panel may be configured to individually detect one or more (e.g., 1, 2, 3, 4, 5, 6, or 7) of *Acinetobacter baumannii,* Enterococcus faecium, Enterococcus faecalis, *Klebsiella pneumoniae, Pseudomonas aeruginosa, Staphylococcus aureus,* and a *Candida* spp. For example, the panel may be configured to individually detect *Acinetobacter baumannii, Enterococcus faecium, Enterococcus faecalis, Klebsiella pneumoniae, Pseudomonas aeruginosa, Staphylococcus aureus,* and a *Candida* spp. The panel can be detected using the exemplary primers and probes described herein.

The panel may be configured to detect a pan-bacterial marker. In any of the above panels, the analyte may be a nucleic acid (e.g., an amplified target nucleic acid, as described above), or a polypeptide (e.g., a polypeptide derived from the pathogen or a pathogen-specific antibody produced by a host subject, for example, an IgM or IgG antibody). Multiple analytes (e.g., multiple amplicons) may be used to detect a pathogen.

### Examples

The following examples are put forth so as to provide those of ordinary skill in the art with a complete disclosure and description of how the methods, devices, systems, and compositions described herein are performed, made, and evaluated, and are intended to be purely exemplary of the invention and are not intended to limit the scope of what the inventors regard as their invention.

### Example 1: Targeted Therapy Following Pathogen Identification by T2MR Detection

Rapid determination of the identity of a pathogen in a biological sample can allow immediate targeted therapy. In this example, pathogen detection and identification is performed using a T2Dx® instrument (T2 Biosystems, Lexington, MA). A sample obtained from a subject (e.g., a 1.7-2 mL whole blood sample from a human suspected of having a BSI such as Candidemia) is inserted into the device, for example, as described in WO 2012/054639. The method described in Example 22 of WO 2012/054639 can be used for detection and identification of *Candida* species. Briefly, a sample obtained from the patient is inserted into the device. If the sample is a whole blood sample, the next step typically includes blood cell lysis and concentration, for instance, by (a) by mixing the whole blood sample with an erythrocyte lysis agent solution to produce disrupted red blood cells, (b) centrifuging the sample to form a supernatant and a pellet, discarding some or all of the supernatant, and optionally washing the pellet (for example, by resuspending the pellet in volume of a buffer, and centrifuging the pellet), and (c) lysing cells in the pellet (which may include white blood cells and/or pathogen cells) to form a lysate. Next, one or more target nucleic acid(s) is amplified in the lysate, for instance, using PCR. For detection of *Candida* species, a forward primer that includes the oligonucleotide sequence 5'-GGC ATG CCT GTT TGA GCG TC-3' (SEQ ID NO: 13) and a reverse primer that includes the oligonucleotide sequence 5'-GCT TAT TGA TAT GCT TAA GTT CAG CGG GT-3' (SEQ ID NO: 14) can be used for amplification. Typically, the lysate volume used for amplification is approximately 50 µL. Typically, the target nucleic acid(s) are amplified without performing any wash steps or other purification steps, such that amplification occurs in the presence of whole blood proteins and non-target nucleic acids (e.g., subject DNA from white blood cells). Next, each target nucleic acid is hybridized with magnetic particles coated with specific probes that specifically hybridize to the target nucleic acid, leading to changes in the specific aggregation of the magnetic particles in the presence of the amplified target nucleic acid. The capture probes listed in Table 1 may be used for detection and identification of *Candida* spp. Finally, the device measures the T₂ relaxation response in the sample to determine the presence or absence of the pathogen.

Upon identification of the pathogen, an appropriate therapy for the pathogen is administered to the subject immediately without a requirement for culturing the pathogen for AST testing (Figure 1B). Coupling T2MR-based species detection and identification can therefore, in some cases, lead to targeted therapy within 3-5 hours from the time that the sample is obtained from the patient.

In one specific example, a method as described above is used to detect the presence of an *Aspergillus* spp., for instance, by amplifying a target nucleic acid with a forward primer and a reverse primer that are specific for the genus of *Aspergillus* and detecting the presence or absence of the nucleic acid based on the T₂ relaxation response in the sample. If an *Aspergillus* spp. pathogen is present in a sample (e.g., blood) obtained from the subject, voriconazole is administered to the subject immediately without a requirement for culturing the *Aspergillus* spp. for AST testing.

In another specific example, a method as described above is used to detect the presence of methicillin-resistant S. *aureus.* First, one or more target nucleic acids characteristic of methicillin-resistant S. *aureus,* such as a nucleic acid that includes part or all of the *mec*A gene and/or a nucleic acid that is specific for S. *aureus,* is amplified. The presence or absence of the one or more nucleic acids is determined as described above based on the T₂ relaxation response for each sample. If methicillin-resistant S. *aureus* is present in the sample, vancomycin is administered to the subject immediately without a requirement for culturing the methicillin-resistant S. *aureus* for AST testing.

In yet another specific example, a method as described above is used to detect the presence of the species *Cryptococcus neoformans,* for instance, by amplifying a target nucleic acid with a forward primer and a reverse primer that are specific for the species *Cryptococcus neoformans* and detecting the presence or absence of the nucleic acid based on the T₂ relaxation response in the sample. If an Aspergillus spp. pathogen is present in a sample (e.g., blood) obtained from the subject, amphotericin B and 5-fluorocytosine are administered to the subject immediately without a requirement for culturing the *Cryptococcus neoformans* for AST testing.

In a still further example, a method as described above is used to detect the presence of the species *Enterococcus faecium,* for instance, by amplifying a target nucleic acid with a forward primer and a reverse primer that are specific for the species *Enterococcus faecium* and detecting the presence or absence of the nucleic acid based on the T₂ relaxation response in the sample. If *Enterococcus faecium* is present in a sample (e.g., blood) obtained from the subject, daptomycin and/or linizolid are administered to the subject immediately without a requirement for culturing the *Enterococcus faecium* for AST testing.

### Example 2: Rapid AST Determination following T2MR and Pathogen Subculture

In some instances, T2MR-based methods for pathogen detection and identification, as described herein, allow for rapid AST results by allowing for subculture of a pathogen into more favorable media, allowing faster growth to yield sufficient biomass for AST testing (see, e.g., Figures 1C and 2).

A blood sample is obtained from a subject suspected to be suffering from Candidemia. A portion of the blood sample is used to inoculate a blood culture bottle (e.g., a BD BACTEC™ aerobic blood culture bottle). The blood culture bottles are incubated as recommended by the manufacturer. In parallel, an aliquot of the blood sample obtained from the patient is subjected to a T2MR-based detection method, for example, as described in Example 1 above. In this example, the T2MR-based method identifies *Candida albicans* as being present in the blood sample.

Next, based on the identification of *Candida albicans* in the blood sample, the blood culture bottle is sampled by lysis filtration or lysis centrifugation followed by plating of the cell pellet on to Samouraud Glucose media, thereby encouraging growth of *Candida albicans* and early growth of the isolated colonies. These colonies are subjected to AST testing, either using an agar-based AST method (e.g., ETEST®) or a full panel AST device (e.g., VITEK® 2). Several appropriate anti-fungal drugs are tested, e.g., fluconazole, itraconazole, amphotericin B, micafungin, voriconazole, and caspofungin. In this example, the AST testing reveals that the *Candida albicans* isolate present in the subject's blood sample is particularly sensitive to fluconazole. Based on this result, an appropriate dose of fluconazole is administered to the subject, thereby treating the subject's Candidemia.

### Example 3: Rapid AST Determination Following T2MR and Expression Analysis

T2MR-based methods for pathogen detection and identification, as described herein, allow for rapid AST results in conjunction with expression analysis to determine whether the pathogen expresses one or more genes characteristic of the pathogen, such as antimicrobial resistance genes and/or virulence factors (see, e.g., Figure 1D).

A blood sample is obtained from a subject suspected to be suffering from bacteremia. A portion of the blood sample is used to inoculate a blood culture bottle (e.g., a BD BACTEC™ aerobic blood culture bottle). The blood culture bottles are incubated as recommended by the manufacturer. In parallel, an aliquot of the blood sample obtained from the patient is subjected to a T2MR-based detection method, for example, the method described in Example 22 of WO 2012/054639 in conjunction with a T2Dx® device. In this example, the T2MR-based method identifies S. *aureus* as being present in the blood sample within 3 hours from the start of the assay.

Next, based on the identification of S. *aureus* in the blood sample, the blood culture bottle is sampled by lysis filtration or lysis centrifugation followed by plating of the cell pellet on to chromogenic media (e.g., CHROMAGAR™ MRSA (Chromagar)) or directly on to an agar-based antimicrobial gradient test (e.g., an ETEST®) in order to test whether the S. *aureus* isolate from the subject's blood is sensitive to oxacillin, vancomycin, trimethoprim-sulfamethoxazole (BACTRIM™), doxycycline, daptomycin, and linezolid. Additionally, T2MR-based real-time PCR is performed as described, for example, in Example 19 of WO 2012/054639, is used to determine the expression level of antibiotic resistance markers such as *mec*A*.* Note that the T2MR-based real-time PCR could also have been performed during the time of the initial T2MR-based detection and identification of S. *aureus.*

In this example, the real-time PCR result indicates that the S. *aureus* expresses the *mec*A gene, which indicates that the S. *aureus* isolate is likely to be methicillin-resistant. The results of the growth of the S. *aureus* isolate on the chromogenic media also indicate that the S. *aureus* isolate is resistant to methicillin, based on the color of the colonies that form on the plate. The agar-based antimicrobial gradient test indicates that the S. *aureus* isolate is sensitive to trimethoprim-sulfamethoxazole. Based on these results, an effective amount of trimethoprim-sulfamethoxazole is administered to the subject, thereby treating the bacteremia.

### Example 4: T2MR-based Detection and Identification of Pathogens followed by T2MR-based AST

In some instances, T2MR-based approaches are used to perform AST without requiring the use of conventional AST approaches. Figure 1E shows a schematic example in which T2MR-based detection and identification of the pathogen followed by additional blood draws taken from the patient that are subjected to T2MR-based AST. However, it is also contemplated that T2MR-based AST could also be used in place of, or in addition to, any of the AST methods described in the preceding examples.

A blood sample is obtained from a subject suspected to be suffering from Candidemia. The blood sample obtained from the patient is subjected to a T2MR-based detection method, for example, the method described in Example 22 of WO 2012/054639 in conjunction with a T2Dx® device. Briefly, PCR is performed using a forward primer that includes the oligonucleotide sequence 5'-GGC ATG CCT GTT TGA GCG TC-3' (SEQ ID NO: 13) and a reverse primer that includes the oligonucleotide sequence 5'-GCT TAT TGA TAT GCT TAA GTT CAG CGG GT-3' (SEQ ID NO: 14). Magnetic particles conjugated individually to the capture probes described in Table 1 are used to test for the presence of *Candida albicans, Candida glabrata, Candida krusei, Candida parapsilosis,* and *Candida tropicalis.* In this example, *Candida glabrata* is identified as being present in the subject's blood sample within 3 h from the start of the assay.

Based on the T2MR-based detection and identification of *Candida glabrata* in the subject's blood, a subsequent blood draw is taken directly to media that contain either no antimicrobial agents or pre-determined levels of fluconazole, itraconazole, amphotericin B, or caspofungin. Aliquots are taken from these media at time intervals, and the aliquots are subjected to T2MR-based real-time PCR is performed as described, for example, in Example 19 of WO 2012/054639, to detect the expression level of a housekeeping gene such as beta-actin (ACT1) as a proxy for growth of the C. *glabrata* isolate in the media. A growth curve is made by plotting the T₂ signal over time. T2MR may allow for earlier detection of growth, thereby shortening the front end and back end of the AST process. This approach reveals that the C. *glabrata* isolate is particularly sensitive to fluconazole. An appropriate dose of fluconazole is administered to the subject, thereby treating the subject's Candidemia.

Alternatively, or in addition, an additional blood draw is taken directly to a lysis centrifugation system (e.g., ISOLATOR® lysis centrifugation system), and a pellet of the C. *glabrata* isolate is subsequently used to inoculate a full panel AST device (e.g., VITEK® 2) in order to perform AST.

### Example 5: Species identification and expression analysis of key transcripts by T2MR

In some instances, T2MR-based approaches are used for species detection and expression analysis of key transcripts, including inducible antimicrobial resistance genes, housekeeping genes (e.g., energy metabolism genes) (Figure 1F).

A blood sample is obtained from a subject suspected to be suffering from Candidemia. The blood sample obtained from the patient is subjected to a T2MR-based detection method, for example, as described above in Example 4. In this example, *Candida albicans* is identified as being present in the subject's blood sample within 3 h from the start of the assay.

Based on the T2MR-based detection and identification of *Candida albicans* in the subject's blood, a subsequent blood draw is taken directly to media that contain either no antimicrobial agents or pre-determined levels of antimicrobial agents such as fluconazole, itraconazole, amphotericin B, and caspofungin. Aliquots are taken from these media at time intervals, and the aliquots are subjected to T2MR-based real-time PCR is performed as described, for example, in Example 19 of WO 2012/054639, to detect the expression level of the multiple drug resistance gene MDR1 as well as a housekeeping gene such as beta-actin (ACT1) as a proxy for growth of the C. *albicans* isolate in the media. This analysis indicates that the expression of MDR1 is relatively low in the C. *albicans* isolate in the subject's blood. A growth curve is made by plotting the T2 signal over time. This approach reveals that the C. *albicans* isolate is particularly sensitive to fluconazole. An appropriate dose of fluconazole is administered to the subject, thereby treating the subject's Candidemia.

Following the initial administration of fluconazole, subsequent blood draws are obtained from the patient and subjected to T2MR-based real-time PCR to determine the expression level of MDR1 in the C. *albicans* isolate present in the subject's blood. This analysis reveals that the expression level of MDR1 in the subsequent blood draw is elevated compared to the initial pre-treatment blood draw. Based on this result, the therapy is switched from fluconazole to a different agent, e.g., amphotericin B, which is successful in treating the subject.

### Example 6: T2MR-based Detection and Identification of Pathogens followed by T2MR-based AST Involving Direct Cell Detection

In some instances, T2MR-based approaches are used to perform AST (see, e.g., Figure 1E, which shows a schematic example in which T2MR-based detection and identification of the pathogen is followed by additional blood draws taken from the patient that are subjected to T2MR-based AST). In some instances, T2MR-based AST involves direct cell detection using magnetic particles that bind to the cell surface of pathogens.

In one such example, a blood sample is obtained from a subject suspected to be suffering from bacteremia. A portion of the blood sample is used to inoculate a blood culture bottle (e.g., a BD BACTEC™ aerobic blood culture bottle). The blood culture bottles are incubated as recommended by the manufacturer. In parallel, an aliquot of the blood sample obtained from the patient is subjected to a T2MR-based detection method, for example, the method described in Example 22 of WO 2012/054639 in conjunction with a T2Dx® device. In this example, the T2MR-based method identifies S. *aureus* as being present in the blood sample within 3 hours from the start of the assay.

Based on the T2MR-based detection and identification of S. *aureus* in the subject's blood, a subsequent blood draw is taken directly to media that contain either no antimicrobial agents or pre-determined levels of oxacillin, vancomycin, trimethoprim-sulfamethoxazole (BACTRIM™), doxycycline, daptomycin, or linezolid. Aliquots are taken from these media at time intervals, and the aliquots are subjected to T2MR-based direct detection of S. *aureus* cells, as described, for example, in Example 4 of International Patent Publication No. WO 2012/129281. S. *aureus* cells are detected by labeling with magnetic nanoparticles. Briefly, magnetic nanoparticles are conjugated with vancomycin, an antibiotic that recognizes D-alanyl-D-alanine moieties in the bacterial cell wall. These magnetic nanoparticles are added to the aliquots of media containing either no anti-microbial agents or pre-determined levels of oxacillin, vancomycin, (trimethoprim-sulfamethoxazole (BACTRIM™), doxycycline, daptomycin, or linezolid. A growth curve is made by plotting the T₂ signal over time. T2MR may allow for earlier detection of growth, thereby shortening the front end and back end of the AST process. This approach reveals that the S. *aureus* isolate is particularly sensitive to vancomycin. An appropriate dose of vancomycin is administered to the subject, thereby treating the subject's bacteremia.

### Sequence Listing

The following sequences are used throughout the application. "/i6diPr/" indicates 2,6-Diaminopurine, "Nitlnd" indicates 5' 5-Nitroindole, "/5AmMC12/" indicates 5' amino modifier C12, and "/3AmMO/" indicates 3' amino modifier.

| **Sequence** | **SEQ ID NO:** |
|---|---|
| 5'-CGT TTT CCA AAT CTG TAA CAG ACT GGG-3' | 1 |
| 5'-AGG ACG TTG ATA GG TTG GAT GTG GA-3' | 2 |
| 5'-GGT AGC TAT GTA GGG AAG GGA TAA ACG CTG A-3' | 3 |
| 5'-GCG CTA AGG AGC TTA ACT TCT GTG TTC G-3' | 4 |
| 5'-GAC GGT TGT CCC GGT TTA AGC A-3' | 5 |
| 5'-GCT GGT ATC TTC GAC TGG TCT-3' | 6 |
| 5'-AGG CTG GGT GTG TAA GCG TTG T-3' | 7 |
| 5'-CAA GCA ATT CGG TTG GAT ATC CGT T-3' | 8 |
| 5'-GGT AAT GAATTA CCT /i6diPr/TC TCT GCT GGTTTC TTC TT-3' | 9 |
| 5'-ACC AGC ATC TTC /i6diPr/GC ATC TTC TGT AAA-3' | 10 |
| 5'-GAA GTT ATG TTT /i6diPr/CT ATT CGA ATC GTG GTC CAGT-3' | 11 |
| 5'-GTT GTA AAG CCA TGA TGC TCG TAA CCA-3' | 12 |
| 5'-GGC ATG CCT GTT TGA GCG TC-3' | 13 |
| 5'-GCT TAT TGA TAT GCT TAA GTT CAG CGG GT-3' | 14 |
| 5'-TGA GGC TTG ACT ATA CAA CAC C-3' | 15 |
| 5'- CTA AAA TGA ACA GAT AAA GTA AGA TTC AA-3' | 16 |
| /5AmMC12/TTT TTT TTT TGA GGC TTG ACT ATA CAA CAC C | 17 |
| CTA AAA TGA ACA GAT AAA GTA AGA TTC AAT TTT TTT TT/3AmMO/ | 18 |
| 5'-AAA ACT TAT ATG ACT TCA AAT CCA GTT TT-3' | 19 |
| 5'-TTT ACT CAA TAA AAG ATA ACA CCA CAG-3' | 20 |
| /5AmMC12/ttt ttt ttt AAA ACT TAT ATG ACT TCA AAT CCA GTT TT | 21 |
| TTT ACT CAA TAA AAG ATA ACA CCA CAG Ttt ttt ttt t/3AmMO/ | 22 |
| 5'-TGG ATA AGT AAA AGC AAC TTG GTT-3' | 23 |
| 5'-AAT GAA GAT TCA ACT CAA TAA GAA ACA ACA-3' | 24 |
| /5AmMC12/ttt ttt ttt TGG ATA AGT AAA AGC AAC TTG GTT | 25 |
| AAT GAA GAT TCA ACT CAA TAA GAA ACA ACA ttt ttt ttt/3AmMO/ | 26 |
| 5'-TAC CAA GGC GCT TGA GAG AAC TC-3' | 27 |
| 5'-CTG GTG TGT AGG TGA AGT C-3' | 28 |
| /5AmMC12/TTT TTT TTT TAC CAA GGC GCT TGA GAG AAC TC | 29 |
| CTG GTG TGT AGG TGA AGT CTT TTT TTT T/3AmMO/ | 30 |
| 5'-GTG TGT TGT AGG GTG AAG TCG AC-3' | 31 |
| 5'-CAC CTT GAA ATC ACA TAC CTG A-3' | 32 |
| /5AmMC12/ttt ttt ttt GTG TGT TGT AGG GTG AAG TCG AC | 33 |
| CAC CTT GAA ATC ACA TAC CTG Att ttt ttt t/3AmMO/ | 34 |
| 5'-CCA TTT GAA GTT GTT TAT TAT GC-3' | 35 |
| 5'-GGG AAA TGA TTA ATT ATG CAT TAA ATC-3' | 36 |
| /5AmMC12/TTT TTT TTT CCA TTT GAA GTT GTT TAT TAT GC | 37 |
| GGG AAA TGA TTA ATT ATG CAT TAA ATC TTT TTT TTT /3AmMO/ | 38 |
| 5'-TT TTT CAG ATT TAG GAT TAG TTG ATT-3' | 39 |
| 5'-GAT CCG TAT TGG TTA TAT CAT C-3' | 40 |
| /5AmMC12/TT TTT TTT TTT TTT CAG ATT TAG GAT TAG TTG ATT | 41 |
| GAT CCG TAT TGG TTA TAT CAT CTT TTT TTT T/3AmMO/ | 42 |
| 5'-AM-TTT TTT TTT TGG AAT AAT ACG CCG ACC AGC-3' | 43 |
| 5'-AAG GAT CTA TTT CAG TAT GAT GCA GTT TTT TTT T-AM-3' | 44 |
| | 45 |
| | 46 |
| | 47 |
| | 48 |
| | 49 |
| | 50 |
| | 51 |
| 5'-GCA TTA ATC GAC GGT ATG GTT GAC C-3' | 52 |
| 5'-CCT GCT GAA ACA GGT TTT CCC ACA TA-3' | 53 |
| 5'-AGT GAT GAT GAG TTG TTT GCC AGT G-3' | 54 |
| 5'-TGA ATT GTC GCC GCG TGA CCA G-3' | 55 |
| ACC CAG CGG TTT GAG GGA GAA AC | 56 |
| AAA GTT TGA AGA TAT ACG TGG TGG ACG TTA | 57 |
| CGC ACG CGC AAG ATG GAA ACG | 58 |
| AAG TTC AGC GGG TAT TCC TAC CT | 59 |
| AGC TTT TTG TTG TCT CGC AAC ACT CGC | 60 |
| CTA CCA AAC ACA ATG TGT TTG AGA AG | 61 |
| CCT GAT TTG AGG TCA AAC TTA AAG ACG TCT G | 62 |
| AGT CCT ACC TGA TTT GAG GTC Nitlnd AA | 63 |
| CCG Nitlnd GG GTT TGA GGG AGA AAT | 64 |
| AAA GTT ATG AAATAA ATT GTG GTG GCC ACT AGC | 65 |
| ACC CGG GGGTTT GAG GGA GAA A | 66 |
| AGT CCT ACC TGA TTT GAG GTC GAA | 67 |
| CCG AGG GTT TGA GGG AGA AAT | 68 |
| 5'-GGA AGG GAT CAG GTG GTT CAC TCT T-3' | 69 |
| 5'-AAA ACT TAT GTG ACT TCA AAT CCA GTT TT-3' | 70 |
| 5'-TTT ACT CAA TAA AAG ATA ACA CCA CAG T-3' | 71 |
| /5AmMC12/ttt ttt ttt AAA ACT TAT GTG ACT TCA AAT CCA GTT TT | 72 |
| 5'-TCT GAC GAT TGT GTG TTG TAA GG-3' | 73 |
| 5'-GGA TAG ACG TAA GCC CAA GC-3' | 74 |
| /5AmMC 12/ttt ttt ttt TCT GAC GAT TGT GTG TTG TAA GG | 75 |
| GGA TAG ACG TAA GCC CAA GCtt ttt ttt t/3AmMO/ | 76 |
| | 77 |
| | |

### Other Embodiments

While the invention has been described in connection with specific embodiments thereof, it will be understood that it is capable of further modifications and this application is intended to cover any variations, uses, or adaptations of the invention following, in general, the principles of the invention and including such departures from the present disclosure that come within known or customary practice within the art to which the invention pertains and may be applied to the essential features hereinbefore set forth, and follows in the scope of the claims.

## Claims

1. A method of performing rapid antimicrobial susceptibility testing for a pathogen in a biological sample obtained from a subject, the method comprising:
(a) determining the presence and genus of a pathogen in a biological sample by
(i) preparing an assay sample by contacting a first portion of the biological sample with magnetic particles, wherein the magnetic particles have binding moieties on their surfaces, the binding moieties operative to alter the specific aggregation of the magnetic particles in the presence of an analyte associated with the pathogen;
(ii) placing the assay sample in a device, the device comprising a support defining a well for holding the assay sample, and having an RF coil configured to detect a signal produced by exposing the assay sample to a bias magnetic field created using one or more magnets and an RF pulse sequence;
(iii) exposing the assay sample to the bias magnetic field and the RF pulse sequence;
(iv) following step (iii), measuring the signal produced by the assay sample; and
(v) based on the results of step (iv), determining the presence and genus of the pathogen in the biological sample;
(b) in parallel to step (a), incubating a second portion of the biological sample under conditions suitable for growth of the pathogen to form a pathogen culture; and
(c) comparing the growth rate of a first aliquot of the pathogen culture in the presence of the antimicrobial agent to the growth rate of a second aliquot of the pathogen culture in the absence of the antimicrobial agent, thereby determining whether the pathogen is susceptible to the antimicrobial agent.

2. The method of claim 1, wherein
(i) step (b) further comprises inoculating a growth medium with an aliquot of the biological sample to form a subculture and incubating the subculture under conditions suitable for enhanced growth of the pathogen, wherein the growth medium is selected based on the results of step (a), optionally wherein step (c) comprises comparing the growth rate of a first aliquot of the subculture in the presence of the antimicrobial agent to the growth rate of a second aliquot of the subculture in the absence of the antimicrobial agent;
(ii) the method further comprises contacting the pathogen culture with an additive prior to step (c), wherein the additive is selected based on the results of step (a), thereby enhancing growth of the culture; and/or
(iii) the method further comprises a step of performing centrifugation, filtration, or lysis centrifugation of the pathogen culture or an additional portion of the biological sample prior to step (c), thereby producing a pellet comprising the pathogen, optionally wherein the method further comprises plating the pellet or a portion thereof on one or more media plates selected based on the genus of the pathogen, and incubating the one or more media plates under conditions suitable for growth of the pathogen, particularly wherein the one or more media plates are used in step (c) to compare the growth rates of the pathogen in the first and second aliquots of the pathogen culture.

3. A method of performing rapid antimicrobial susceptibility testing for a pathogen in a biological sample obtained from a subject, the method comprising:
(a) determining the presence and genus of a pathogen in a biological sample by
(i) preparing an assay sample by contacting a first portion of the biological sample with magnetic particles, wherein the magnetic particles have binding moieties on their surfaces, the binding moieties operative to alter the specific aggregation of the magnetic particles in the present of an analyte associated with the pathogen;
(ii) placing the assay sample in a device, the device comprising a support defining a well for holding the assay sample, and having an RF coil configured to detect a signal produced by exposing the assay sample to a bias magnetic field created using one or more magnets and an RF pulse sequence;
(iii) exposing the assay sample to the bias magnetic field and the RF pulse sequence;
(iv) following step (iii), measuring the signal produced by the assay sample; and
(v) based on the results of step (iv), determining the presence and genus of the pathogen in the biological sample;
(b) comparing the growth rate of the pathogen in a first aliquot of an additional biological sample in the presence of the antimicrobial agent to the growth rate of the pathogen in a second aliquot of the additional biological sample in the absence of the antimicrobial agent, thereby determining whether the pathogen is susceptible to the antimicrobial agent, wherein the additional biological sample has been obtained from the patient following step (a).

4. The method of claim 3, wherein
(i) step (b) further comprises inoculating a growth medium with an aliquot of the additional biological sample to form a subculture and incubating the subculture under conditions suitable for enhanced growth of the pathogen, wherein the growth medium is selected based on the results of step (a), optionally wherein step (c) comprises comparing the growth rate of a first aliquot of the subculture in the presence of the antimicrobial agent to the growth rate of a second aliquot of the subculture in the absence of the antimicrobial agent; or
(ii) the method further comprises a step of performing centrifugation, filtration, or lysis centrifugation of the additional biological sample prior to step (c), thereby producing a pellet comprising the pathogen, optionally wherein the method further comprises plating the pellet or a portion thereof on one or more media plates selected based on the genus of the pathogen, and incubating the one or more media plates under conditions suitable for growth of the pathogen, particularly wherein the one or more media plates are used in step (c) to compare the growth rates of the pathogen in the first and second aliquots of the additional biological sample.

5. The method of any one of claims 1-4, wherein
(i) growth rates are determined using a broth dilution test, a disk diffusion test, an antimicrobial gradient test, chromogenic media, an enzyme activity assay, and/or an automated instrument, optionally wherein the antimicrobial gradient test is an epsilometer test;
(ii) the antimicrobial agent of step (c) is selected based on the results of step (a);
(iii) a plurality of antimicrobial agents are tested in step (c) to determine an antimicrobial susceptibility profile of the pathogen; optionally wherein at least 4 or at least 10 antimicrobial agents are tested in step (c);
(iv) amplifying is performed by asymmetric polymerase chain reaction (PCR); and/or
(v) step (a) comprises assaying for the presence of a panel of pathogens.

6. The method of any one of claims 1-5, wherein step (a) or (c) further comprises quantifying the expression level of a target nucleic acid characteristic of the pathogen, optionally wherein step (a) or (c) comprises amplifying the target nucleic acid characteristic of the pathogen in an reaction mixture in a detection tube resulting in the production of an amplicon corresponding to the target nucleic acid characteristic of the pathogen,
wherein the method is performed in the device recited in step (a), said reaction mixture comprising a portion of the biological sample comprising the target nucleic acid characteristic of the pathogen, primers specific for said target nucleic acid, and superparamagnetic particles, said superparagmagnetic particles operable to aggregate or disaggregate in the presence of the amplicon; and
said amplification comprising the following steps:
(i) performing one or more cycles of amplification;
(ii) exposing said reaction mixture to conditions permitting the aggregation or disaggregation of said superparamagnetic particles;
(iii) exposing the sample to a bias magnetic field and an RF pulse sequence;
(iv) following step (iii), measuring the signal from the detection tube;
(v) repeating steps (i)-(iv) until a desired amount of amplification is obtained; and
(vi) on the basis of the result of step (iv), quantifying the amplicons present at the corresponding cycle of amplification;
wherein the initial quantity of target nucleic acid characteristic of the antimicrobial resistance gene in said sample is determined based on the quantity of amplicons determined at each cycle of said amplification.

7. The method of any one of claims 1-6, wherein
(i) step (a) further comprises determining the titer of the pathogen;
(ii) the method is capable of detecting 10 CFU, 3CFU or 1 CFU of the pathogen per milliliter of the biological sample;
(iii) the method further comprises selecting a therapy comprising an antimicrobial agent for the subject based on the results of step (c), optionally wherein the method further comprises determining the presence of the pathogen in the subsequent biological sample in a subsequent biological sample obtained from the subject following administration of the antimicrobial agent, particularly further comprising and/or particularly further comprising quantifying the expression level of a target nucleic acid characteristic of the pathogen in the subsequent biological sample;
(iv) the genus is a taxonomic family or a taxonomic genus; and/or
(v) the species of the pathogen is not determined by step (a) or step (a) further comprises determining the species of the pathogen, optionally the antimicrobial agent of step (c) is selected based on the species of the pathogen, further optionally the species is a taxonomic species.

8. The method of any one of claims 1-7, wherein
(i) the analyte is an amplicon characteristic of the pathogen generated by amplifying a corresponding target nucleic acid in the presence of a forward and a reverse primer; and/or
(ii) the magnetic particles comprise a first population of magnetic particles conjugated to a first probe, and a second population of magnetic particles conjugated to a second probe, wherein the magnetic particles form aggregates in the presence of the amplicon characteristic of the pathogen.

9. The method of any one of claims 1-8, wherein
(i) substep (i) comprises adding to the liquid sample from 1×10⁶ to 1×10¹³ magnetic particles per milliliter of the liquid sample;
(ii) the magnetic particles have a mean diameter of from 700 nm to 950 nm;
(iii) the magnetic particles have a T₂ relaxivity per particle of from 1×10⁹ to 1×10¹² mM⁻¹s⁻¹; and/or
(iv) a plurality of assay samples are prepared in substep (i) by independently contacting each member of the plurality of assay samples with a population of magnetic particles configured to form aggregates in the presence of an analyte associated with a member of the panel of pathogens, and wherein each member of the plurality of assay samples is subjected to substeps (ii) through (iv) of the method.

10. The method of any one of claims 1-9, wherein the steps of the method are completed within 2 days, within 12 hours or within 7 hours.

11. The method of any one of claims 1-10, wherein the biological sample is selected from the group consisting of whole blood, cerebrospinal fluid (CSF), pleural fluid, urine, or synovial fluid.

12. The method of any one of claims 1-11, wherein the pathogen is a fungal pathogen, a bacterial pathogen, a protozoan pathogen, or a viral pathogen.

13. The method of claim 12, wherein:
(i) the fungal pathogen is a *Candida* spp, optionally wherein the *Candida* spp. is selected from the group consisting of *Candida albicans, Candida guilliermondii, Candida glabrata, Candida krusei, Candida lusitaniae, Candida parapsilosis,* and *Candida tropicalis;*
(ii) the bacterial pathogen is selected from the group consisting of *Escherichia coli,* Acinetobacter baumannii, *Enterococcus faecalis, Enterococcus faecium, Klebsiella pneumoniae, Pseudomonas aeruginosa, Staphylococcus aureus,* Borrelia burgdorferi, *Borrelia afzelii, Borrelia garinii, Rickettsia rickettsii, Anaplasma phagocytophilum, Coxiella burnetii, Ehrlichia chaffeensis, Ehrlichia ewingii, Francisella tularensis, Streptococcus pneumoniae,* and *Neisseria meningitides;* or
(iii) the protozoan pathogen is *Babesia microti* or *Babesia divergens.*

14. The method of claim 12, whereinthe pathogen is associated with bloodstream infection, sepsis, septic arthritis, pneumonia, peritonitis, osteomyelitis, meningitis, urinary tract infection or Lyme disease.

15. The method of claim 14, wherein the bloodstream infection is fungemia, bacteremia, or viremia, particularly wherein the fungemia is Candidemia.

## Patentansprüche

1. Verfahren zum Durchführen des schnellen Testens der antimikrobiellen Empfindlichkeit für ein Pathogen in einer von einem Subjekt erhaltenen biologischen Probe, wobei das Verfahren umfasst:
(a) Bestimmen des Auftretens und der Gattung eines Pathogens in einer biologischen Probe durch
(i) Herstellen einer Testprobe durch Inkontaktbringen eines ersten Teils der biologischen Probe mit magnetischen Partikeln, wobei die magnetischen Partikel Bindungsgruppen auf ihren Oberflächen aufweisen, wobei die Bindungsgruppen funktionsfähig sind, um die spezifische Aggregation der magnetischen Partikel in Gegenwart eines mit dem Pathogen assoziierten Analyten zu verändern;
(ii) Einbringen der Testprobe in eine Vorrichtung, wobei die Vorrichtung einen Träger umfasst, der eine Vertiefung zum Halten der Testprobe definiert und eine HF-Spule aufweist, die konfiguriert ist, um ein Signal zu detektieren, das erzeugt wird, indem die Testprobe einem Vormagnetisierungsfeld ausgesetzt wird, das durch Verwenden eines oder mehrerer Magnete und einer HF-Pulssequenz erzeugt wird;
(iii) Aussetzen der Testprobe dem Vormagnetisierungsmagnetfeld und der HF-Pulssequenz;
(iv) nach Schritt (iii), Messen des von der Testprobe erzeugten Signals; und
(v) basierend auf den Ergebnissen von Schritt (iv), Bestimmen des Auftretens und der Gattung des Pathogens in der biologischen Probe;
(b) parallel zu Schritt (a), Inkubieren eines zweiten Teils der biologischen Probe unter für das Wachstum des Pathogens geeigneten Bedingungen, um eine Pathogenkultur zu bilden; und
(c) Vergleichen der Wachstumsrate eines ersten Aliquots der Pathogenkultur in Gegenwart des antimikrobiellen Mittels mit der Wachstumsrate eines zweiten Aliquots der Pathogenkultur in Abwesenheit des antimikrobiellen Mittels, wodurch bestimmt wird, ob das Pathogen empfindlich gegenüber dem antimikrobiellen Mittel ist.

2. Verfahren gemäß Anspruch 1, wobei
(i) Schritt (b) ferner das Beimpfen eines Wachstumsmediums mit einem Aliquot der biologischen Probe, um eine Subkultur zu bilden, und das Inkubieren der Subkultur unter für ein verstärktes Wachstum des Pathogens geeigneten Bedingungen, umfasst, wobei das Wachstumsmedium basierend auf den Ergebnissen von Schritt (a) ausgewählt ist, wobei optional Schritt (c) das Vergleichen der Wachstumsrate eines ersten Aliquots der Subkultur in Gegenwart des antimikrobiellen Mittels mit der Wachstumsrate eines zweiten Aliquots der Subkultur in Abwesenheit des antimikrobiellen Mittels umfasst;
(ii) das Verfahren ferner das Inkontaktbringen der Pathogenkultur mit einem Additiv vor Schritt (c) umfasst, wobei das Additiv basierend auf den Ergebnissen von Schritt (a) ausgewählt ist, wodurch das Wachstum der Kultur verstärkt wird; und/oder
(iii) das Verfahren ferner einen Schritt des Durchführens einer Zentrifugation, Filtration oder Lysezentrifugation der Pathogenkultur oder eines zusätzlichen Teils der biologischen Probe vor Schritt (c) umfasst, wodurch ein Pellet umfassend das Pathogen hergestellt wird, wobei optional das Verfahren ferner das Ausplattieren des Pellets oder eines Teils davon auf eine oder mehrere Medienplatten umfasst, die basierend auf der Gattung des Pathogens ausgewählt sind, und Inkubieren der einen oder mehreren Medienplatten unter für das Wachstum des Pathogens geeigneten Bedingungen, insbesondere wobei die eine oder mehreren Medienplatten in Schritt (c) verwendet werden, um die Wachstumsraten des Pathogens in den ersten und zweiten Aliquots der Pathogenkultur zu vergleichen.

3. Verfahren zum Durchführen des schnellen Testens der antimikrobiellen Empfindlichkeit für ein Pathogen in einer von einem Subjekt erhaltenen biologischen Probe, wobei das Verfahren umfasst:
(a) Bestimmen des Auftretens und der Gattung eines Pathogens in einer biologischen Probe durch
(i) Herstellen einer Testprobe durch Inkontaktbringen eines ersten Teils der biologischen Probe mit magnetischen Partikeln, wobei die magnetischen Partikel Bindungsgruppen auf ihren Oberflächen aufweisen, wobei die Bindungsgruppen funktionsfähig sind, um die spezifische Aggregation der magnetischen Partikel in Gegenwart eines mit dem Pathogen assoziierten Analyten zu verändern;
(ii) Einbringen der Testprobe in eine Vorrichtung, wobei die Vorrichtung einen Träger umfasst, der eine Vertiefung zum Halten der Testprobe definiert und eine HF-Spule aufweist, die konfiguriert ist, um ein Signal zu detektieren, das erzeugt wird, indem die Testprobe einem Vormagnetisierungsfeld ausgesetzt wird, das durch Verwenden eines oder mehrerer Magnete und einer HF-Pulssequenz erzeugt wird;
(iii) Aussetzen der Testprobe dem Vormagnetisierungsmagnetfeld und der HF-Pulssequenz;
(iv) nach Schritt (iii), Messen des von der Testprobe erzeugten Signals; und
(v) basierend auf den Ergebnissen von Schritt (iv), Bestimmen des Auftretens und der Gattung des Pathogens in der biologischen Probe;
(b) Vergleichen der Wachstumsrate des Pathogens in einem ersten Aliquot einer zusätzlichen biologischen Probe in Gegenwart des antimikrobiellen Mittels mit der Wachstumsrate des Pathogens in einem zweiten Aliquot der zusätzlichen biologischen Probe in Abwesenheit des antimikrobiellen Mittels, wodurch bestimmt wird, ob das Pathogen empfindlich gegenüber dem antimikrobiellen Mittel ist, wobei die zusätzliche biologische Probe von dem Patienten nachfolgend auf Schritt (a) erhalten wurde.

4. Verfahren gemäß Anspruch 3, wobei
(i) Schritt (b) ferner das Beimpfen eines Wachstumsmediums mit einem Aliquot der zusätzlichen biologischen Probe, um eine Subkultur zu bilden, und das Inkubieren der Subkultur unter für ein verstärktes Wachstum des Pathogens geeigneten Bedingungen, umfasst, wobei das Wachstumsmedium basierend auf den Ergebnissen von Schritt (a) ausgewählt ist, wobei optional Schritt (c) das Vergleichen der Wachstumsrate eines ersten Aliquots der Subkultur in Gegenwart des antimikrobiellen Mittels mit der Wachstumsrate eines zweiten Aliquots der Subkultur in Abwesenheit des antimikrobiellen Mittels umfasst; oder
(ii) das Verfahren ferner einen Schritt des Durchführens einer Zentrifugation, Filtration oder Lysezentrifugation der zusätzlichen biologischen Probe vor Schritt (c) umfasst, wodurch ein Pellet umfassend das Pathogen hergestellt wird, wobei optional das Verfahren ferner das Ausplattieren des Pellets oder eines Teils davon auf eine oder mehrere Medienplatten umfasst, die basierend auf der Gattung des Pathogens ausgewählt sind, und Inkubieren der einen oder mehreren Medienplatten unter für das Wachstum des Pathogens geeigneten Bedingungen, insbesondere wobei die eine oder mehreren Medienplatten in Schritt (c) verwendet werden, um die Wachstumsraten des Pathogens in den ersten und zweiten Aliquots der zusätzlichen biologischen Probe zu vergleichen.

5. Verfahren gemäß einem beliebigen der Ansprüche 1-4, wobei
(i) die Wachstumsraten durch Verwenden eines Nährlösungsverdünnungstests, eines Scheibendiffusionstests, eines antimikrobiellen Gradiententests, chromogener Medien, eines Enzymaktivitätstests und/oder eines automatisierten Instruments bestimmt werden, wobei optional der antimikrobielle Gradiententest ein Epsilometertest ist;
(ii) das antimikrobielle Mittel von Schritt (c) basierend auf den Ergebnissen von Schritt (a) ausgewählt ist;
(iii) eine Vielzahl antimikrobieller Mittel in Schritt (c) getestet werden, um ein Profil der antimikrobiellen Empfindlichkeit des Pathogens zu bestimmen; wobei optional mindestens 4 oder mindestens 10 antimikrobielle Mittel in Schritt (c) getestet werden;
(iv) Amplifizieren durch asymmetrische Polymerase-Kettenreaktion (polymerase chain reaction, PCR) durchgeführt wird; und/oder
(v) Schritt (a) das Testen auf das Auftreten eines Panels von Pathogenen umfasst.

6. Verfahren gemäß einem beliebigen der Ansprüche 1-5, wobei Schritt (a) oder (c) ferner die Quantifizierung des Expressionsniveaus einer für das Pathogen charakteristischen Zielnukleinsäure umfasst, wobei optional Schritt (a) oder (c) das Amplifizieren der für das Pathogen charakteristischen Zielnukleinsäure in einem Reaktionsgemisch in einem Detektionsröhrchen umfasst, was in der Produktion eines Amplikons resultiert, das der für das Pathogen charakteristischen Zielnukleinsäure entspricht,
wobei das Verfahren in der in Schritt (a) genannten Vorrichtung durchgeführt wird, wobei das Reaktionsgemisch einen Teil der biologischen Probe umfassend die für das Pathogen charakteristische Zielnukleinsäure, für die Zielnukleinsäure spezifische Primer und superparamagnetische Partikel umfasst, wobei die superparamagnetischen Partikel fähig sind in Gegenwart des Amplikons zu aggregieren oder zu disaggregieren; und
wobei die Amplifikation die folgenden Schritte umfasst:
(i) Durchführen eines oder mehrerer Amplifikationszyklen;
(ii) Aussetzen der Reaktionsmischung an Bedingungen, die die Aggregation oder Disaggregation der superparamagnetischen Partikel erlauben;
(iii) Aussetzen der Probe einem Vormagnetisierungsfeld und einer HF-Pulssequenz;
(iv) nachfolgend Schritt (iii), Messen des Signals von dem Detektionsröhrchen;
(v) Wiederholen der Schritte (i)-(iv) bis eine gewünschte Menge der Amplifizierung erreicht ist; und
(vi) auf der Grundlage des Ergebnisses von Schritt (iv), Quantifizieren der beim entsprechenden Amplifikationszyklus vorhandenen Amplikons;
wobei die anfängliche Menge an Zielnukleinsäure, die für das antimikrobielle Resistenzgen in der Probe charakteristisch ist, basierend auf der bei jedem Amplifikationszyklus bestimmten Menge an Amplikons bestimmt wird.

7. Verfahren gemäß einem beliebigen der Ansprüche 1-6, wobei
(i) Schritt (a) ferner die Bestimmung des Titers des Pathogens umfasst;
(ii) das Verfahren in der Lage ist, 10 CFU, 3 CFU oder 1 CFU des Pathogens pro Milliliter der biologischen Probe nachzuweisen;
(iii) das Verfahren ferner das Auswählen einer Therapie, umfassend ein antimikrobielles Mittel für das Subjekt, basierend auf den Ergebnissen von Schritt (c), umfasst, wobei optional das Verfahren ferner das Bestimmen des Auftretens des Pathogens in der nachfolgenden biologischen Probe in einer nachfolgenden biologischen Probe, die von dem Subjekt nachfolgend der Verabreichung des antimikrobiellen Mittels erhalten wird, umfasst, insbesondere wobei das Verfahren ferner umfasst und/oder insbesondere ferner das Quantifizieren des Expressionsniveaus einer für das Pathogen charakteristischen Zielnukleinsäure in der nachfolgenden biologischen Probe umfasst;
(iv) die Gattung eine taxonomische Familie oder eine taxonomische Gattung ist; und/oder
(v) die Spezies des Pathogens nicht durch Schritt (a) bestimmt wird oder Schritt (a) ferner das Bestimmen der Spezies des Pathogens umfasst, wobei optional das antimikrobielle Mittel von Schritt (c) basierend auf der Spezies des Pathogens ausgewählt ist, wobei ferner optional die Spezies eine taxonomische Spezies ist.

8. Verfahren gemäß einem beliebigen der Ansprüche 1-7, wobei
(i) der Analyt ein für das Pathogen charakteristisches Amplikon ist, das durch Amplifizieren einer entsprechenden Zielnukleinsäure in Gegenwart eines vorwärts- und eines rückwärtsgerichteten Primers erzeugt wird; und/oder
(ii) die magnetischen Partikel eine erste Population von magnetischen Partikeln, die mit einer ersten Sonde konjugiert sind, und eine zweite Population von magnetischen Partikeln, die mit einer zweiten Sonde konjugiert sind, umfassen, wobei die magnetischen Partikel in Gegenwart des für das Pathogen charakteristischen Amplikons Aggregate bilden.

9. Verfahren gemäß einem beliebigen der Ansprüche 1-8, wobei
(i) Teilschritt (i) das Zugeben zu der flüssigen Probe von 1 x 10⁶ bis 1 x 10¹³ magnetischen Partikeln pro Milliliter der flüssigen Probe umfasst;
(ii) die magnetischen Partikel einen mittleren Durchmesser von 700 nm bis 950 nm aufweisen;
(iii) die magnetischen Partikel eine T₂-Relaxivität pro Partikel von 1 x 10⁹ bis 1 x 10¹² mM⁻¹ s⁻¹ aufweisen; und/oder
(iv) eine Vielzahl von Testproben in Teilschritt (i) hergestellt wird durch unabhängiges Inkontaktbringen jedes Mitglieds der Vielzahl von Testproben mit einer Population magnetischer Partikel, die konfiguriert sind, um in Gegenwart eines mit einem Mitglied des Panels von Pathogenen assoziierten Analyten Aggregate zu bilden, und wobei jedes Mitglied der Vielzahl von Testproben den Teilschritten (ii) bis (iv) des Verfahrens unterzogen wird.

10. Verfahren gemäß einem beliebigen der Ansprüche 1-9, wobei die Schritte des Verfahrens innerhalb von 2 Tagen, innerhalb von 12 Stunden oder innerhalb von 7 Stunden abgeschlossen werden.

11. Verfahren gemäß einem beliebigen der Ansprüche 1-10, wobei die biologische Probe ausgewählt ist aus der Gruppe bestehend aus Vollblut, Liquor (cerebrospinal fluid, CSF), Pleuraflüssigkeit, Urin oder Synovialflüssigkeit.

12. Verfahren gemäß einem beliebigen der Ansprüche 1-11, wobei das Pathogen ein Pilzpathogen, ein bakterielles Pathogen, ein Protozoen-Pathogen oder ein virales Pathogen ist.

13. Verfahren gemäß Anspruch 12, wobei:
(i) das Pilzpathogen ein *Candida* spp. ist, wobei optional der *Candida* spp. ausgewählt ist aus der Gruppe bestehend aus *Candida albicans, Candida guilliermondii, Candida glabrata, Candida krusei, Candida lusitaniae, Candida parapsilosis* und *Candida tropicalis;*
(ii) das bakterielle Pathogen ausgewählt ist aus der Gruppe bestehend aus *Escherichia coli, Acinetobacter baumannii, Enterococcus faecalis, Enterococcus faecium, Klebsiella pneumoniae, Pseudomonas aeruginosa, Staphylococcus aureus, Borrelia burgdorferi, Borrelia afzelii, Borrelia garinii, Rickettsia rickettsii, Anaplasma phagocytophilum, Coxiella burnetii, Ehrlichia chaffeensis, Ehrlichia ewingii, Francisella tularensis, Streptococcus pneumoniae* und *Neisseria meningitides;* oder
(iii) das Protozoen-Pathogen *Babesia microti* oder *Babesia divergens* ist.

14. Verfahren gemäß Anspruch 12, wobei das Pathogen mit einer Blutbahninfektion, Sepsis, septischer Arthritis, Lungenentzündung, Peritonitis, Osteomyelitis, Meningitis, Harnwegsinfektion oder Lyme-Krankheit assoziiert ist.

15. Verfahren gemäß Anspruch 14, wobei die Blutbahninfektion eine Fungämie, Bakteriämie oder Virämie ist, insbesondere wobei die Fungämie eine Candidämie ist.

## Revendications

1. Procédé pour effectuer un antibiogramme rapide pour un agent pathogène dans un échantillon biologique obtenu chez un sujet, le procédé comprenant :
(a) la détermination de la présence et du genre d'un agent pathogène dans un échantillon biologique par
(i) la préparation d'un échantillon de test en mettant en contact une première partie de l'échantillon biologique avec des particules magnétiques, où les particules magnétiques comportent des fragments de liaison sur leurs surfaces, les fragments de liaison étant fonctionnels pour altérer l'agrégation spécifique des particules magnétiques en présence d'un analyte associé à l'agent pathogène ;
(ii) la mise en place de l'échantillon de test dans un dispositif, le dispositif comprenant un support définissant un puits pour contenir l'échantillon de test, et comportant une antenne RF configurée pour détecter un signal produit en exposant l'échantillon de test à un champ magnétique de polarisation créé en utilisant un ou plusieurs aimants et une séquence d'impulsions RF ;
(iii) l'exposition de l'échantillon de test au champ magnétique de polarisation et à la séquence d'impulsions RF ;
(iv) après l'étape (iii), la mesure du signal produit par l'échantillon de test ; et
(v) en se basant sur les résultats de l'étape (iv), la détermination de la présence et du genre de l'agent pathogène dans l'échantillon biologique ;
(b) en parallèle à l'étape (a), l'incubation d'une seconde partie de l'échantillon biologique dans des conditions appropriées pour permettre la croissance de l'agent pathogène afin de former une culture d'agent pathogène ; et
(c) la comparaison du taux de croissance d'une première aliquote de la culture d'agent pathogène en présence de l'agent antimicrobien au taux de croissance d'une seconde aliquote de la culture d'agent pathogène en l'absence de l'agent antimicrobien, en déterminant ainsi si l'agent pathogène est sensible à l'agent antimicrobien.

2. Procédé selon la revendication 1, dans lequel
(i) l'étape (b) comprend en outre l'inoculation d'un milieu de croissance avec une aliquote de l'échantillon biologique pour former une sous-culture et l'incubation de la sous-culture dans des conditions appropriées pour permettre une croissance améliorée de l'agent pathogène, où le milieu de croissance est sélectionné en se basant sur les résultats de l'étape (a), facultativement dans lequel l'étape (c) comprend la comparaison du taux de croissance d'une première aliquote de la sous-culture en présence de l'agent antimicrobien au taux de croissance d'une seconde aliquote de la sous-culture en l'absence de l'agent antimicrobien ;
(ii) le procédé comprend en outre la mise en contact de la culture d'agent pathogène avec un additif avant l'étape (c), où l'additif est sélectionné en se basant sur les résultats de l'étape (a), en améliorant ainsi la croissance de la culture ; et/ou
(iii) le procédé comprend en outre une étape consistant à réaliser une centrifugation, une filtration, ou une lyse-centrifugation de la culture d'agent pathogène ou d'une partie supplémentaire de l'échantillon biologique avant l'étape (c), en produisant ainsi un culot comprenant l'agent pathogène, facultativement où le procédé comprend en outre l'ensemencement du culot ou d'une partie de celui-ci sur une ou plusieurs plaques de milieu sélectionnées en se basant sur le genre de l'agent pathogène, et l'incubation de la une ou plusieurs plaques de milieu dans des conditions appropriées pour permettre la croissance de l'agent pathogène, particulièrement dans lequel la une ou plusieurs plaques de milieu sont utilisées à l'étape (c) pour comparer les taux de croissance de l'agent pathogène dans les première et seconde aliquotes de la culture d'agent pathogène.

3. Procédé pour effectuer un antibiogramme rapide pour un agent pathogène dans un échantillon biologique obtenu chez un sujet, le procédé comprenant :
(a) la détermination de la présence et du genre d'un agent pathogène dans un échantillon biologique par
(i) la préparation d'un échantillon de test en mettant en contact une première partie de l'échantillon biologique avec des particules magnétiques, où les particules magnétiques comportent des fragments de liaison sur leurs surfaces, les fragments de liaison étant fonctionnels pour altérer l'agrégation spécifique des particules magnétiques en présence d'un analyte associé à l'agent pathogène ;
(ii) la mise en place de l'échantillon de test dans un dispositif, le dispositif comprenant un support définissant un puits pour contenir l'échantillon de test, et comportant une antenne RF configurée pour détecter un signal produit en exposant l'échantillon de test à un champ magnétique de polarisation créé en utilisant un ou plusieurs aimants et une séquence d'impulsions RF ;
(iii) l'exposition de l'échantillon de test au champ magnétique de polarisation et à la séquence d'impulsions RF ;
(iv) après l'étape (iii), la mesure du signal produit par l'échantillon de test ; et
(v) en se basant sur les résultats de l'étape (iv), la détermination de la présence et du genre de l'agent pathogène dans l'échantillon biologique ;
(b) la comparaison du taux de croissance de l'agent pathogène dans une première aliquote d'un échantillon biologique supplémentaire en présence de l'agent antimicrobien au taux de croissance de l'agent pathogène dans une seconde aliquote de l'échantillon biologique supplémentaire en l'absence de l'agent antimicrobien, en déterminant ainsi si l'agent pathogène est sensible à l'agent antimicrobien, où l'échantillon biologique supplémentaire a été obtenu chez le patient après l'étape (a) .

4. Procédé selon la revendication 3, dans lequel
(i) l'étape (b) comprend en outre l'inoculation d'un milieu de croissance avec une aliquote de l'échantillon biologique supplémentaire pour former une sous-culture et l'incubation de la sous-culture dans des conditions appropriées pour permettre une croissance améliorée de l'agent pathogène, où le milieu de croissance est sélectionné en se basant sur les résultats de l'étape (a), facultativement dans lequel l'étape (c) comprend la comparaison du taux de croissance d'une première aliquote de la sous-culture en présence de l'agent antimicrobien au taux de croissance d'une seconde aliquote de la sous-culture en l'absence de l'agent antimicrobien ; ou
(ii) le procédé comprend en outre une étape consistant à réaliser une centrifugation, une filtration, ou une lyse-centrifugation de l'échantillon biologique supplémentaire avant l'étape (c), en produisant ainsi un culot comprenant l'agent pathogène, facultativement où le procédé comprend en outre l'ensemencement du culot ou d'une partie de celui-ci sur une ou plusieurs plaques de milieu sélectionnées en se basant sur le genre de l'agent pathogène, et l'incubation de la une ou plusieurs plaques de milieu dans des conditions appropriées pour permettre la croissance de l'agent pathogène, particulièrement dans lequel la une ou plusieurs plaques de milieu sont utilisées à l'étape (c) pour comparer les taux de croissance de l'agent pathogène dans les première et seconde aliquotes de l'échantillon biologique supplémentaire.

5. Procédé selon l'une quelconque des revendications 1 à 4, dans lequel
(i) les taux de croissance sont déterminés en utilisant un test de dilution en bouillon, un test de diffusion sur disque, un test de gradient antimicrobien, des milieux chromogènes, un dosage de l'activité enzymatique, et/ou un instrument automatisé, facultativement dans lequel le test de gradient antimicrobien est un test d'epsilomètre ;
(ii) l'agent antimicrobien de l'étape (c) est sélectionné en se basant sur les résultats de l'étape (a) ;
(iii) une pluralité d'agents antimicrobiens sont testés à l'étape (c) afin de déterminer un profil de sensibilité antimicrobienne de l'agent pathogène ; facultativement dans lequel au moins 4 ou au moins 10 agents antimicrobiens sont testés à l'étape (c) ;
(iv) l'amplification est réalisée par une réaction en chaîne par polymérase (PCR) asymétrique ; et/ou
(v) l'étape (a) comprend le test de la présence d'un panel d'agents pathogènes.

6. Procédé selon l'une quelconque des revendications 1 à 5, dans lequel l'étape (a) ou (c) comprend en outre la quantification du taux d'expression d'un acide nucléique cible caractéristique de l'agent pathogène, facultativement dans lequel l'étape (a) ou (c) comprend l'amplification de l'acide nucléique cible caractéristique de l'agent pathogène dans un mélange réactionnel dans un tube de détection, en ayant pour résultat la production d'un amplicon correspondant à l'acide nucléique cible caractéristique de l'agent pathogène,
dans lequel le procédé est réalisé dans le dispositif décrit à l'étape (a), ledit mélange réactionnel comprenant une partie de l'échantillon biologique comprenant l'acide nucléique cible caractéristique de l'agent pathogène, des amorces spécifiques audit acide nucléique cible, et des particules superparamagnétiques, lesdites particules superparamagnétiques étant fonctionnelles pour s'agréger ou se désagréger en présence de l'amplicon ; et
ladite amplification comprenant les étapes suivantes consistant à :
(i) réaliser un ou plusieurs cycles d'amplification ;
(ii) exposer ledit mélange réactionnel à des conditions permettant l'agrégation ou la désagrégation desdites particules superparamagnétiques ;
(iii) l'exposition de l'échantillon à un champ magnétique de polarisation et à une séquence d'impulsions RF ;
(iv) après l'étape (iii), la mesure du signal à partir du tube de détection ;
(v) la répétition des étapes (i)-(iv) jusqu'à ce qu'une quantité d'amplification souhaitée soit obtenue ; et
(vi) en se basant sur le résultat de l'étape (iv), la quantification des amplicons présents au cycle d'amplification correspondant ;
dans lequel la quantité initiale de l'acide nucléique cible caractéristique du gène de résistance antimicrobienne dans ledit échantillon est déterminée en se basant sur la quantité d'amplicons déterminés à chaque cycle de ladite amplification.

7. Procédé selon l'une quelconque des revendications 1 à 6, dans lequel
(i) l'étape (a) comprend en outre la détermination du titre de l'agent pathogène ;
(ii) le procédé est capable de détecter 10 UFC, 3 UFC ou 1 UFC de l'agent pathogène par millilitre de l'échantillon biologique ;
(iii) le procédé comprend en outre la sélection d'une thérapie comprenant un agent antimicrobien pour le sujet en se basant sur les résultats de l'étape (c), facultativement où le procédé comprend en outre la détermination de la présence de l'agent pathogène dans l'échantillon biologique subséquent dans un échantillon biologique subséquent obtenu chez le sujet après l'administration de l'agent antimicrobien, particulièrement comprenant en outre et/ou particulièrement comprenant en outre la quantification du taux d'expression d'un acide nucléique cible caractéristique de l'agent pathogène dans l'échantillon biologique subséquent ;
(iv) le genre est une famille taxonomique ou un genre taxonomique ; et/ou
(v) l'espèce de l'agent pathogène n'est pas déterminée par l'étape (a) ou l'étape (a) comprend en outre la détermination de l'espèce de l'agent pathogène, facultativement l'agent antimicrobien de l'étape (c) est sélectionné en se basant sur l'espèce de l'agent pathogène, facultativement en outre l'espèce est une espèce taxonomique.

8. Procédé selon l'une quelconque des revendications 1 à 7, dans lequel
(i) l'analyte est un amplicon caractéristique de l'agent pathogène généré en amplifiant un acide nucléique cible correspondant en présence d'une amorce sens et d'une amorce antisens ; et/ou
(ii) les particules magnétiques comprennent une première population de particules magnétiques conjuguées à une première sonde, et une seconde population de particules magnétiques conjuguées à une seconde sonde, dans lequel les particules magnétiques forment des agrégats en présence de l'amplicon caractéristique de l'agent pathogène.

9. Procédé selon l'une quelconque des revendications 1 à 8, dans lequel
(i) la sous-étape (i) comprend l'ajout, à l'échantillon liquide, de 1 × 10⁶ à 1 × 10¹³ particules magnétiques par millilitre de l'échantillon liquide ;
(ii) les particules magnétiques possèdent un diamètre moyen compris entre 700 nm et 950 nm ;
(iii) les particules magnétiques possèdent une relaxation T₂ par particule comprise entre 1 × 10⁹ à 1 × 10¹² mM⁻¹s⁻¹; et/ou
(iv) une pluralité d'échantillons de test sont préparés à la sous-étape (i) en mettant indépendamment en contact chaque membre de la pluralité d'échantillons de test avec une population de particules magnétiques configurées pour former des agrégats en présence d'un analyte associé à un membre du panel d'agents pathogènes, et dans lequel chaque membre de la pluralité d'échantillons de test est soumis aux sous-étapes (ii) à (iv) du procédé.

10. Procédé selon l'une quelconque des revendications 1 à 9, dans lequel les étapes du procédé sont réalisées en 2 jours, en 12 heures ou en 7 heures.

11. Procédé selon l'une quelconque des revendications 1 à 10, dans lequel l'échantillon biologique est sélectionné dans le groupe consistant en du sang total, du liquide céphalorachidien (LCR), du liquide pleural, de l'urine, ou du liquide synovial.

12. Procédé selon l'une quelconque des revendications 1 à 11, dans lequel l'agent pathogène est un agent pathogène fongique, un agent pathogène bactérien, un agent pathogène protozoaire, ou un agent pathogène viral.

13. Procédé selon la revendication 12, dans lequel :
(i) l'agent pathogène fongique est une espèce *Candida,* facultativement dans lequel l'espèce *Candida* est sélectionnée dans le groupe consistant en *Candida albicans, Candida guilliermondii, Candida glabrata, Candida krusei, Candida lusitaniae, Candida parapsilosisr,* et *Candida tropicalis ;*
(ii) l'agent pathogène bactérien est sélectionné dans le groupe consistant en *Escherichia coli,* Acinetobacter baumannii, *Enterococcus faecalis, Enterococcus faecium, Klebsiella pneumoniae, Pseudomonas aeruginosa, Staphylococcus aureus,* Borrelia burgdorferi, *Borrelia afzelii, Borrelia garinii, Rickettsia rickettsii, Anaplasma phagocytophilum, Coxiella burnetii, Ehrlichia chaffeensis, Ehrlichia ewingii, Francisella tularensis, Streptococcus pneumoniae,* et *Neisseria meningitides* ; ou
(iii) l'agent pathogène protozoaire est *Babesia microti* ou *Babesia divergens.*

14. Procédé selon la revendication 12, dans lequel l'agent pathogène est associé à une infection de la circulation sanguine, un sepsis, l'arthrite septique, une pneumonie, la péritonite, l'ostéomyélite, la méningite, une infection des voies urinaires ou la maladie de Lyme.

15. Procédé selon la revendication 14, dans lequel l'infection de la circulation sanguine est une fongémie, une bactériémie, ou une virémie, particulièrement dans lequel la fongémie est une candidémie.


### SEQUENCE LISTING

<110> T2 Biosystems, Inc.
<120> RAPID ANTIMICROBIAL SUSCEPTIBILITY TESTING USING HIGH-SENSITIVITY DIRECT DETECTION METHODS
<130> 50713-099WO2
<150> US 62/281,603
   <151> 2016-01-21
<160> 77
<170> PatentIn version 3.5
<210> 1
   <211> 27
   <212> DNA
   <213> Artificial Sequence
<220>
   <223> Synthetic Construct
<400> 1
   cgttttccaa atctgtaaca gactggg 27
<210> 2
   <211> 25
   <212> DNA
   <213> Artificial Sequence
<220>
   <223> Synthetic Construct
<400> 2
   aggacgttga taggttggat gtgga 25
<210> 3
   <211> 31
   <212> DNA
   <213> Artificial Sequence
<220>
   <223> Synthetic Construct
<400> 3
   ggtagctatg tagggaaggg ataaacgctg a 31
<210> 4
   <211> 28
   <212> DNA
   <213> Artificial Sequence
<220>
   <223> Synthetic Construct
<400> 4
   gcgctaagga gcttaacttc tgtgttcg 28
<210> 5
   <211> 22
   <212> DNA
   <213> Artificial Sequence
<220>
   <223> Synthetic Construct
<400> 5
   gacggttgtc ccggtttaag ca 22
<210> 6
   <211> 21
   <212> DNA
   <213> Artificial Sequence
<220>
   <223> Synthetic Construct
<400> 6
   gctggtatct tcgactggtc t 21
<210> 7
   <211> 22
   <212> DNA
   <213> Artificial Sequence
<220>
   <223> Synthetic Construct
<400> 7
   aggctgggtg tgtaagcgtt gt 22
<210> 8
   <211> 25
   <212> DNA
   <213> Artificial Sequence
<220>
   <223> Synthetic Construct
<400> 8
   caagcaattc ggttggatat ccgtt 25
<210> 9
   <211> 35
   <212> DNA
   <213> Artificial Sequence
<220>
   <223> Synthetic Construct
<220>
   <221> misc_feature
   <222> (16) .. (16)
   <223> n is 2,6-diaminopurine
<400> 9
   ggtaatgaat tacctntctc tgctggtttc ttctt 35
<210> 10
   <211> 27
   <212> DNA
   <213> Artificial Sequence
<220>
   <223> Synthetic Construct
<220>
   <221> misc_feature
   <222> (13)..(13)
   <223> n is 2,6-diaminopurine
<400> 10
   accagcatct tcngcatctt ctgtaaa 27
<210> 11
   <211> 34
   <212> DNA
   <213> Artificial Sequence
<220>
   <223> Synthetic Construct
<220>
   <221> misc_feature
   <222> (13)..(13)
   <223> n is 2,6-diaminopurine
<400> 11
   gaagttatgt ttnctattcg aatcgtggtc cagt 34
<210> 12
   <211> 27
   <212> DNA
   <213> Artificial Sequence
<220>
   <223> Synthetic Construct
<400> 12
   gttgtaaagc catgatgctc gtaacca 27
<210> 13
   <211> 20
   <212> DNA
   <213> Artificial Sequence
<220>
   <223> Synthetic Construct
<400> 13
   ggcatgcctg tttgagcgtc 20
<210> 14
   <211> 29
   <212> DNA
   <213> Artificial Sequence
<220>
   <223> Synthetic Construct
<400> 14
   gcttattgat atgcttaagt tcagcgggt 29
<210> 15
   <211> 22
   <212> DNA
   <213> Artificial Sequence
<220>
   <223> Synthetic Construct
<400> 15
   tgaggcttga ctatacaaca cc 22
<210> 16
   <211> 29
   <212> DNA
   <213> Artificial Sequence
<220>
   <223> Synthetic Construct
<400> 16
   ctaaaatgaa cagataaagt aagattcaa 29
<210> 17
   <211> 32
   <212> DNA
   <213> Artificial Sequence
<220>
   <223> Synthetic Construct
<220>
   <221> misc_feature
   <222> (1)..(1)
   <223> n is 5' amino modifier C12
<400> 17
   nttttttttt tgaggcttga ctatacaaca cc 32
<210> 18
   <211> 39
   <212> DNA
   <213> Artificial Sequence
<220>
   <223> Synthetic Construct
<220>
   <221> misc_feature
   <222> (39) .. (39)
   <223> n is 3' amino modifier
<400> 18
   ctaaaatgaa cagataaagt aagattcaat ttttttttn 39
<210> 19
   <211> 29
   <212> DNA
   <213> Artificial Sequence
<220>
   <223> Synthetic Construct
<400> 19
   aaaacttata tgacttcaaa tccagtttt 29
<210> 20
   <211> 27
   <212> DNA
   <213> Artificial Sequence
<220>
   <223> Synthetic Construct
<400> 20
   tttactcaat aaaagataac accacag 27
<210> 21
   <211> 39
   <212> DNA
   <213> Artificial Sequence
<220>
   <223> Synthetic Construct
<220>
   <221> misc_feature
   <222> (1)..(1)
   <223> n is 5' amino modifier C12
<400> 21
   nttttttttt aaaacttata tgacttcaaa tccagtttt 39
<210> 22
   <211> 38
   <212> DNA
   <213> Artificial Sequence
<220>
   <223> Synthetic Construct
<220>
   <221> misc_feature
   <222> (38)..(38)
   <223> n is 3' amino modifier
<400> 22
   tttactcaat aaaagataac accacagttt tttttttn 38
<210> 23
   <211> 24
   <212> DNA
   <213> Artificial Sequence
<220>
   <223> Synthetic Construct
<400> 23
   tggataagta aaagcaactt ggtt 24
<210> 24
   <211> 30
   <212> DNA
   <213> Artificial Sequence
<220>
   <223> Synthetic Construct
<400> 24
   aatgaagatt caactcaata agaaacaaca 30
<210> 25
   <211> 34
   <212> DNA
   <213> Artificial Sequence
<220>
   <223> Synthetic Construct
<220>
   <221> misc_feature
   <222> (1)..(1)
   <223> n is 5' amino modifier C12
<400> 25
   nttttttttt tggataagta aaagcaactt ggtt 34
<210> 26
   <211> 40
   <212> DNA
   <213> Artificial Sequence
<220>
   <223> Synthetic Construct
<220>
   <221> misc_feature
   <222> (40)..(40)
   <223> n is 3' amino modifier
<400> 26
   aatgaagatt caactcaata agaaacaaca tttttttttn 40
<210> 27
   <211> 23
   <212> DNA
   <213> Artificial Sequence
<220>
   <223> Synthetic Construct
<400> 27
   taccaaggcg cttgagagaa ctc 23
<210> 28
   <211> 19
   <212> DNA
   <213> Artificial Sequence
<220>
   <223> Synthetic Construct
<400> 28
   ctggtgtgta ggtgaagtc 19
<210> 29
   <211> 33
   <212> DNA
   <213> Artificial Sequence
<220>
   <223> Synthetic Construct
<220>
   <221> misc_feature
   <222> (1)..(1)
   <223> n is 5' amino modifier C12
<400> 29
   nttttttttt taccaaggcg cttgagagaa ctc 33
<210> 30
   <211> 29
   <212> DNA
   <213> Artificial Sequence
<220>
   <223> Synthetic Construct
<220>
   <221> misc_feature
   <222> (29) .. (29)
   <223> n is 3' amino modifier
<400> 30
   ctggtgtgta ggtgaagtct ttttttttn 29
<210> 31
   <211> 23
   <212> DNA
   <213> Artificial Sequence
<220>
   <223> Synthetic Construct
<400> 31
   gtgtgttgta gggtgaagtc gac 23
<210> 32
   <211> 22
   <212> DNA
   <213> Artificial Sequence
<220>
   <223> Synthetic Construct
<400> 32
   caccttgaaa tcacatacct ga 22
<210> 33
   <211> 33
   <212> DNA
   <213> Artificial Sequence
<220>
   <223> Synthetic Construct
<220>
   <221> misc_feature
   <222> (1)..(1)
   <223> n is 5' amino modifier C12
<400> 33
   nttttttttt gtgtgttgta gggtgaagtc gac 33
<210> 34
   <211> 32
   <212> DNA
   <213> Artificial Sequence
<220>
   <223> Synthetic Construct
<220>
   <221> misc_feature
   <222> (32)..(32)
   <223> n is 3' amino modifier
<400> 34
   caccttgaaa tcacatacct gatttttttt tn 32
<210> 35
   <211> 23
   <212> DNA
   <213> Artificial Sequence
<220>
   <223> Synthetic Construct
<400> 35
   ccatttgaag ttgtttatta tgc 23
<210> 36
   <211> 27
   <212> DNA
   <213> Artificial Sequence
<220>
   <223> Synthetic Construct
<400> 36
   gggaaatgat taattatgca ttaaatc 27
<210> 37
   <211> 33
   <212> DNA
   <213> Artificial Sequence
<220>
   <223> Synthetic Construct
<220>
   <221> misc_feature
   <222> (1)..(1)
   <223> 5' amino modifier C12
<400> 37
   nttttttttt ccatttgaag ttgtttatta tgc 33
<210> 38
   <211> 37
   <212> DNA
   <213> Artificial Sequence
<220>
   <223> Synthetic Construct
<220>
   <221> misc_feature
   <222> (37)..(37)
   <223> n is 3' amino modifier
<400> 38
   gggaaatgat taattatgca ttaaatcttt ttttttn 37
<210> 39
   <211> 26
   <212> DNA
   <213> Artificial Sequence
<220>
   <223> Synthetic Construct
<400> 39
   tttttcagat ttaggattag ttgatt 26
<210> 40
   <211> 22
   <212> DNA
   <213> Artificial Sequence
<220>
   <223> Synthetic Construct
<400> 40
   gatccgtatt ggttatatca tc 22
<210> 41
   <211> 36
   <212> DNA
   <213> Artificial Sequence
<220>
   <223> Synthetic Construct
<220>
   <221> misc_feature
   <222> (1)..(1)
   <223> n is 5' amino modifier C12
<400> 41
   nttttttttt tttttcagat ttaggattag ttgatt 36
<210> 42
   <211> 32
   <212> DNA
   <213> Artificial Sequence
<220>
   <223> Synthetic Construct
<220>
   <221> misc_feature
   <222> (32)..(32)
   <223> n is 3' amino modifier
<400> 42
   gatccgtatt ggttatatca tctttttttt tn 32
<210> 43
   <211> 31
   <212> DNA
   <213> Artificial Sequence
<220>
   <223> Synthetic Construct
<220>
   <221> misc_feature
   <222> (1)..(1)
   <223> n is 5' amino modifier C12
<400> 43
   nttttttttt tggaataata cgccgaccag c 31
<210> 44
   <211> 35
   <212> DNA
   <213> Artificial Sequence
<220>
   <223> Synthetic Construct
<220>
   <221> misc_feature
   <222> (35)..(35)
   <223> n is 3' amino modifier
<400> 44
   aaggatctat ttcagtatga tgcagttttt ttttn 35
<210> 45
   <211> 200
   <212> DNA
   <213> Artificial Sequence
<220>
   <223> Synthetic Construct
<400> 45
   tgccgaagcg ttttccaaat ctgtaacaga ctgggctgat tgaatcttac tttatctgtt 60
   cattttagct agaggtataa ctaaatcaag ttgtcttgca tatttaagaa tcgattgatg 120
   ctttatatac aactgcttgg gtgttgtata gtcaagcctc acgagcaatt agtattggtc 180
   agcttcacat atcactatgc 200
<210> 46
   <211> 200
   <212> DNA
   <213> Artificial Sequence
<220>
   <223> Synthetic Construct
<400> 46
   gcatgggaac aggtgtatcc ttctcgctat cgccaccaca ctgggtgttg tttcttattg 60
   agttgaatct tcattcactc aaaactggat tgaagtttga atcaaaataa ccaagttgct 120
   tttacttatc cattctttgg ttaagtcctc gaccgattag tattggtccg ctccaactat 180
   cactagcctt ccacttccaa 200
<210> 47
   <211> 200
   <212> DNA
   <213> Artificial Sequence
<220>
   <223> Synthetic Construct
<400> 47
   gcatggttac aggtgtatcc ttctcgctat cgccaccaca ctgtggtgtt atcttttatt 60
   gagtaaattt tgttcactca aaactggatt tgaagtcata taagtttttt tccgagttct 120
   tttcttttaa cctattggtt aagtcctcga tcgattagta tcagtccgct ccatacatca 180
   ctgtacttcc actcctgacc 200
<210> 48
   <211> 200
   <212> DNA
   <213> Artificial Sequence
<220>
   <223> Synthetic Construct
<400> 48
   cagctccatc cgcagggact tcacctacac accagcgtgc cttctcccga agttacggca 60
   ccattttgcc tagttccttc acccgagttc tctcaagcgc cttggtattc tctacctgac 120
   cacctgtgtc ggtttggggt acgatttgat gttacctgat gcttagaggc ttttcctgga 180
   agcagggcat ttgttacttc 200
<210> 49
   <211> 169
   <212> DNA
   <213> Artificial Sequence
<220>
   <223> Synthetic Construct
<400> 49
   cgcttgggct tacgtctatc cggattcagg tatgtgattt caaggtgttt tgcggttcat 60
   gcgaactttc ggttcgtcga cttcacctta caacacacaa tcgtcagatt gtttgggtgt 120
   tatatggtca agcctcacgg gcaattagta ctggttagct caacgcctc 169
<210> 50
   <211> 151
   <212> DNA
   <213> Artificial Sequence
<220>
   <223> Synthetic Construct
<400> 50
   tttaccacta acaccataga aattataacg gtcaatgcca tgatttaatg cataattaat 60
   catttcccat tgcactgcat aacttccggc aaaatgacgg aatgcatttg atgtaccacc 120
   agcataataa acaacttcaa atgggttgat a 151
<210> 51
   <211> 186
   <212> DNA
   <213> Artificial Sequence
<220>
   <223> Synthetic Construct
<400> 51
   tgtgatttaa acaagtttac taaggcatca tttttctcgc gaccttcaaa tggcacgata 60
   tctttatcat atagatgata taaccaatac ggatctaatt taacatataa acattgatgt 120
   tgctgtaaat atttatctaa ctcttttaaa taataatcaa ctaatcctaa atctgaaaaa 180
   tccatt 186
<210> 52
   <211> 25
   <212> DNA
   <213> Artificial Sequence
<220>
   <223> Synthetic Construct
<400> 52
   gcattaatcg acggtatggt tgacc 25
<210> 53
   <211> 26
   <212> DNA
   <213> Artificial Sequence
<220>
   <223> Synthetic Construct
<400> 53
   cctgctgaaa caggttttcc cacata 26
<210> 54
   <211> 25
   <212> DNA
   <213> Artificial Sequence
<220>
   <223> Synthetic Construct
<400> 54
   agtgatgatg agttgtttgc cagtg 25
<210> 55
   <211> 22
   <212> DNA
   <213> Artificial Sequence
<220>
   <223> Synthetic Construct
<400> 55
   tgaattgtcg ccgcgtgacc ag 22
<210> 56
   <211> 23
   <212> DNA
   <213> Artificial Sequence
<220>
   <223> Synthetic Construct
<400> 56
   acccagcggt ttgagggaga aac 23
<210> 57
   <211> 30
   <212> DNA
   <213> Artificial Sequence
<220>
   <223> Synthetic Construct
<400> 57
   aaagtttgaa gatatacgtg gtggacgtta 30
<210> 58
   <211> 21
   <212> DNA
   <213> Artificial Sequence
<220>
   <223> Synthetic Construct
<400> 58
   cgcacgcgca agatggaaac g 21
<210> 59
   <211> 23
   <212> DNA
   <213> Artificial Sequence
<220>
   <223> Synthetic Construct
<400> 59
   aagttcagcg ggtattccta cct 23
<210> 60
   <211> 27
   <212> DNA
   <213> Artificial Sequence
<220>
   <223> Synthetic Construct
<400> 60
   agctttttgt tgtctcgcaa cactcgc 27
<210> 61
   <211> 26
   <212> DNA
   <213> Artificial Sequence
<220>
   <223> Synthetic Construct
<400> 61
   ctaccaaaca caatgtgttt gagaag 26
<210> 62
   <211> 31
   <212> DNA
   <213> Artificial Sequence
<220>
   <223> Synthetic Construct
<400> 62
   cctgatttga ggtcaaactt aaagacgtct g 31
<210> 63
   <211> 24
   <212> DNA
   <213> Artificial Sequence
<220>
   <223> Synthetic Construct
<220>
   <221> misc_feature
   <222> (22) .. (22)
   <223> n is 5' 5-Nitroindole
<400> 63
   agtcctacct gatttgaggt cnaa 24
<210> 64
   <211> 21
   <212> DNA
   <213> Artificial Sequence
<220>
   <223> Synthetic Construct
<220>
   <221> misc_feature
   <222> (4) .. (4)
   <223> n is 5' 5-Nitroindole
<400> 64
   ccgngggttt gagggagaaa t 21
<210> 65
   <211> 33
   <212> DNA
   <213> Artificial Sequence
<220>
   <223> Synthetic Construct
<400> 65
   aaagttatga aataaattgt ggtggccact agc 33
<210> 66
   <211> 22
   <212> DNA
   <213> Artificial Sequence
<220>
   <223> Synthetic Construct
<400> 66
   acccgggggt ttgagggaga aa 22
<210> 67
   <211> 24
   <212> DNA
   <213> Artificial Sequence
<220>
   <223> Synthetic Construct
<400> 67
   agtcctacct gatttgaggt cgaa 24
<210> 68
   <211> 21
   <212> DNA
   <213> Artificial Sequence
<220>
   <223> Synthetic Construct
<400> 68
   ccgagggttt gagggagaaa t 21
<210> 69
   <211> 25
   <212> DNA
   <213> Artificial Sequence
<220>
   <223> Synthetic Construct
<400> 69
   ggaagggatc aggtggttca ctctt 25
<210> 70
   <211> 29
   <212> DNA
   <213> Artificial Sequence
<220>
   <223> Synthetic Construct
<400> 70
   aaaacttatg tgacttcaaa tccagtttt 29
<210> 71
   <211> 28
   <212> DNA
   <213> Artificial Sequence
<220>
   <223> Synthetic Construct
<400> 71
   tttactcaat aaaagataac accacagt 28
<210> 72
   <211> 39
   <212> DNA
   <213> Artificial Sequence
<220>
   <223> Synthetic Construct
<220>
   <221> misc_feature
   <222> (1)..(1)
   <223> n is 5' amino modifier C12
<400> 72
   nttttttttt aaaacttatg tgacttcaaa tccagtttt 39
<210> 73
   <211> 23
   <212> DNA
   <213> Artificial Sequence
<220>
   <223> Synthetic Construct
<400> 73
   tctgacgatt gtgtgttgta agg 23
<210> 74
   <211> 20
   <212> DNA
   <213> Artificial Sequence
<220>
   <223> Synthetic Construct
<400> 74
   ggatagacgt aagcccaagc 20
<210> 75
   <211> 33
   <212> DNA
   <213> Artificial Sequence
<220>
   <223> Synthetic Construct
<220>
   <221> misc_feature
   <222> (1)..(1)
   <223> n is 5' amino modifier C12
<400> 75
   nttttttttt tctgacgatt gtgtgttgta agg 33
<210> 76
   <211> 30
   <212> DNA
   <213> Artificial Sequence
<220>
   <223> Synthetic Construct
<220>
   <221> misc_feature
   <222> (30)..(30)
   <223> n is 3' amino modifier
<400> 76
   ggatagacgt aagcccaagc tttttttttn 30
<210> 77
   <211> 198
   <212> DNA
   <213> Artificial Sequence
<220>
   <223> Synthetic Construct
<400> 77
   gcatggttac aggtgtatcc ttctcgctat cgccaccaca ctgtggtgtt atcttttatt      60
   gagtaaattt tgttcactca aaactggatt tgaagtcata taagtttttt tccgagttct     120
   tttcttttaa cctattggtt aagtcctcga tcgattagta tcagtccgct ccatacatca     180
   ctgtacttcc actcctga                                                   198